# EUROPÄISCHE PATENTSCHRIFT

(11) **EP 2 875 699 B1**
(45) Veröffentlichungstag und Bekanntmachung des Hinweises auf die Patenterteilung: **15.02.2017**
(21) Anmeldenummer: 13732368.9
(22) Anmeldetag: 27.06.2013
(51) Int. Cl.: H05B 33/20, H01L 27/32, C09K 11/06, C07C 211/61, C07D 307/91, H01L 51/00, H01L 51/50

(54) **DERIVATE VON 2-DIARYLAMINOFLUOREN UND DIESE ENTHALTNDE ORGANISCHE ELEKTRONISCHE VERBINDUNGEN**
DERIVATES OF 2-DIARYLAMINOFLUOREN AND ORGANIC ELECTRONIC DEVICES COMPRISING THE SAME
DERIVES DE 2-DIARYLAMINOFLUOREN ET DISPOSITIVES ELECTRONIQUES ET ORGANIQUES COMPRENANT CES MATERIAUX

(30) Priorität: 23.07.2012 EP 12005370
(43) Veröffentlichungstag der Anmeldung: 27.05.2015
(73) Patentinhaber: Merck Patent GmbH, 64293 Darmstadt (DE)
(72) Erfinder: MUJICA-FERNAUD, Teresa, 64289 Darmstadt (DE); MONTENEGRO, Elvira, 69469 Weinheim (DE); PARHAM, Amir, Hossain, 65929 Frankfurt am Main (DE); BUESING, Arne, 65929 Frankfurt am Main (DE); VOGES, Frank, 67098 Bad Duerkheim (DE)
(86) Internationale Anmeldenummer: PCT/EP2013/001892
(87) Internationale Veröffentlichungsnummer: WO 2014/015938

(56) Entgegenhaltungen:
- EP-A1- 2 314 565
- EP-A1- 2 415 752
- WO-A1-2004/020387
- WO-A1-2007/072838
- WO-A1-2007/086701
- DE-A1-102010 045 405
- JP-B- 3 824 385
- US-A1- 2012 161 615

## Beschreibung

Die vorliegende Erfindung betrifft neue organische Verbindungen, die Verwendung von Verbindung in einer elektrolumineszierenden Vorrichtung, sowie eine elektrolumineszierende Vorrichtung enthaltend wenigstens eine der Verbindungen. Weiterhin betrifft die vorliegende Erfindung ein Verfahren zur Herstellung der Verbindungen sowie Zusammensetzungen und Formulierungen enthaltend wenigstens eine der Verbindungen.

Die Entwicklung von funktionellen Verbindungen zur Verwendung in elektronischen Vorrichtungen ist aktuell Gegenstand intensiver Forschung. Ziel ist hierbei insbesondere die Entwicklung von Verbindungen, mit denen verbesserte Eigenschaften der elektrolumineszierenden Vorrichtungen in einem oder mehreren relevanten Punkten erzielt werden können, wie beispielsweise Leistungseffizienz, Lebensdauer oder Farbkoordinaten des emittierten Lichts.

Unter dem Begriff elektrolumineszierenden Vorrichtung werden gemäß der vorliegenden Erfindung unter anderem organische lichtemittierende Transistoren (OLETs), organische Feld-Quench-Devices (OFQDs), organische lichtemittierende elektrochemische Zellen (OLECs, LECs oder LEECs), organische Laserdioden (O-Laser) und organische lichtemittierende Dioden (OLEDs) verstanden.

Von besonderem Interesse ist die Bereitstellung von Verbindungen zur Verwendung in den zuletzt genannten, als OLEDs bezeichneten elektronischen Vorrichtungen. Der allgemeine Aufbau sowie das Funktionsprinzip von OLEDs ist dem Fachmann bekannt und unter anderem offenbart in US 4539507, US 5151629, EP 0676461 und WO 1998/27136.

Betreffend die Leistungsdaten von OLEDs sind noch weitere Verbesserungen erforderlich, insbesondere in Hinblick auf eine breite kommerzielle Verwendung, beispielsweise in Anzeigevorrichtungen oder als Lichtquellen. Von besonderer Bedeutung sind in diesem Zusammenhang die Lebensdauer, die Effizienz und die Betriebsspannung der OLEDs sowie die realisierten Farbwerte. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

In diesem Zusammenhang besteht insbesondere Bedarf an alternativen Lochtransportmaterialien. Bei Lochtransportmaterialien gemäß dem Stand der Technik steigt im Allgemeinen die Spannung mit der Schichtdicke der Lochtransportschicht an. In der Praxis wäre häufig eine höhere Schichtdicke der Lochtransportschicht wünschenswert, dies hat jedoch oftmals eine höhere Betriebsspannung und schlechtere Leistungsdaten zur Folge. In diesem Zusammenhang besteht Bedarf an neuen Lochtransportmaterialien, die eine hohe Ladungsträgerbeweglichkeit aufweisen, so dass dickere Lochtransportschichten mit lediglich geringem Anstieg der Betriebsspannung realisiert werden können.

Im Stand der Technik wird die Verwendung von verschiedenen Fluorenen als Ladungstransportmaterial in elektronischen und elektrolumineszierenden Vorrichtungen beschrieben.

JP 3824385 B2 offenbart in Position 2 und 7 substituiert Fluorene, die mit Dibenzofuranen oder Carbazolen substituiert sind.

US 2012/20012832 offenbart Fluorene, die mit kondensierten Aromaten substituiert sind.

WO 2004/020387 offenbart Fluorene, die in Position 2 mit einer Amingruppe substituiert sind, wobei die Amingruppe selbst zweimal mit jeweils einer Phenylgruppe substituiert ist.

In JP 05-303221 wird die Verwendung von 2- und 4-substituierten Fluorenen als photosensitive Verbindung offenbart. Die Verwendung in elektrolumineszierenden Vorrichtungen wie OLEDs oder OLECs wird hierin nicht beschrieben.

EP2415752 A1 offenbart ähnliche Vorrichtungen und Verbindungen. Trotz der bereits bekannten Verbindungen besteht unverändert Bedarf an neuen Lochtransport- und Lochinjektionsmaterialien zur Verwendung in

OLEDs. Insbesondere besteht Bedarf an Materialien, mit denen die oben genannten, hoch erwünschten Verbesserungen der Leistungsdaten und Eigenschaften der OLEDs erreicht werden können.

Ebenfalls besteht Bedarf an neuen Matrixmaterialien zur Verwendung in OLEDs sowie in anderen elektronischen Vorrichtungen. Insbesondere besteht Bedarf an Matrixmaterialien für phosphoreszierende Dotanden sowie an Matrixmaterialien für Mixed-Matrix-Systeme, welche bevorzugt zu guter Effizienz, hoher Lebensdauer und geringer Betriebsspannung der elektronischen Vorrichtungen führen.

Der vorliegenden Erfindung liegt somit die Aufgabe zu Grunde, elektroluminesziernede Vorrichtungen und Verbindungen, welche sich zur Verwendung in elektrolumineszierenden Vorrichtungen wie beispielsweise OLEDs eignen, bereitzustellen und welche insbesondere als Lochtransportmaterialien und/oder als Lochinjektionsmaterialien und/oder als Matrixmaterialien eingesetzt werden können.

Im Rahmen der vorliegenden Erfindung wurde überraschend gefunden, dass sich Verbindungen der unten angegebenen Formel (1) ausgezeichnet für die oben genannten Verwendungen in elektroluminesziernden Vorrichtungen eignen.

Gegenstand der Erfindung ist somit eine elektrolumineszierende Vorrichtung enthaltend wenigstens eine Verbindung der Formel (1) 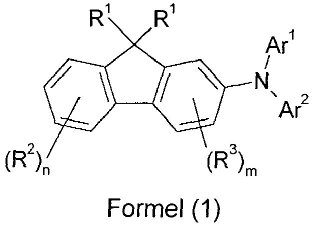 wobei für die verwendeten Symbole und Indices gilt:
- Ar¹, Ar²: sind bei jedem Auftreten gleich oder verschieden ein Aromat oder Heteroaromat mit 10 bis 60 aromatischen Ringatomen, welche mit einem oder mehreren Resten R⁴, die gleich oder verschieden voneinander sind, substituiert sein können, wobei beide Gruppen Ar¹ oder Ar² jeweils mindestens zwei oder mehr aromatische oder heteroaromatische Ringe enthalten.
- R¹: ist bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵-, -C≡C-, Si(R⁵)₂, C=O, C=S, C=NR⁵, -C(=O)O-, -C(=O)NR⁵ P(=O)(R⁵), -O-, -S-, SO oder SO₂ ersetzt sein können und wobei ein oder mehrere H-Atome in den oben genannten Gruppen durch D, F, Cl, Br, I; CN oder NO₂ ersetzt sein können, oder ein aromatisches oder heteroaro-matisches Ringsystem mit 6 bis 30 Ringatomen, das jeweils durch einen oder mehrere Reste R⁵ substituiert sein kann, oder ein kondensiertes Ringsystem mit 9 bis 30 Ringatomen, das jeweils durch einen oder mehrere Reste R⁵ substituiert sein kann, wobei im Fall aromatischer oder heteroaromatischer kondensierter Ringe nicht mehr als 10 Ringatome enthalten sein dürfen; beide Reste R¹ können auch miteinander einen Ringschluss bilden können, so dass eine Spiro Verbindung entsteht, wobei an den durch die beiden Reste R¹ gebildeten Ring keine aromatischen oder heteroaromatischen Ringe kondensiert sind;
- R², R³ und R⁴: sind bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵-, -C=C-, Si(R⁵)₂, C=O, C=S, C=NR⁵, -C(=O)O-, -C(=O)NR⁵-, P(=O)(R⁵), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 Ringatomen, das jeweils durch einen oder mehrere Reste R⁵ substituiert sein kann;
- R⁵: ist bei jedem Auftreten gleich oder verschieden H, D, F, Cl, Br, I, C(=O)R⁶, CN, Si(R⁶)₃, NO₂, P(=O)(R⁶)₂, S(=O)_{R}⁶, S(=O)₂R⁶, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁶ substituiert sein können und wobei eine oder mehrere CH₂-Gruppen in den oben genannten Gruppen durch -R⁶C=CR⁶-, -C≡C-, Si(R⁶)₂, C=O, C=S, C=NR⁶, -C(=O)O-, -C(=O)NR⁶-, P(=O)(R⁶), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁶ substituiert sein kann, oder eine Aryloxy- oder Heteroaryloxygruppe mit 5 bis 30 aromatischen Ringatomen, die durch einen oder mehrere Reste R⁶ substituiert sein kann;
- R⁶: ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können;
- n: ist 0, 1, 2, 3 oder 4;
- m: ist 0, 1, 2 oder 3;
mit der Maßgabe, dass die Verbindung nach Formel (1) neben der einen Fluoren Gruppe und neben den möglichen kondensierten oder polycyclischen Gruppen in Position 9 des Fluorens keine weiteren polycyclischen oder kondensierten Gruppen enthält.

Die Zählweise am Fluoren ist dabei wie folgt festgelegt.

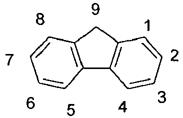

Bevorzugt ist eine elktrolumineszierende Vorrichtung, die wenigstens eine Verbindungen der Formel (1) enthält, wobei beide Reste R¹ identisch sind.

Es ist bevorzugt, wenn die elektrolumineszierende Vorrichtung wenigstens eine Verbindung der Formel (1) enthält, die dadurch charakterisiert ist, dass m gleich 1 oder 0 ist, ganz bevorzugt ist m gleich 0.

Es ist weiterhin bevorzugt, wenn die elektrolumineszierende Vorrichtung wenigstens eine Verbindung der Formel (1) enthält, die dadurch charakterisiert ist, dass n gleich 2, 1 oder 0 ist, ganz bevorzugt ist n gleich 0 oder 1.

Bevorzugt ist die Verbindung der Formel (1) ausgewählt aus einer Verbindung der Formel (2), 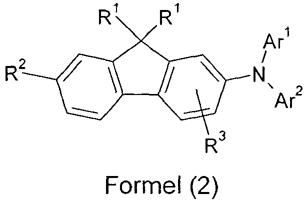 wobei die Symbole wie oben angegeben definiert sind.

Ganz bevorzugt ist eine Verbindung nach Formel (2), wobei beide Reste R¹ identische sind.

In einer weiterhin bevorzugten Ausführungsform der vorliegenden Erfindung enthält die elektrolumineszierende Vorrichtung wenigstens eine Verbindung der Formel (3), wobei weiterhin bevorzugt eine Verbindung der Formel (3) ist bei der die Reste R¹ identisch sind.

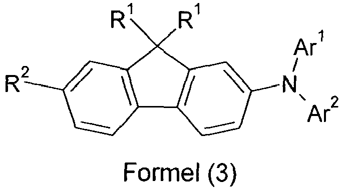

In einer ganz bevorzugten Ausführungsform der vorliegenden Erfindung ist R² gleich H oder eine geradkettige Alkylgruppe mit 1 bis 20 C-Atomen oder eine verzweigte oder cyclische Alkylgruppe mit 3 bis 20 C-Atomen, wobei die oben genannten Gruppen jeweils mit einem oder mehreren Resten R⁵ substituiert sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R⁵ substituiert sein kann;

Besonders bevorzugt ist R² gleich H oder ein aromatisches Ringsystem mit 6 bis 30 aromatischen Ringatomen, das jeweils durch einen oder mehrere Reste R⁵ substituiert sein kann.

In einer ganz besonders bevorzugten Ausführungsform enthält die elektrolumineszierende Vorrichtung wenigstens eine Verbindung der Formel (3), wobei R² gleich H ist und beide R¹ gleich oder verschieden voneinander, bevorzugt gleich, ein aromatisches oder heteroaromatisches Ringsystem mit 6 bis 30 aromatischen Ringatomen, das jeweils durch einen oder mehrere Reste R⁵ substituiert sein kann.

Insbesondere bevorzugt ist R² in den Formeln (1) bis (3) gleich Phenyl, Biphenyl, Terphenyl, Quarterphenyl, die mit einem oder mehreren Resten R⁵ substituiert sein können, wobei weiterhin bevorzugt ist, wenn diese unsubstituiert sind, oder H.

In einer weiteren ganz besonders bevorzugten Ausführungsform enthält die elektrolumineszierende Vorrichtung wenigstens eine Verbindung der Formel (3), wobei R² ein aromatisches Ringsystem mit 6 bis 30 aromatischen Ringatomen ist, das jeweils durch einen oder mehrere Reste R⁵ substituiert sein kann und R¹ gleich oder verschieden voneinander, bevorzugt gleich, eine geradkettige Alkylgruppe mit 1 bis 20 C-Atomen oder eine verzweigte oder cyclische Alkylgruppe mit 3 bis 20 C-Atomen ist, wobei die oben genannten Gruppen jeweils mit einem oder mehreren Resten R⁵ substituiert sein können und wobei ein oder mehrere H-Atome in den oben genannten Gruppen durch D, F, Cl, Br, I, CN oder NO₂ ersetzt sein können.

Es ist weiterhin bevorzugt, wenn die elektrolumineszierende Vorrichtung wenigstens eine Verbindung der Formel (4) enthält, 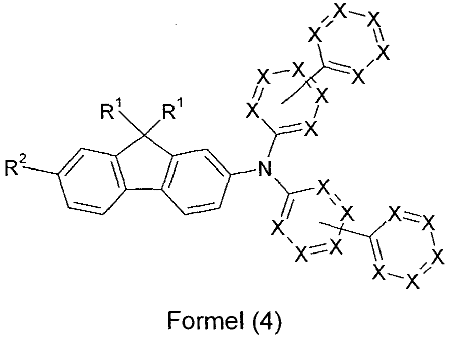 wobei X gleich oder verschieden bei jedem Auftreten N oder CR⁴ ist, wobei nur 3 der Gruppen X pro Ring N sein können. Es ist ganz bevorzugt, wenn X in Formel (4) gleich CR⁴ ist und wobei obige Definitionen für die Reste R¹, R² und R⁴ gelten.

Bevorzugte Reste Ar¹ und Ar² sind ausgewählt aus der in der folgenden Tabelle aufgeführten Reste mit den Formeln (5) bis (60), wobei die Reste, wie oben bereits angegeben, mit einem oder mehreren Resten R⁴, die gleich oder verschieden voneinander sind, substituiert sein können;

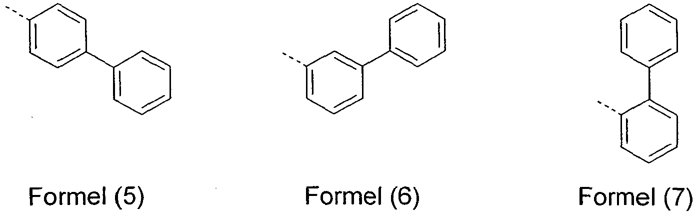

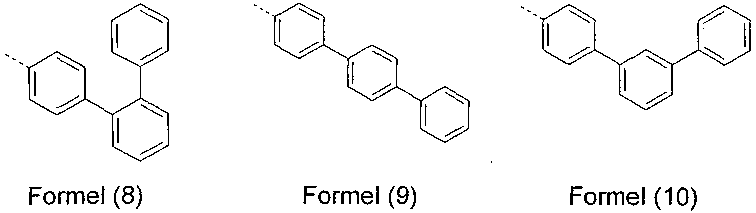

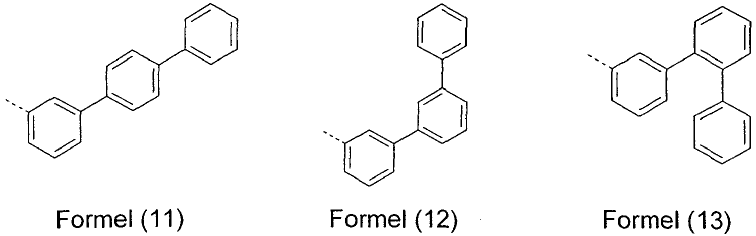

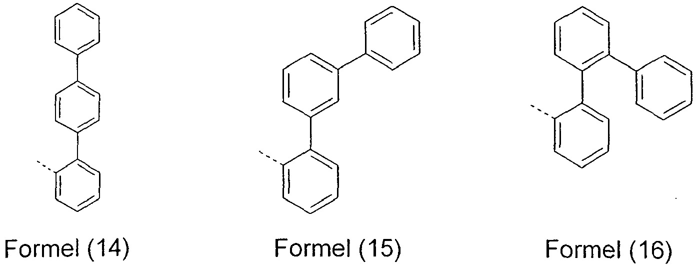

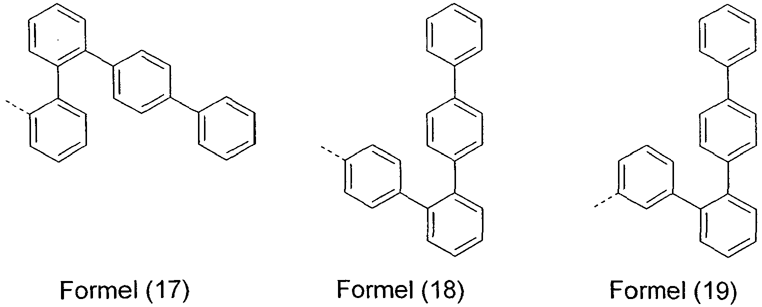

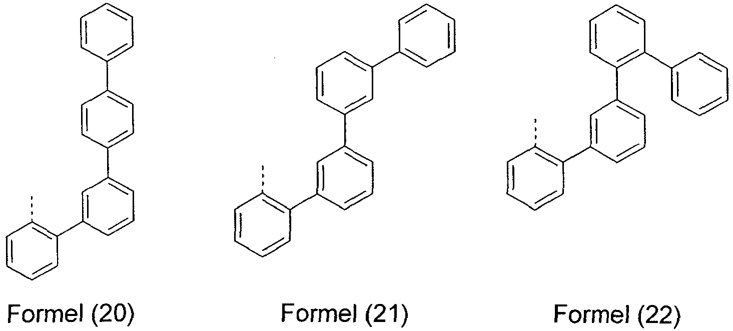

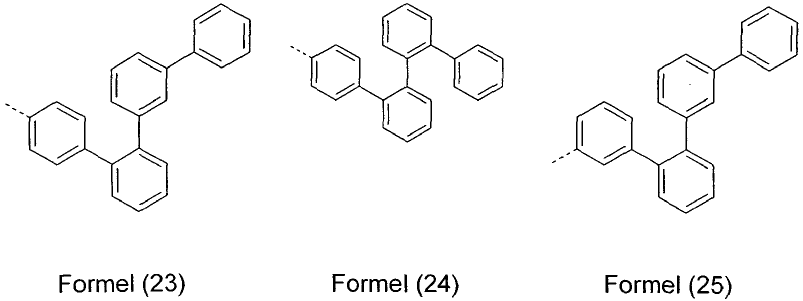

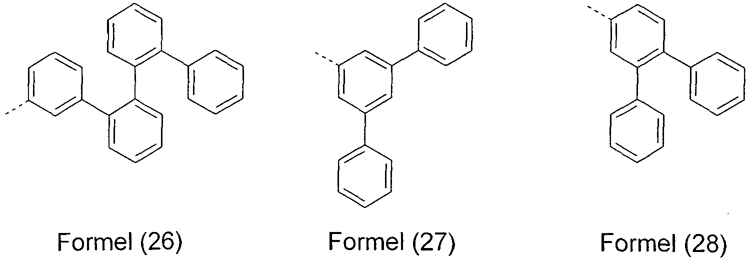

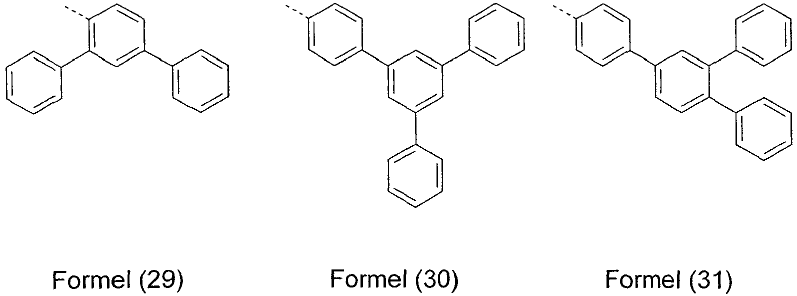

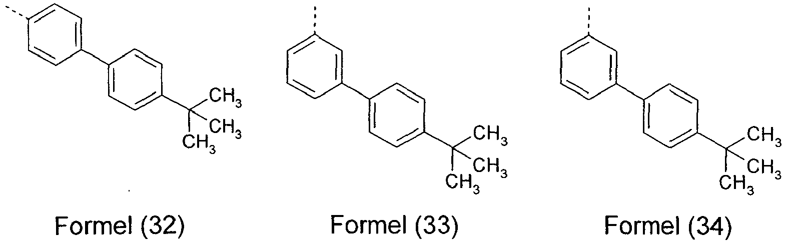

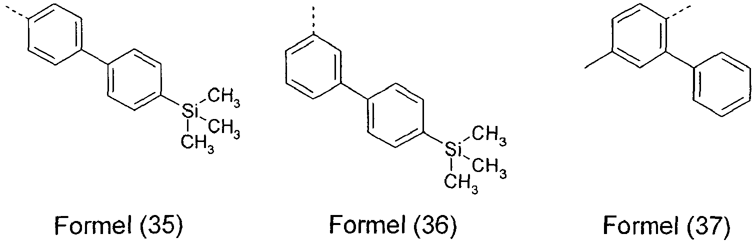

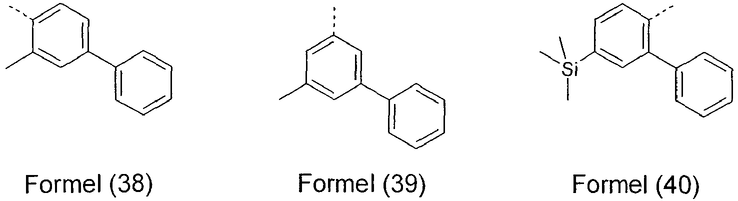

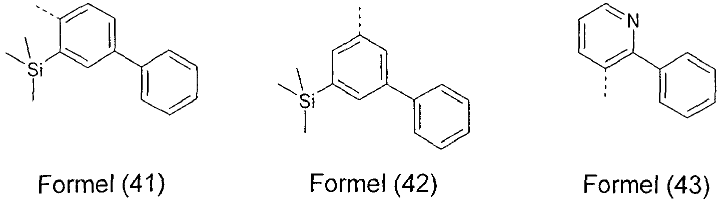

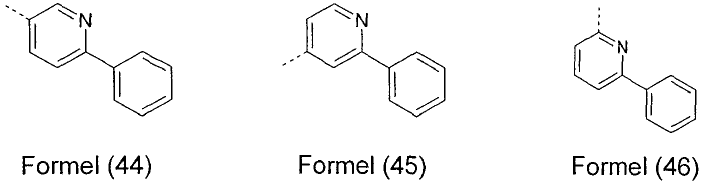

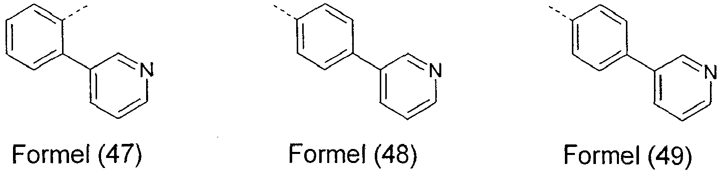

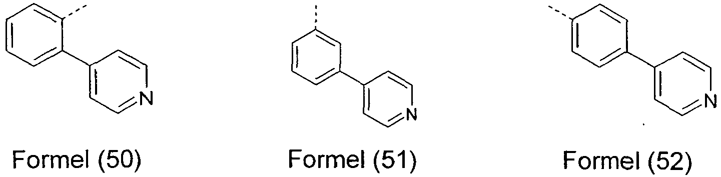

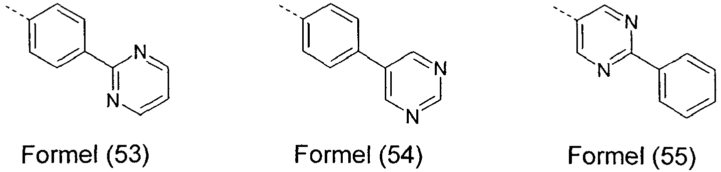

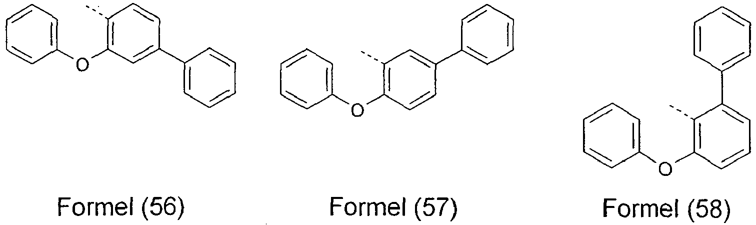

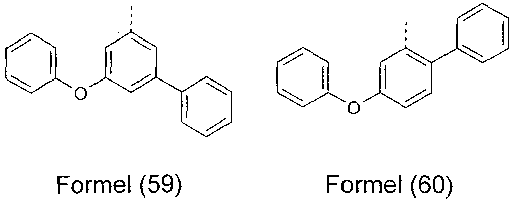

Bevorzugt im Sinne der vorliegenden Erfindung ist eine elktrolumineszierende Vorrichtung, die wenigstens eine Verbindungen der Formel (1) enthält, wobei Ar¹ und Ar² lediglich aromatische Ringe, nicht aber heteroaromatische Ringe enthält.

Insbesondere bevorzugt sind Ar¹ und Ar², gleich oder verschieden, Biphenyl, Terphenyl, Quarterphenyl, die mit einem oder mehreren Resten R⁴ substituiert sein können, wobei weiterhin bevorzugt ist, wenn diese unsubstituiert sind.

In einer weiterhin ganz bevorzugten Ausführungsform der vorliegenden Erfindung gilt für die Verbindung nach Formel (1), dass
- beide Reste R¹: identisch sind und ausgewählt werden aus einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, wobei die oben genannten Gruppen jeweils mit einem oder mehreren Resten R⁵ substituiert sein können und wobei ein oder mehrere H-Atome in den oben genannten Gruppen durch D, F, Cl, Br, I, CN oder NO₂ ersetzt sein können, oder einem aromatischen oder heteroaromatischen Ringsystem mit 6 bis 30 Ringatomen, das jeweils durch einen oder mehrere Reste R⁵ substituiert sein kann, oder ein kondensiertes Ringsystem mit 9 bis 30 Ringatomen, das jeweils durch einen oder mehrere Reste R⁵ substituiert sein kann, wobei im Fall aromatischer oder heteroaromatischer kondensierter Ringe nicht mehr als 10 Ringatome enthalten sein dürfen; beide Reste R¹ können auch miteinander einen Ringschluss bilden können, so dass eine Spiro Verbindung entsteht, wobei an den durch die beiden Reste R¹ gebildeten Ring keine aromatischen oder heteroaromatischen Ringe kondensiert sind;
- n: gleich 1 ist und der Rest R² ist in Position 7 des Fluorens;
- m: gleich 0 ist;
- R²: gleich eine Alkylgruppe mit 1 bis 20 C-Atomen oder eine verzweigte oder cyclische Alkylgruppe mit 3 bis 20 C-Atomen, eine Pyridyl-, Phenyl-, Biphenyl-, Terphenyl-, Quarterphenylgruppe, wobei die Gruppen mit einem oder mehreren Resten R⁵ substituiert sein können, wobei weiterhin bevorzugt ist, wenn die aromatischen oder die heteroaromatische Gruppe unsubstituiert sind, oder H ist;
- Ar¹ und Ar²,: gleich oder verschieden sind und ausgewählt werden aus Biphenyl, Terphenyl, Quarterphenyl, die mit einem oder mehreren Resten R⁴ substituiert sein können, wobei weiterhin bevorzugt ist, wenn diese unsubstituiert sind.

In einer weiterhin noch ganz bevorzugten Ausführungsform der vorliegenden Erfindung gilt für die Verbindung nach Formel (1), dass
- beide Reste R¹: identisch sind und ausgewählt werden aus einer geradkettigen Alkylgruppe mit 1 bis 20 C-Atomen oder einer verzweigten oder cyclische Alkylgruppe mit 3 bis 20 C-Atomen, wobei die Gruppen jeweils mit einem oder mehreren Resten R⁵ substituiert sein können und wobei ein oder mehrere H-Atome in den oben genannten Gruppen durch D, F, Cl, Br, I, CN oder NO₂ ersetzt sein können;
- n: gleich 1 ist und der Rest R² ist in Position 7 des Fluorens;
- m: gleich 0 ist;
- R²: gleich H oder eine Alkylgruppe mit 1 bis 20 C-Atomen oder eine verzweigte oder cyclische Alkylgruppe mit 3 bis 20 C-Atomen, wobei die Gruppen mit einem oder mehreren Resten R⁵ substituiert sein können, wobei R2 gleich H bevorzugt ist;
- Ar¹ und Ar²,: gleich oder verschieden sind und ausgewählt werden aus Biphenyl, Terphenyl, Quarterphenyl, die mit einem oder mehreren Resten R⁴ substituiert sein können, wobei weiterhin bevorzugt ist, wenn diese unsubstituiert sind.

In einer weiterhin noch ganz bevorzugten Ausführungsform der vorliegenden Erfindung gilt für die Verbindung nach Formel (1), dass
- beide Reste R¹: identisch sind und ausgewählt werden aus einer geradkettigen Alkylgruppe mit 1 bis 20 C-Atomen oder einer verzweigten oder cyclische Alkylgruppe mit 3 bis 20 C-Atomen, wobei die Gruppen jeweils mit einem oder mehreren Resten R⁵ substituiert sein können und wobei ein oder mehrere H-Atome in den oben genannten Gruppen durch D, F, Cl, Br, I, CN oder NO₂ ersetzt sein können;
- n: gleich 1 ist und der Rest R² ist in Position 7 des Fluorens;
- m: gleich 0 ist;
- R²: gleich Pyridyl-, Phenyl-, Biphenyl-, Terphenyl-, Quarterphenylgruppe ist, wobei die Gruppen mit einem oder mehreren Resten R⁵ substituiert sein können, wobei weiterhin bevorzugt ist, wenn die aromatischen oder die heteroaromatische Gruppe unsubstituiert sind;
- Ar¹ und Ar²,: gleich oder verschieden sind und ausgewählt werden aus Biphenyl, Terphenyl, Quarterphenyl, die mit einem oder mehreren Resten R⁴ substituiert sein können, wobei weiterhin bevorzugt ist, wenn diese unsubstituiert sind

In einer weiterhin noch ganz bevorzugten Ausführungsform der vorliegenden Erfindung gilt für die Verbindung nach Formel (1), dass
- beide Reste R¹: identisch sind und ausgewählt werden aus einem aromatischen oder heteroaromatischen Ringsystem mit 6 bis 30 Ringatomen, das jeweils durch einen oder mehrere Reste R⁵ substituiert sein kann, oder ein kondensiertes Ringsystem mit 9 bis 30 Ringatomen, das jeweils durch einen oder mehrere Reste R⁵ substituiert sein kann, wobei im Fall aromatischer oder heteroaromatischer kondensierter Ringe nicht mehr als 10 Ringatome enthalten sein dürfen;
- n: gleich 1 ist und der Rest R² ist in Position 7 des Fluorens;
- m: gleich 0 ist;
- R²: gleich eine Alkylgruppe mit 1 bis 20 C-Atomen oder eine verzweigte oder cyclische Alkylgruppe mit 3 bis 20 C-Atomen, eine Pyridyl-, Phenyl-, Biphenyl-, Terphenyl-, Quarterphenylgruppe, wobei die Gruppen mit einem oder mehreren Resten R⁵ substituiert sein können, wobei weiterhin bevorzugt ist, wenn die aromatischen oder die heteroaromatische Gruppe unsubstituiert sind, oder H ist, wobei R² gleich H bevorzugt ist;
- Ar¹ und Ar²,: gleich oder verschieden sind und ausgewählt werden aus Biphenyl, Terphenyl, Quarterphenyl, die mit einem oder mehreren Resten R⁴ substituiert sein können, wobei weiterhin bevorzugt ist, wenn diese unsubstituiert sind.

Weiterhin bevorzugt im Sinne der vorliegenden Erfindung ist eine elktrolumineszierende Vorrichtung, die wenigstens eine Verbindungen der Formel (1) enthält, die neben der einen Fluoren Gruppe keine weiteren polycyclischen oder kondensierten Gruppen enthält.

Die Synthese der erfindungsgemäßen Verbindungen kann nach Verfahren hergestellt werden, die dem Fachmann aus dem Stand der Technik bekannt sind. Die Herstellung kann, beispielsweise, mittels Halogenierung, Buchwald-Kupplung und Suzuki-Kupplung erfolgen.

Das folgende Schema zeigt einen bevorzugten Syntheseweg zur Herstellung der erfindungsgemäßen Verbindungen der Formel (1). Zur Synthese der erfindungsgemäßen Verbindungen wird die Fluoren-Verbindung **A** in einer Buchwald-Kupplung mit einem Amin **B** der Formel Ar¹-NH-Ar² umgesetzt

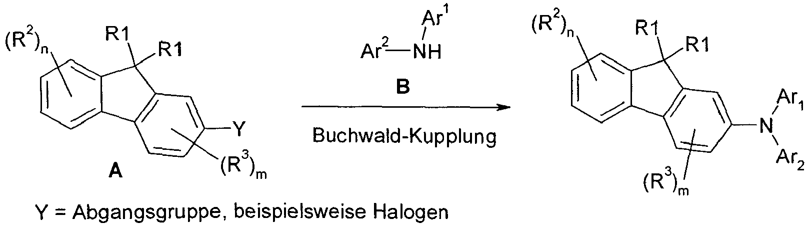

Ein anderer bevorzugter Syntheseweg zur Herstellung der erfindungsgemäßen Verbindungen wird im folgenden Schema dargestellt. Die Carbonsäureestergruppen in Verbindung **C** werden durch Addition einer Alkyl- oder Arylmetallverbindung, beispielsweise einer Alkyl- oder Aryllithium-Verbindung oder einer Alkyl- oder Aryl-Grignardverbindung, zum entsprechenden Alkohol **D** umgesetzt. Dieser kann unter sauren Bedingungen zu Verbindung **E** cyclisiert werden. Schließlich erfolgt eine Buchwald-Kupplung mit einem Amin **B** der Formel Ar¹-NH-Ar²

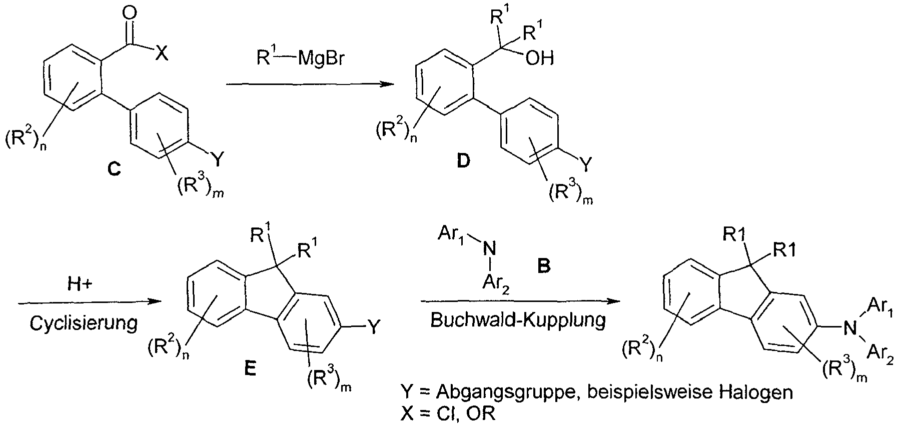

Das folgende Schema zeigt einen weiteren bevorzugten Syntheseweg zur Herstellung erfindungsgemäßer Verbindungen. Hierzu wird das Fluoren **A** in einer Suzuki-Kupplung mit einem Boronsaüre **F** der Formel Ar³-B(OH)₂ umgesetzt. Die Bromierung der resultierenden Verbindungen mit, beispielweise, Brom gefolgt von einer Buchwald-Kupplung mit einem Amin der Formel Ar¹-NH-Ar² ergibt die entsprechenden erfindungsgemäßen Verbindungen.

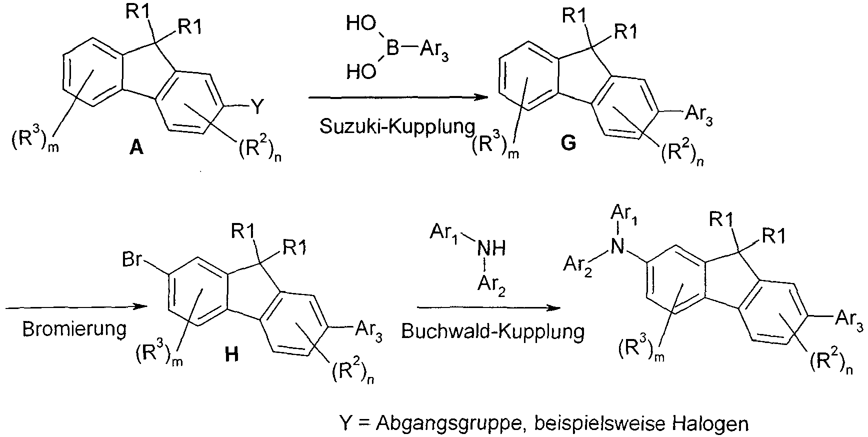

Synthesewege für die Ausgangsverbindungen A, B und C, welche in der Synthese der erfindungsgemäßen Verbindungen eingesetzt werden, sind dem Fachmann bekannt. Weiterhin werden in den Ausführungsbeispielen einige explizite Syntheseverfahren im Detail dargestellt.

Bevorzugte Kupplungsreaktionen sind hierbei die Buchwald-Kupplungen.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Die Oligomerisation bzw. Polymerisation erfolgt dabei bevorzugt über die Halogenfunktionalität bzw. die Boronsäurefunktionalität.

Bevorzugte Verbindungen, die in den erfindungsgemäßen elektrolumineszierenden Vorrichtungen verwendet werden sind in der nachfolgenden Tabelle exemplarisch aufgeführt. 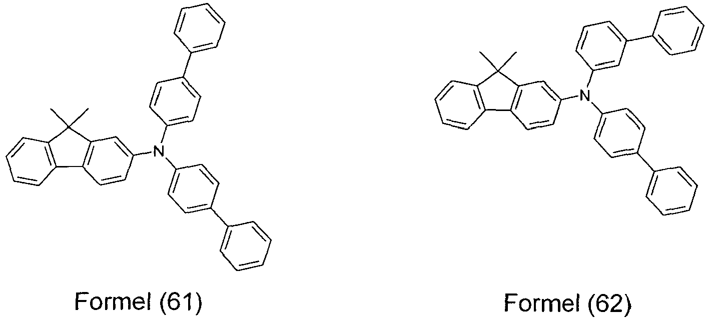 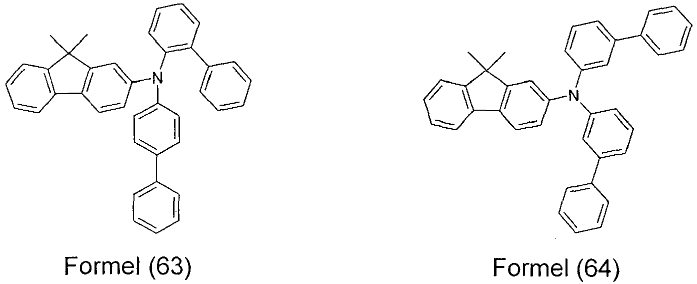 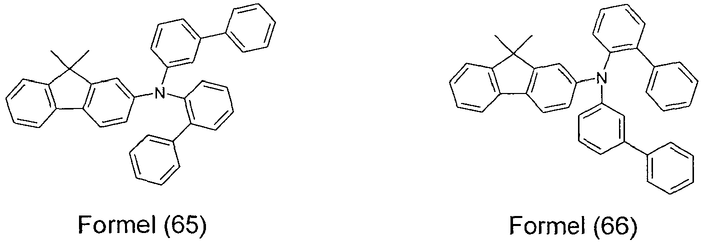 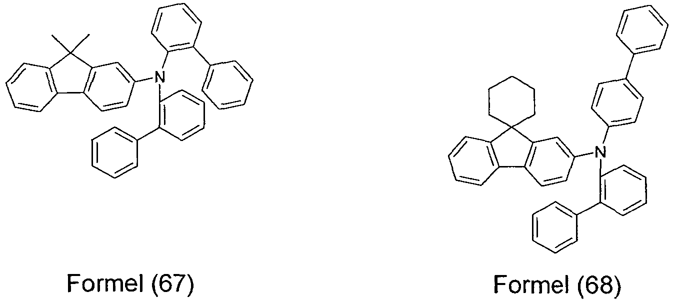 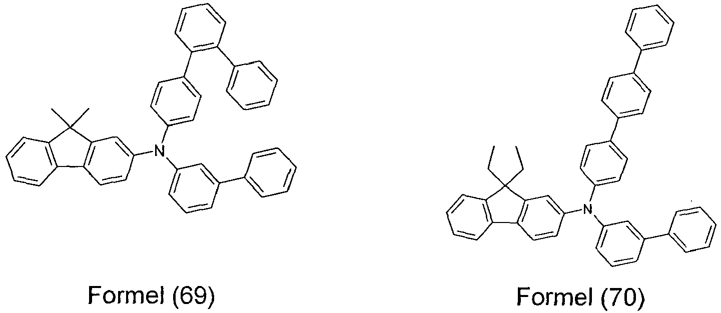 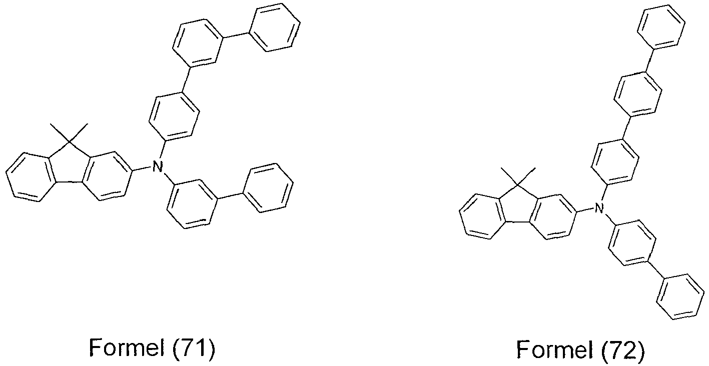 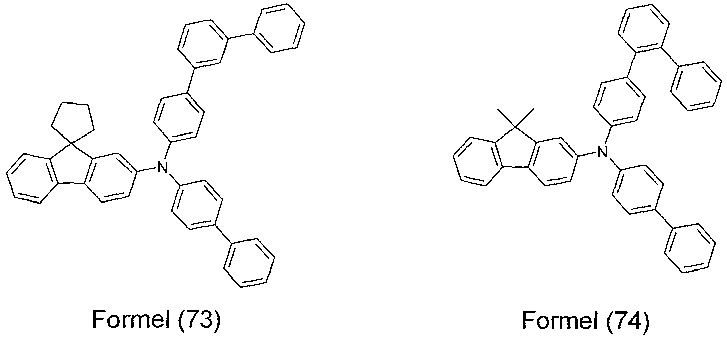 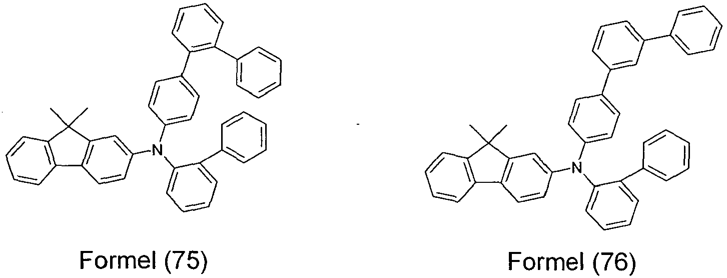 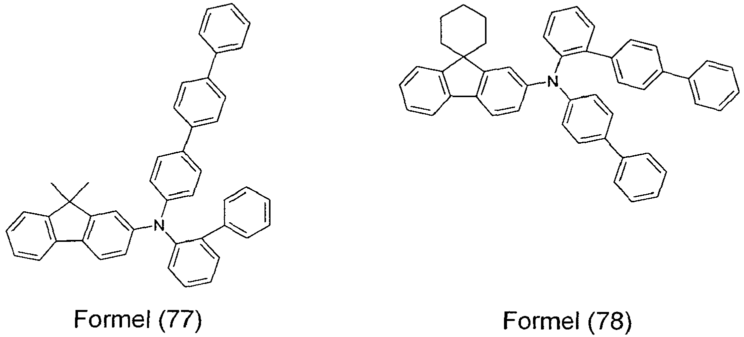 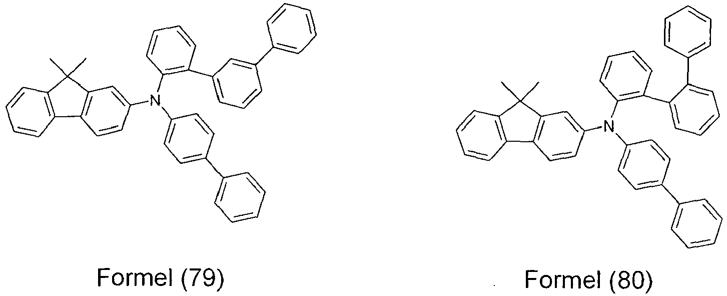 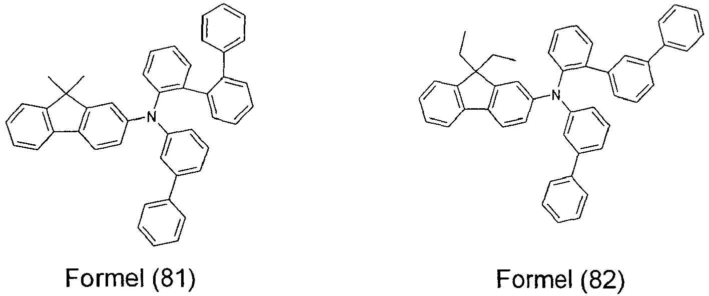 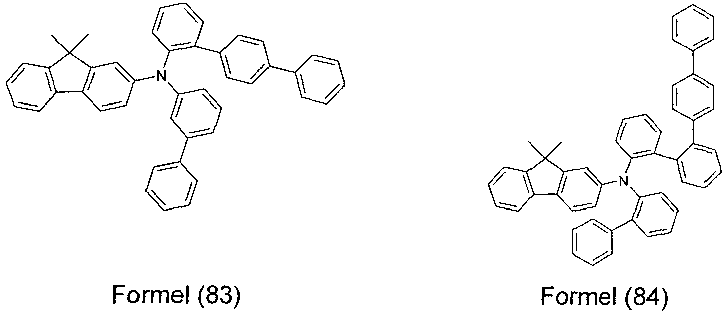 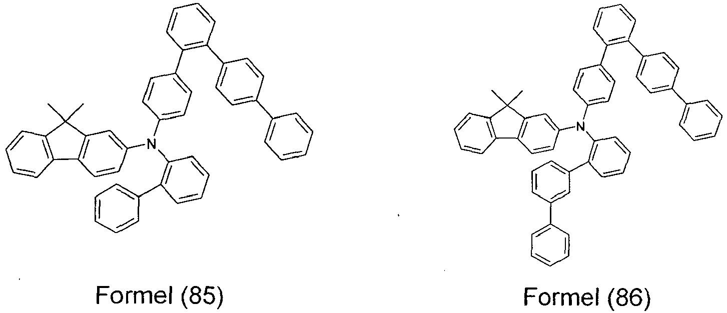 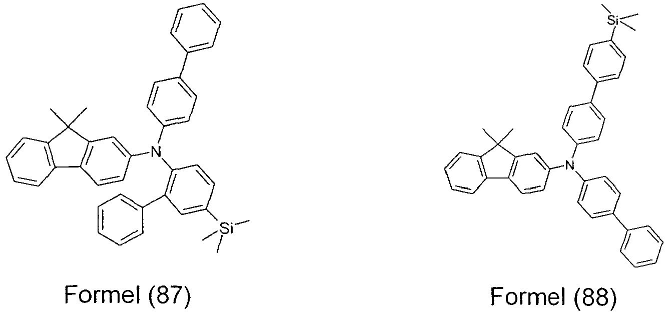 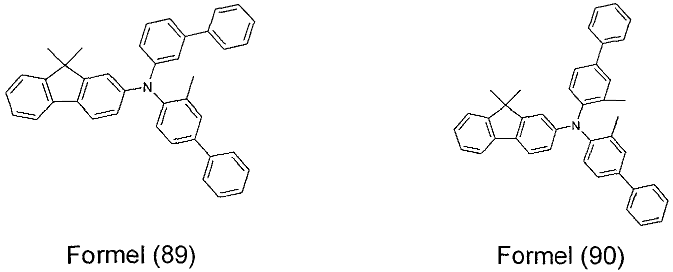 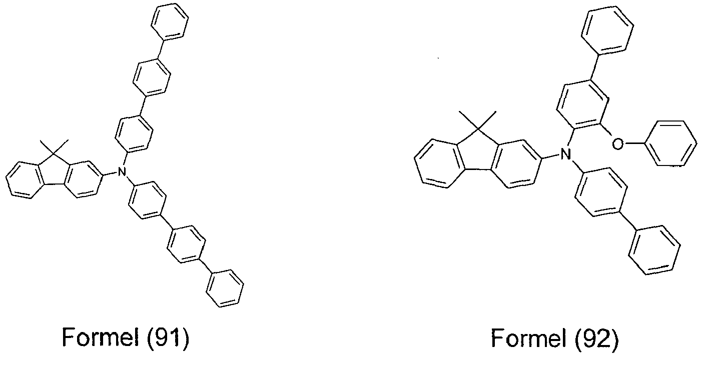 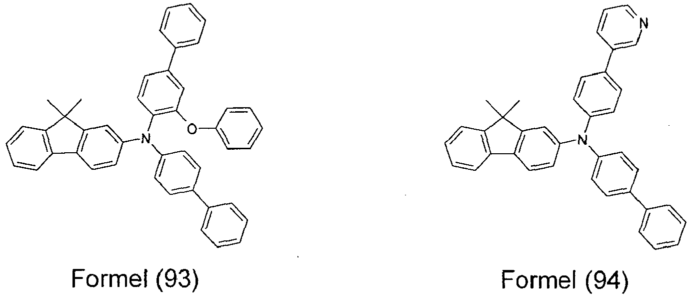 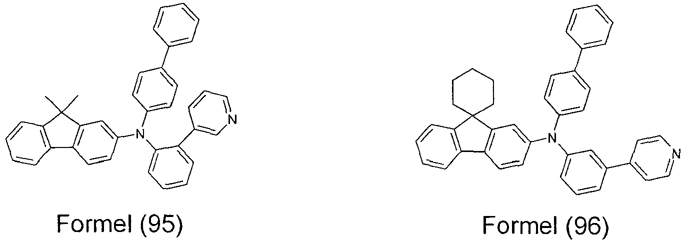 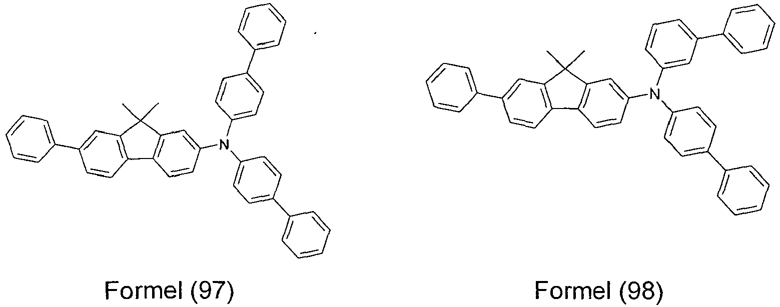 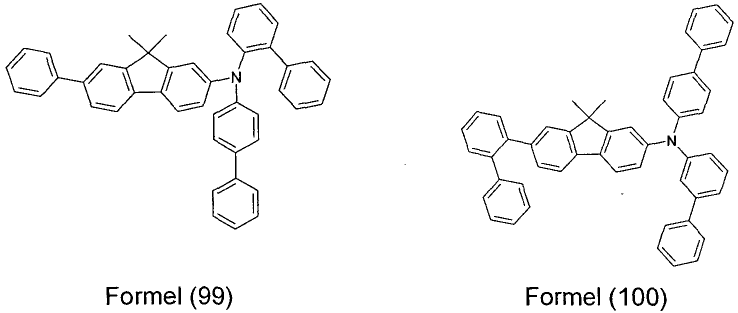 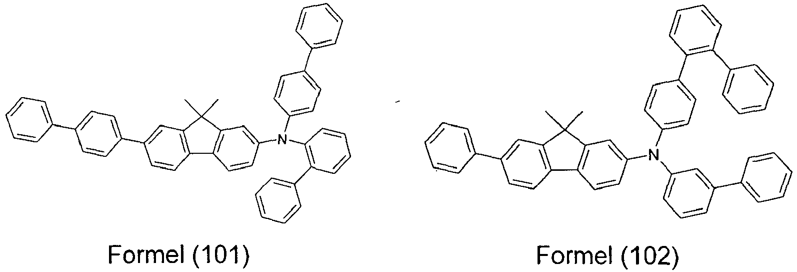 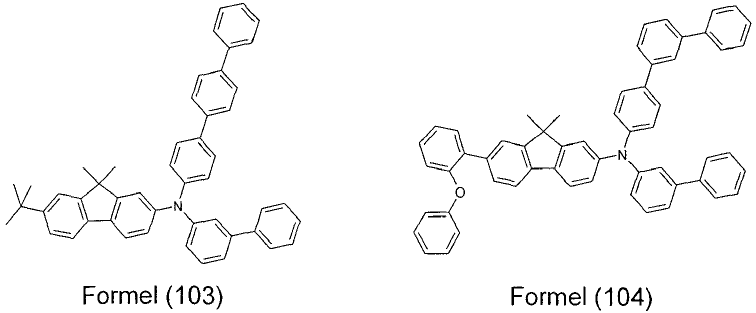 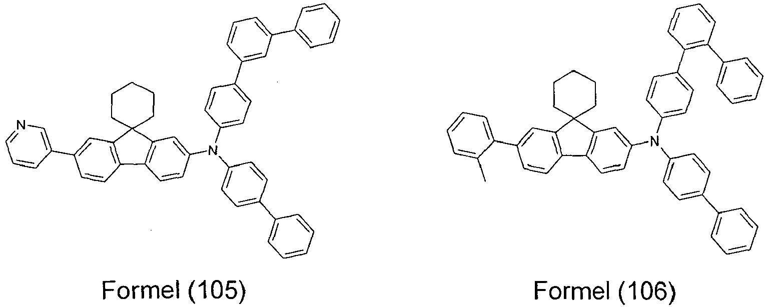 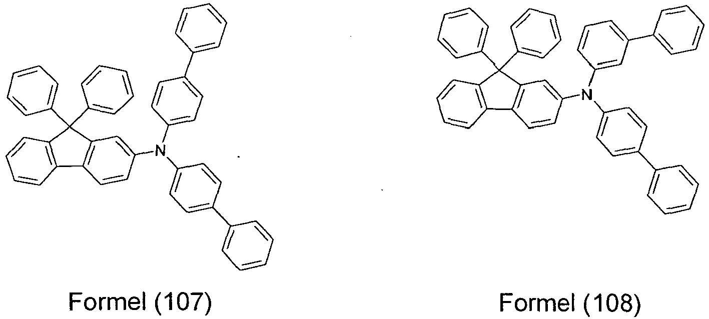 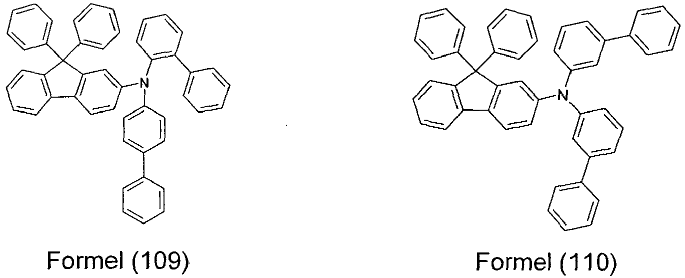 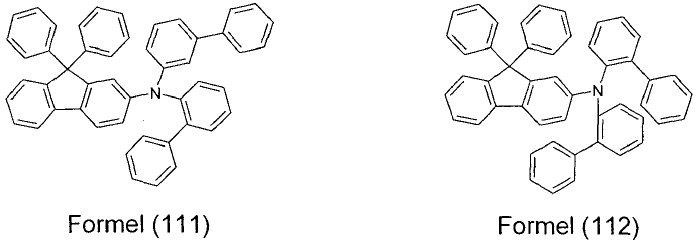 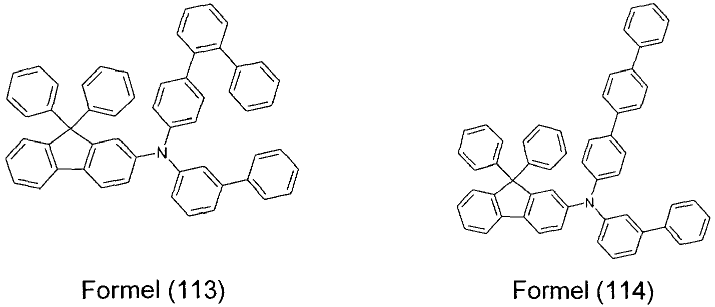 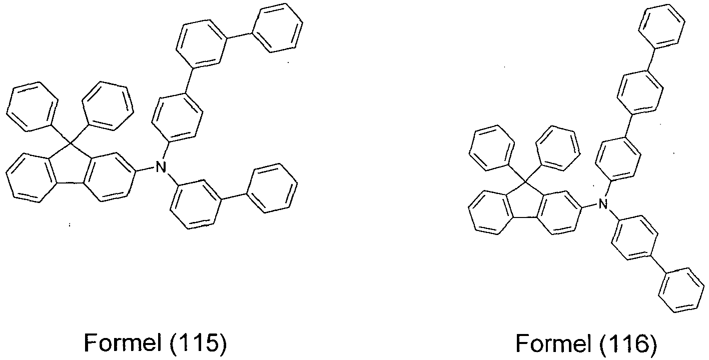 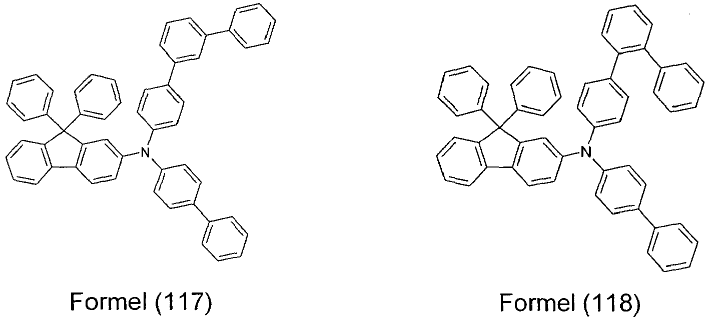 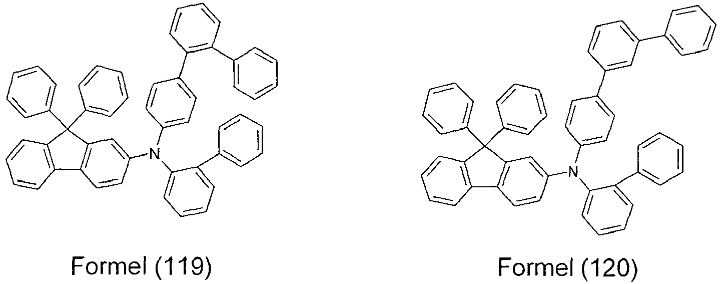 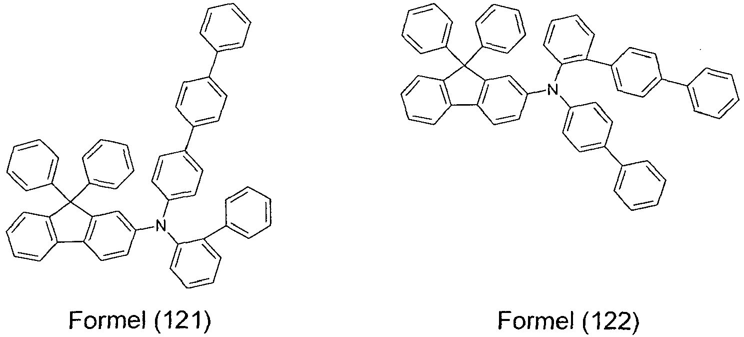 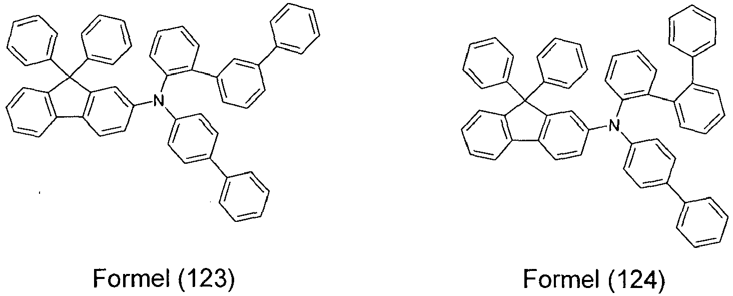 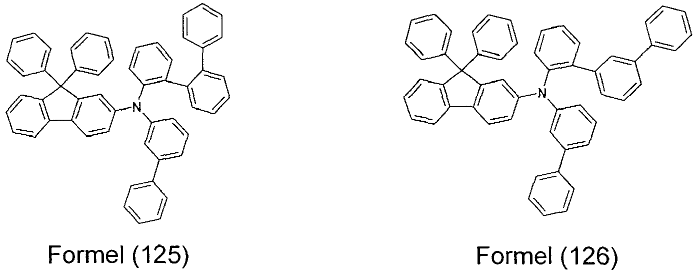 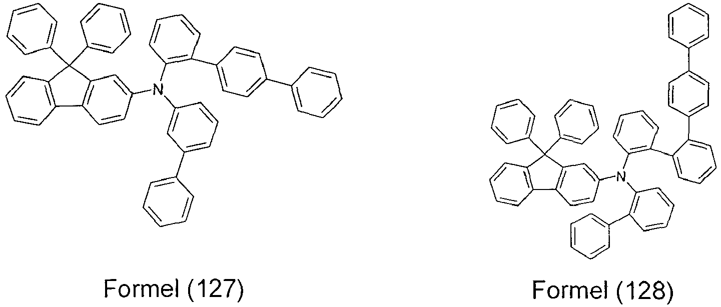 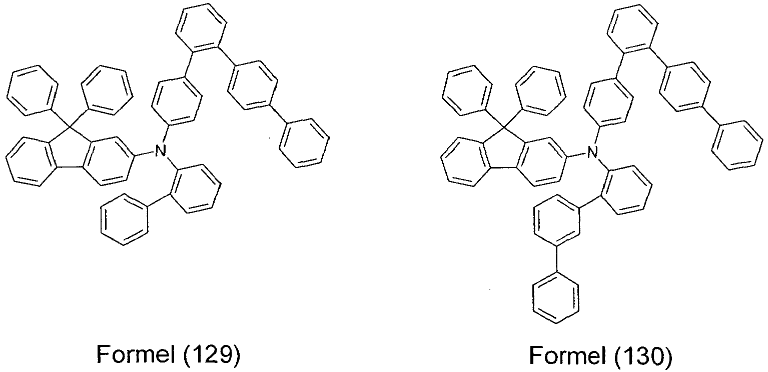 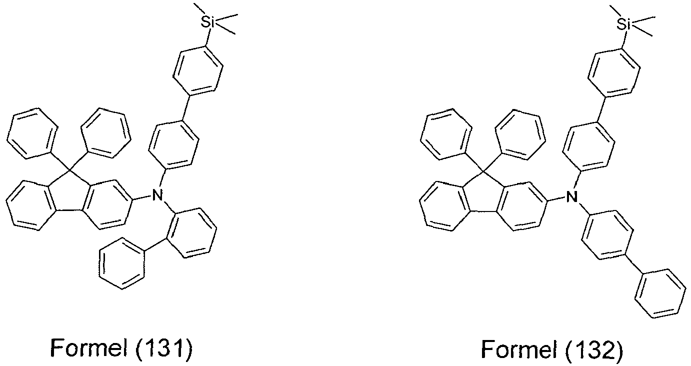 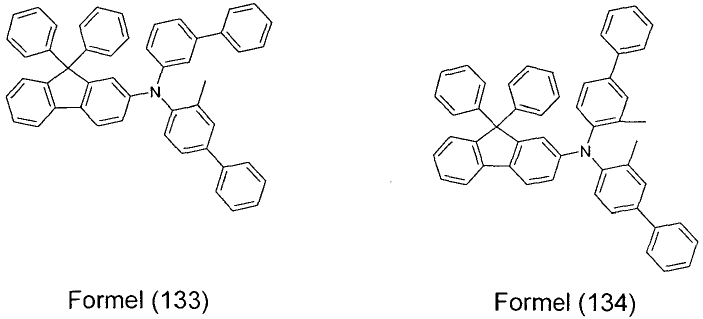 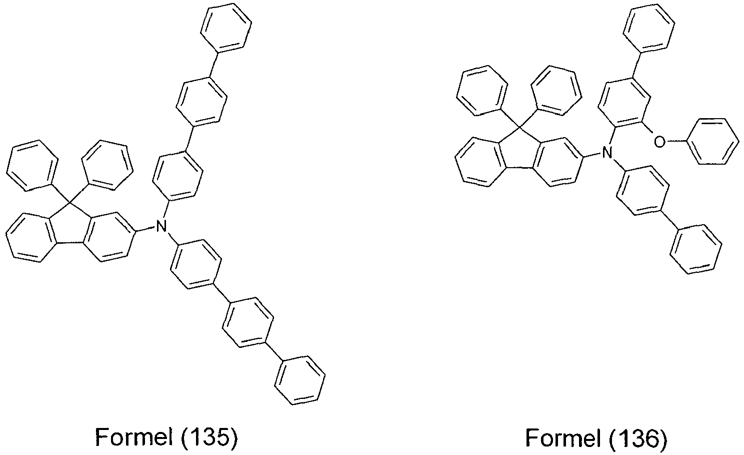 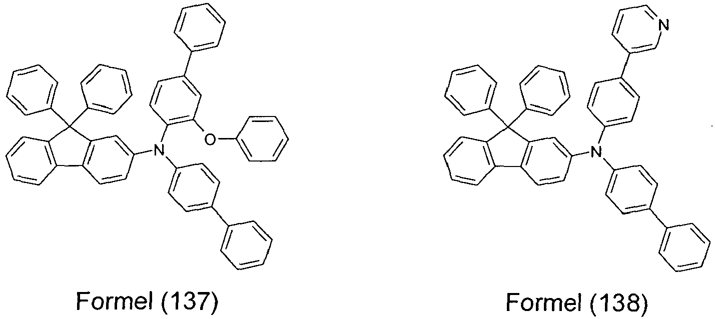 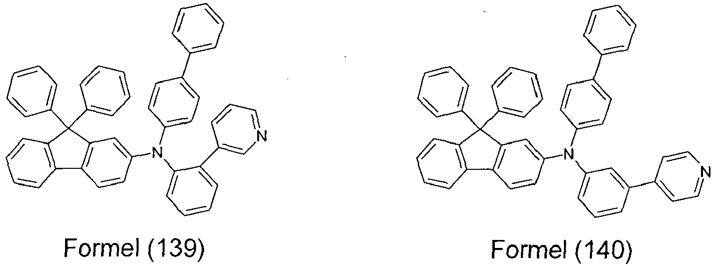 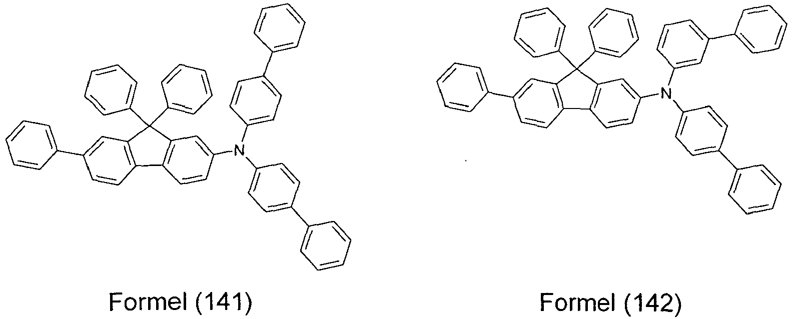 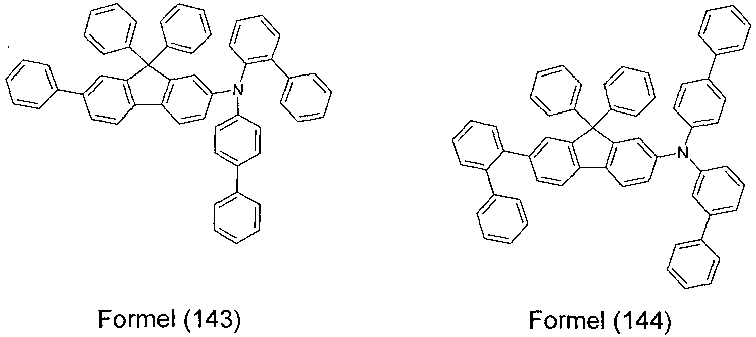 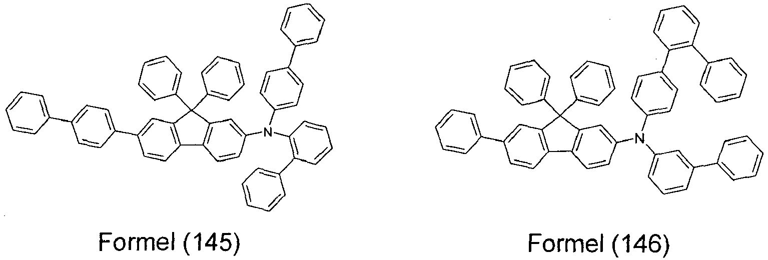 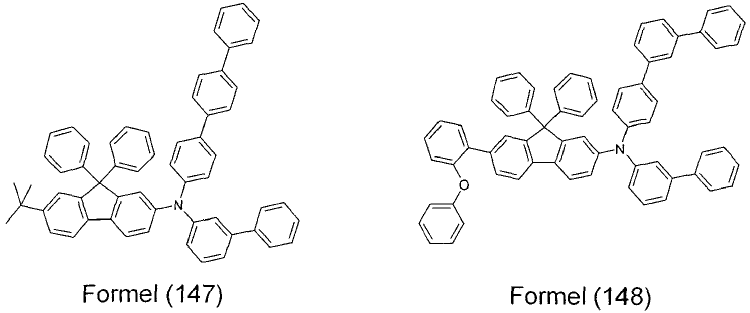 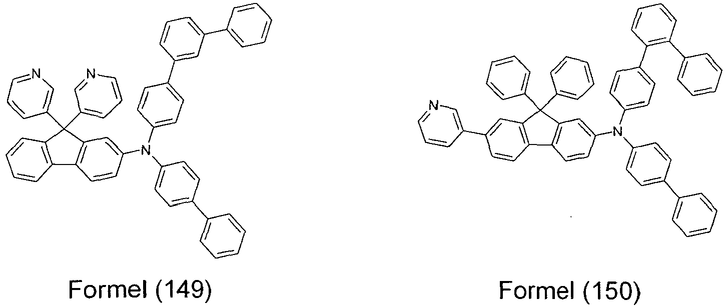 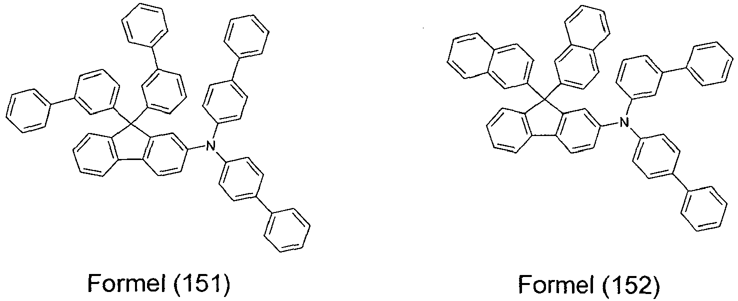 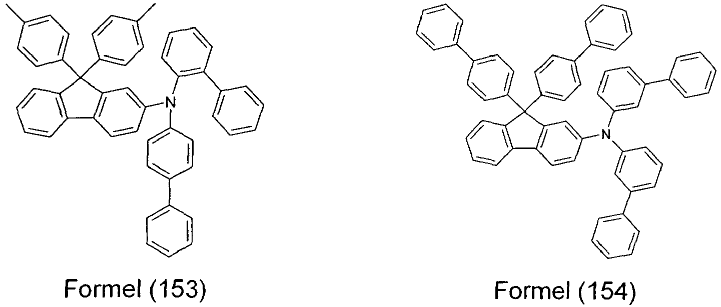 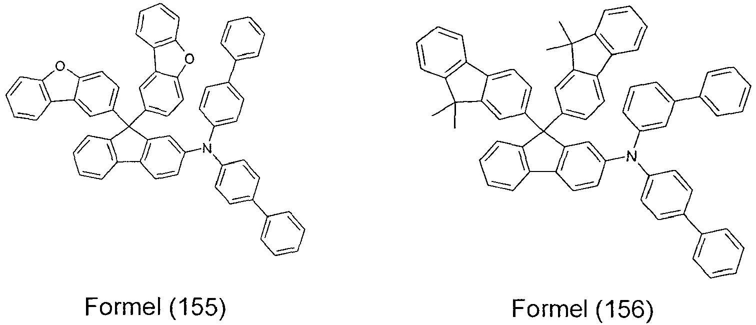 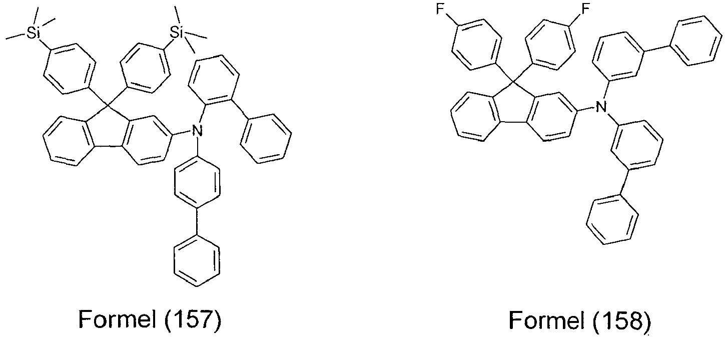 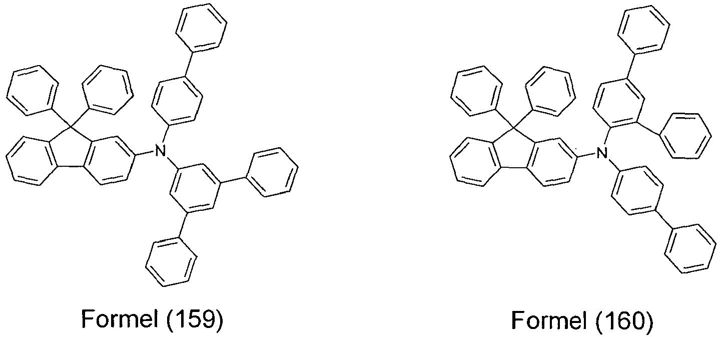 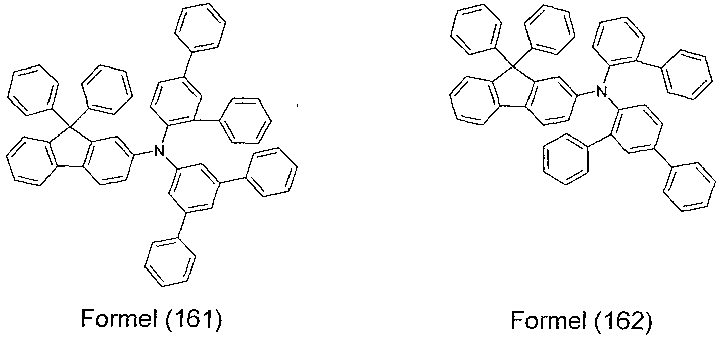 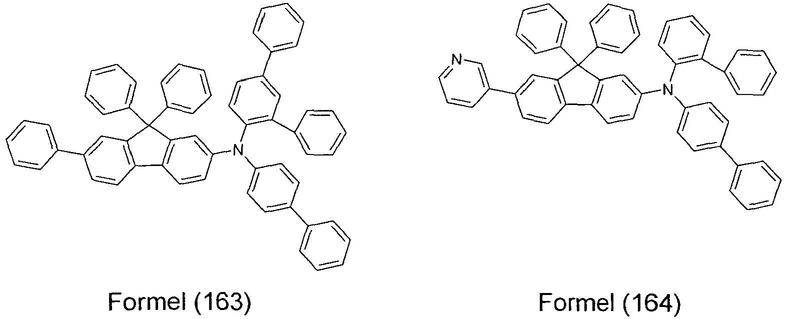 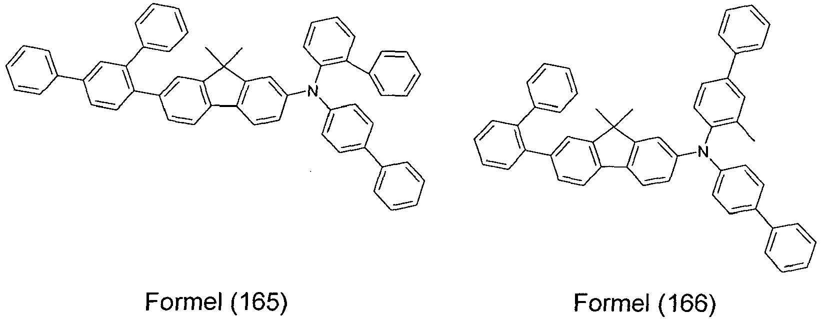 Die vorliegende Erfindung betrifft auch Verbindungen der allgemeinen Formel (167) 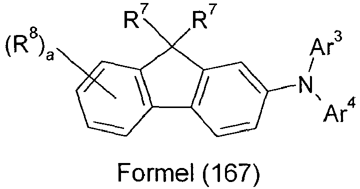 wobei für die verwendeten Symbole in Formel (167) gilt:
- Ar³, Ar⁴: sind bei jedem Auftreten gleich oder verschieden ein aromatisches oder heteroaromatisches Ringsystem mit 10 bis 60 Ringatomen, welche mit einem oder mehreren Resten R⁵, die gleich oder verschieden voneinander sind, substituiert sein können, wobei beide Gruppen Ar³ und Ar⁴ jeweils mindestens zwei oder mehr aromatische oder heteroaromatische, bevorzugt aromatische, Ringe enthalten;
- R⁷: ist gleich bei jedem Auftreten und ausgewählt aus der Gruppe bestehend aus einer geradkettigen Alkyl-, Alkoxy- oder Thioalkylgruppe mit 1 bis 20 C-Atomen oder einer verzweigte oder cyclische Alkyl-, Alkoxy- oder Thioalkylgruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R⁵ substituiert sein können und wobei ein oder mehrere H-Atome in den oben genannten Gruppen durch D, CN oder NO₂ ersetzt sein können, oder ein aromatisches oder heteroaromatisches Ringsystem mit 6 bis 30 Ringatomen, das jeweils durch einen oder mehrere Reste R⁵ substituiert sein kann, wobei R⁵ wie oben angegeben definiert ist oder ein kondensiertes Ringsystem mit 9 bis 30 Ringatomen, das jeweils durch einen oder mehrere Reste R⁵ substituiert sein kann, wobei im Fall aromatischer oder heteroaromatischer kondensierter Ringe nicht mehr als 10 Ringatome indem kondensierten Ringsystem enthalten sein dürfen; beide Reste R⁷ können auch miteinander einen Ringschluss bilden können, so dass eine Spiro Verbindung entsteht, wobei an den durch die beiden Reste R⁷ gebildeten Ring keine aromatischen oder heteroaromatischen Ringe kondensiert sind, und wobei, wenn R⁷ eine geradkettige oder verzweigte Alkylgruppe ist, R⁸ ein aromatisches oder heteroaromatisches Ringsystem mit 6 bis 30 Ringatomen ist, das jeweils durch einen oder mehrere Reste R⁵ substituiert sein kann und wobei R⁵ wie oben angegeben definiert;
- R⁸: H, D oder ein aromatisches oder heteroaromatisches Ringsystem mit 6 bis 30 Ringatomen, das jeweils durch einen oder mehrere Reste R⁵ substituiert sein kann, wobei R⁵ wie oben angegeben definiert ist und wobei wenn R⁸ gleich H ist, R⁷ ein aromatisches oder heteroaromatisches Ringsystem mit 6 bis 30 aromatischen Ringatomen ist, das jeweils durch einen oder mehrere Reste R⁵ substituiert sein kann, wobei R⁵ wie oben angegeben definiert ist;
- a: ist entweder 1, 2, 3 oder 4, bevorzugt 1 oder 2, ganz bevorzugt 1;
mit der Maßgabe, dass die Verbindung nach Formel (167) neben der einen Fluoren Gruppe und neben den möglichen kondensierten oder polycyclischen Gruppen in Position 9 des Fluorens keine weiteren polycyclischen oder kondensierten Gruppen enthält
und mit der Maßgabe, dass die Verbindung keine Halogene enthält.

Bevorzugt ist, wenn die Verbindung der Formel (167) neben der einen Fluoren Gruppe keine weiteren polycyclischen oder kondensierten Gruppen enthält.

Verbindungen der Formel (167) mit a = 1 und R⁸ in Position 7 des Fluorens sind bevorzugt, d.h. solche Verbindungen der Formel (168)

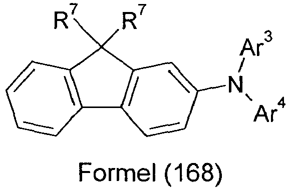

Besonders bevorzugt sind Verbindungen der Formeln (167) oder (168), wobei für die verwendeten Symbole gilt:
- Ar³, Ar⁴: sind bei jedem Auftreten gleich oder verschieden ausgewählt aus Biphenyl, Terphenyl, Quarterphenyl, die mit einem oder mehreren Resten R⁵ substituiert sein können, wobei weiterhin bevorzugt ist, wenn diese unsubstituiert sind;
- R⁷: ist gleich bei jedem Auftreten und ausgewählt aus einem aromatischen oder heteroaromatischen Ringsystem mit 6 bis 30 Ringatomen, das jeweils durch einen oder mehrere Reste R⁵ substituiert sein kann, wobei R⁵ wie oben angegeben definiert ist oder ein kondensiertes Ringsystem mit 9 bis 30 Ringatomen, das jeweils durch einen oder mehrere Reste R⁵ substituiert sein kann, wobei im Fall aromatischer oder heteroaromatischer kondensierter Ringe nicht mehr als 10 Ringatome indem kondensierten Ringsystem enthalten sein dürfen;
mit der Maßgabe, dass die Verbindung nach Formel (168) neben der einen Fluoren Gruppe und neben den möglichen kondensierten oder polycyclischen Gruppen in Position 9 des Fluorens keine weiteren polycyclischen oder kondensierten Gruppen enthält
und mit der Maßgabe, dass die Verbindung keine Halogene enthält.

Weiterhin bevorzugte Verbindungen der Formel (167) sind solche der allgemeinen Formel (169) 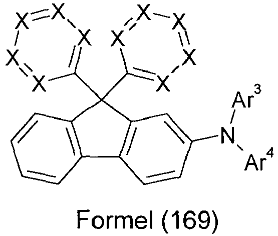 wobei X gleich oder verschieden bei jedem Auftreten N oder CR⁵ ist und R⁵, Ar³ und Ar⁴ wie oben angegeben definiert sind. Es ist bevorzugt, dass X in Formel (169) gleich CR⁵ ist.

Bevorzugt sind Ar¹ und Ar³ in Formel (169) bei jedem Auftreten gleich oder verschieden ausgewählt aus Biphenyl, Terphenyl, Quarterphenyl, die mit einem oder mehreren Resten R⁵ substituiert sein können, wobei weiterhin bevorzugt ist, wenn diese unsubstituiert sind.

Ganz bevorzugt sind Verbindungen der Formel (170)

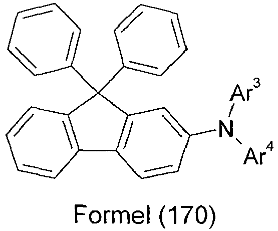

Bevorzugt sind Ar¹ und Ar³ in Formel (170) bei jedem Auftreten gleich oder verschieden ausgewählt aus Biphenyl, Terphenyl, Quarterphenyl, die mit einem oder mehreren Resten R⁵ substituiert sein können, wobei weiterhin bevorzugt ist, wenn diese unsubstituiert sind.

In einer weiteren bevorzugten Ausführungsform der vorliegenden Erfindung ist die Verbindung ausgewählt aus der allgemeinen Formel (171) 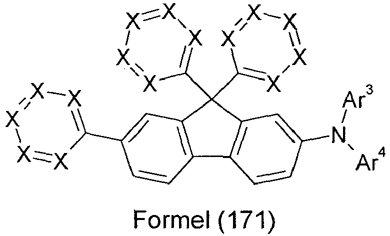 wobei für die verwendeten Symbole obige Definitionen gelten.
Bevorzugt sind Ar¹ und Ar³ in Formel (171) bei jedem Auftreten gleich oder verschieden ausgewählt aus Biphenyl, Terphenyl, Quarterphenyl, die mit einem oder mehreren Resten R⁵ substituiert sein können, wobei weiterhin bevorzugt ist, wenn diese unsubstituiert sind.

Weiterhin bevorzugte Verbindungen der Formel (5) sind Verbindungen der Formel (172) 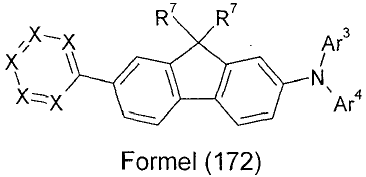 wobei für die verwendeten Symbole gilt:
- X: gleich oder verschieden bei jedem Auftreten N oder CR⁵ und bevorzugt CR⁵ ist und R⁵ wie oben angegeben definiert ist;
- R⁷: ist eine geradkettige Alkylgruppe mit 1 bis 20 C-Atomen oder eine verzweigte oder cyclische Alkylgruppe mit 3 bis 20 C-Atomen, wobei die oben genannten Gruppen jeweils mit einem oder mehreren Resten R⁵ substituiert sein können und wobei ein oder mehrere H-Atome in den oben genannten Gruppen durch D, CN oder NO₂ ersetzt sein können, wobei beide Reste R⁷ auch einen Ringschluss bilden können, so dass eine Spiro-Verbindung entsteht wobei an den durch die beiden Reste R⁷ gebildeten Ring keine aromatischen oder heteroaromatischen Ringe kondensiert sind;
und wobei Ar³ und Ar⁴ wie oben angegeben definiert sind.

Bevorzugt sind Ar¹ und Ar³ in Formel (172) bei jedem Auftreten gleich oder verschieden ausgewählt aus Biphenyl, Terphenyl, Quarterphenyl, die mit einem oder mehreren Resten R⁵ substituiert sein können, wobei weiterhin bevorzugt ist, wenn diese unsubstituiert sind.

In einer weiterhin bevorzugten Ausführungsform der vorliegenden Erfindung ist die Verbindung ausgewählt aus der allgemeinen Formel (173) 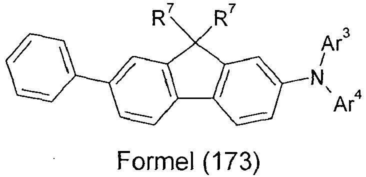 wobei für die verwendeten Symbole gilt:
- R⁷: ist eine geradkettige Alkylgruppe mit 1 bis 20 C-Atomen oder eine verzweigte oder cyclische Alkylgruppe mit 3 bis 20 C-Atomen, wobei die oben genannten Gruppen jeweils mit einem oder mehreren Resten R⁵ substituiert sein können und wobei ein oder mehrere H-Atome in den oben genannten Gruppen durch D, CN oder NO₂ ersetzt sein können, wobei beide Reste R⁷ auch einen Ringschluss bilden können, so dass eine Spiro-Verbindung entsteht, wobei an den durch die beiden Reste R⁷ gebildeten Ring keine aromatischen oder heteroaromatischen Ringe kondensiert sind;

Bevorzugt sind Ar¹ und Ar³ in Formel (173) bei jedem Auftreten gleich oder verschieden ausgewählt aus Biphenyl, Terphenyl, Quarterphenyl, die mit einem oder mehreren Resten R⁵ substituiert sein können, wobei weiterhin bevorzugt ist, wenn diese unsubstituiert sind.

Ganz besonders bevorzugt sind Verbindungen der folgenden, beispielhaft aufgeführten Formeln (174) bis (236).

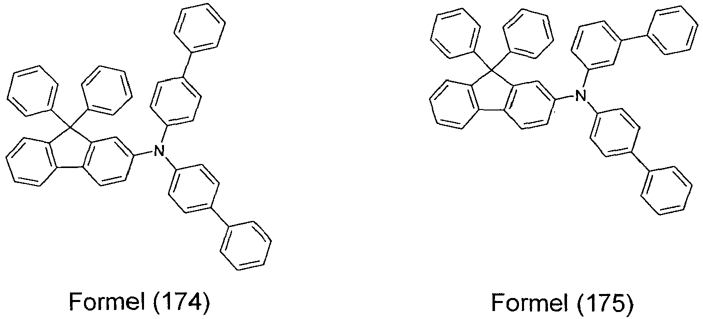

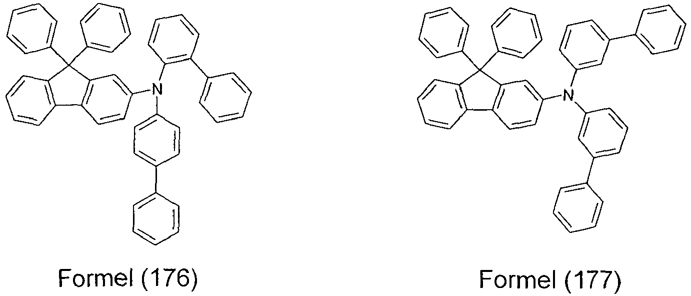

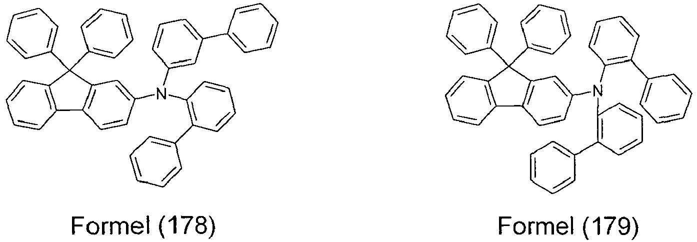

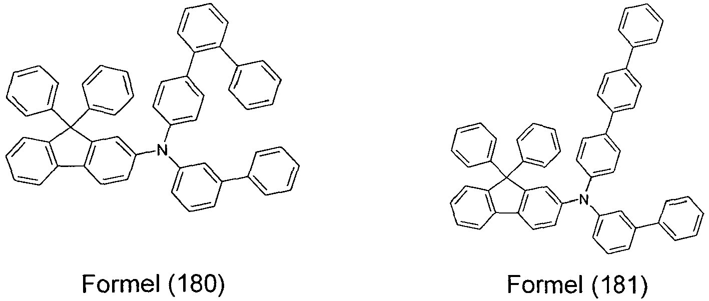

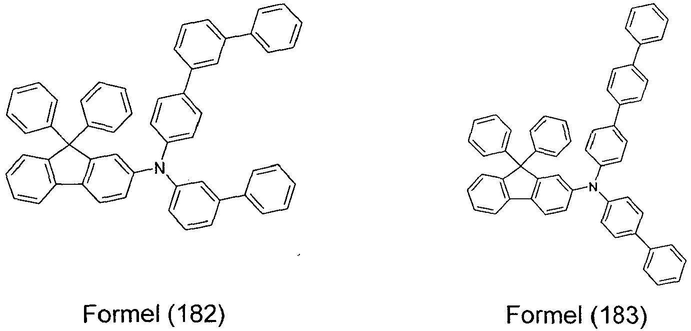

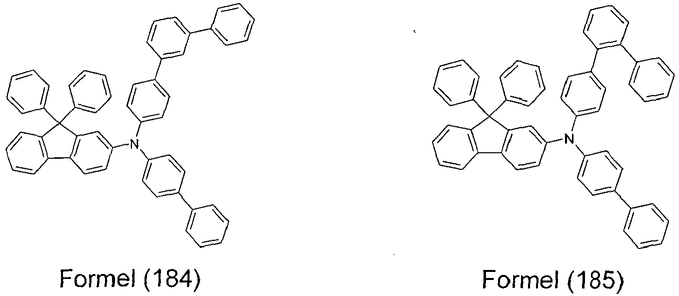

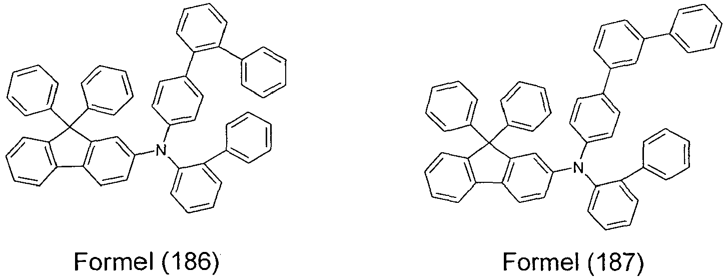

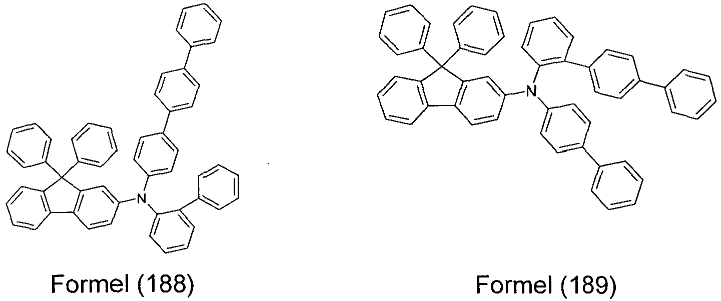

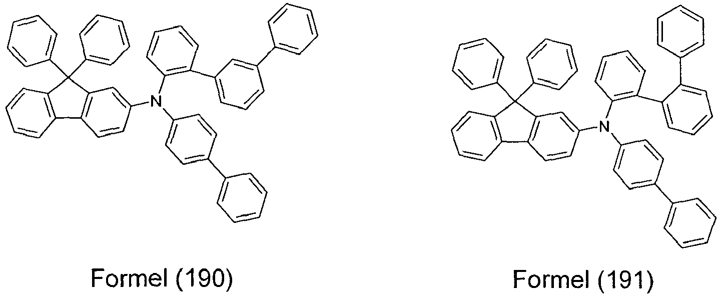

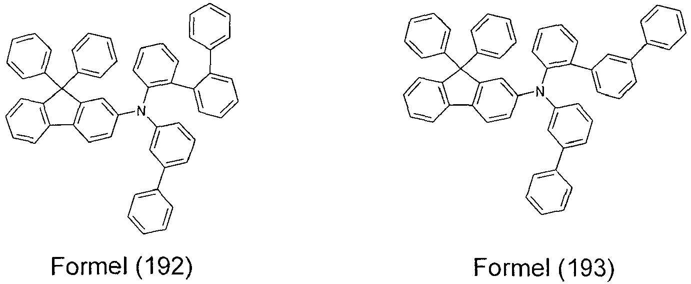

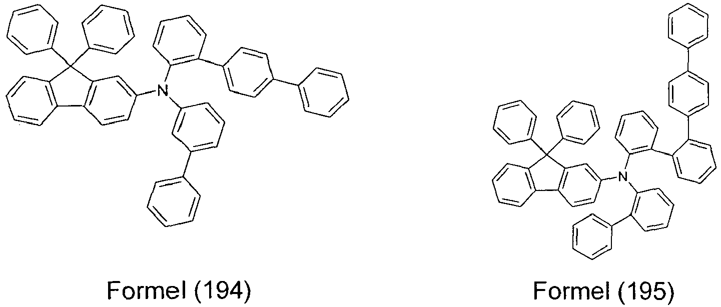

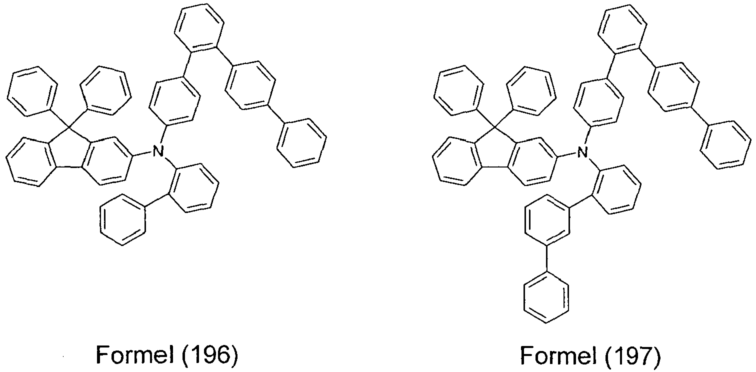

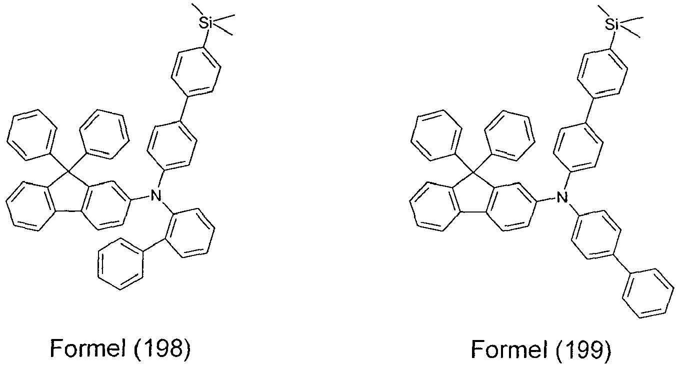

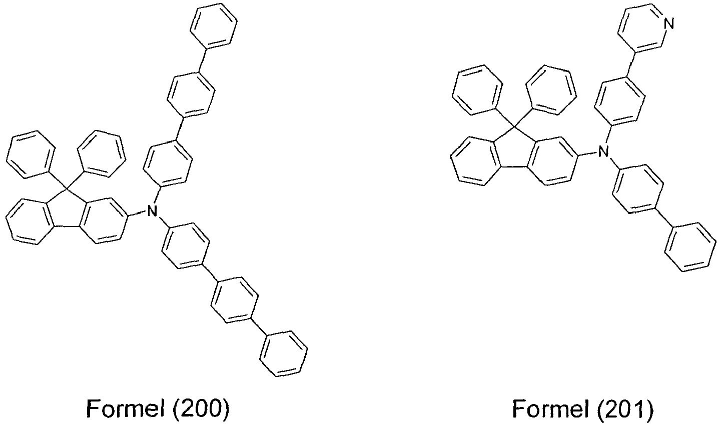

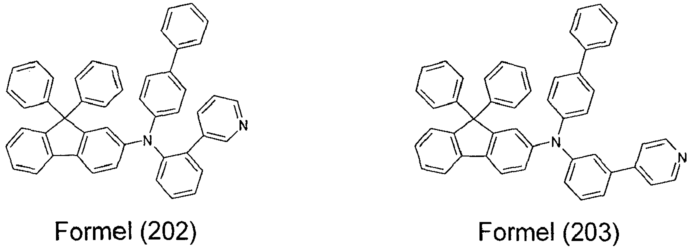

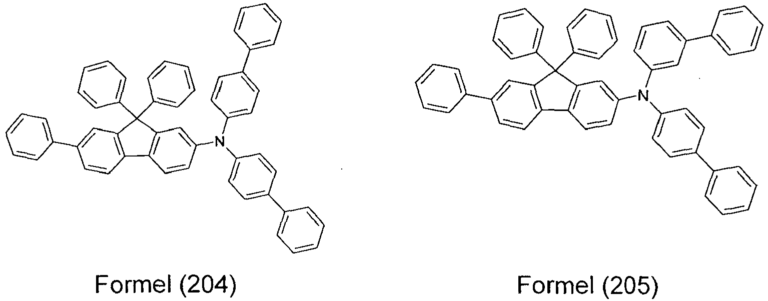

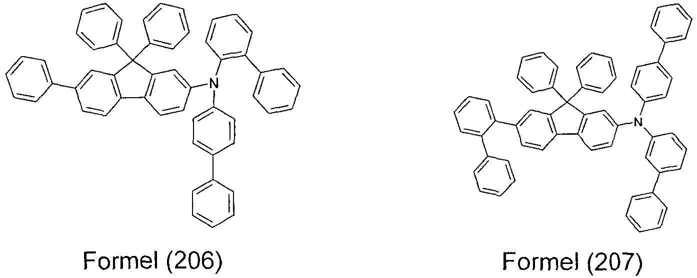

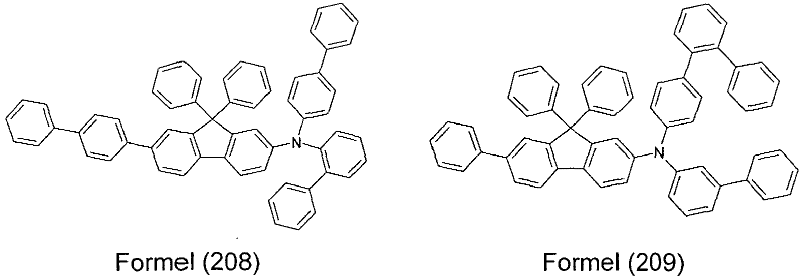

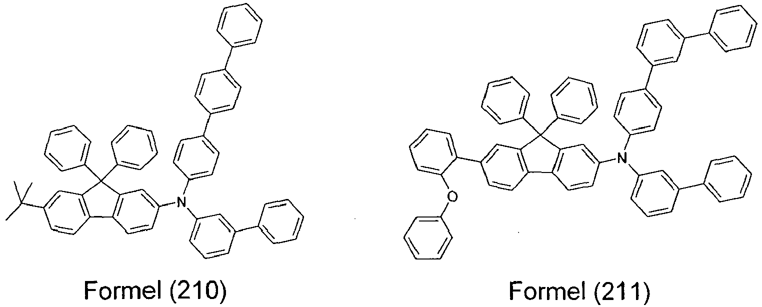

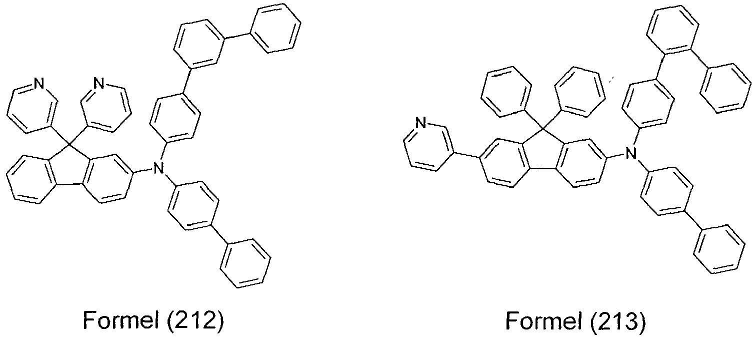

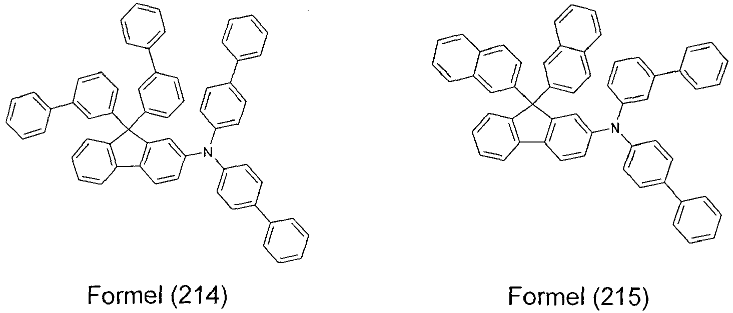

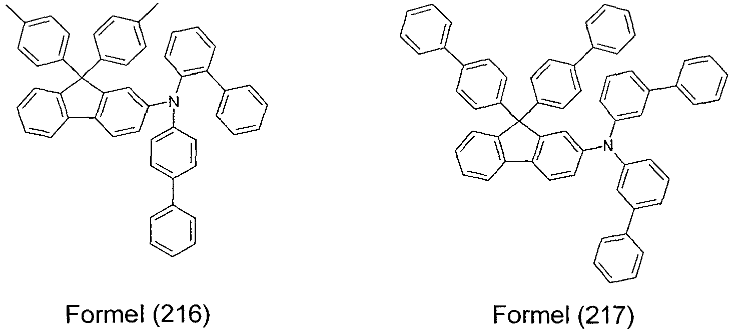

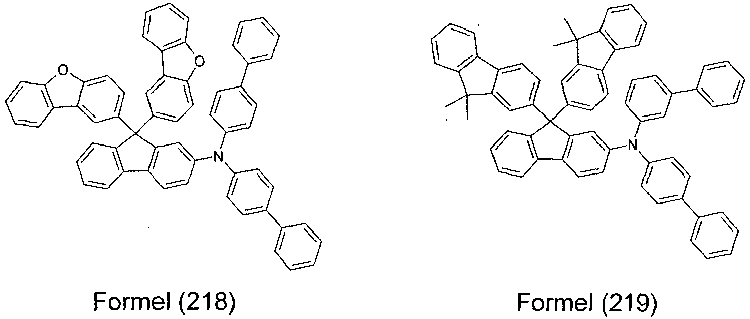

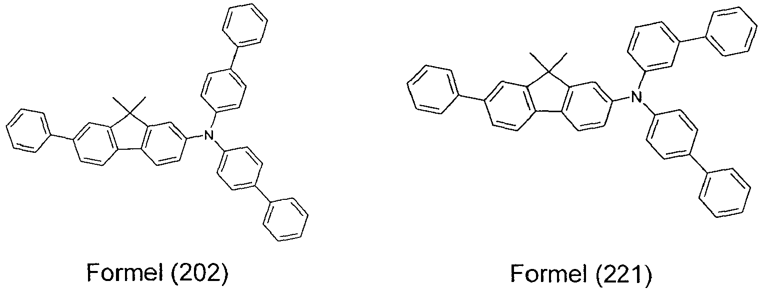

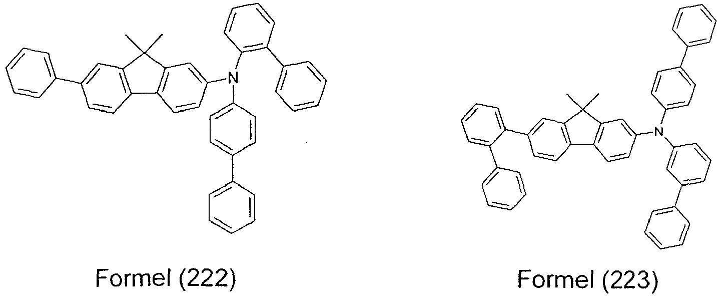

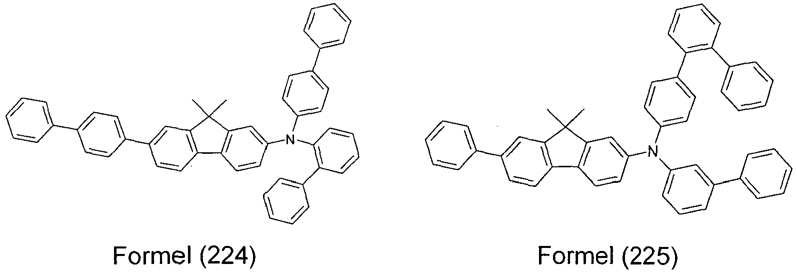

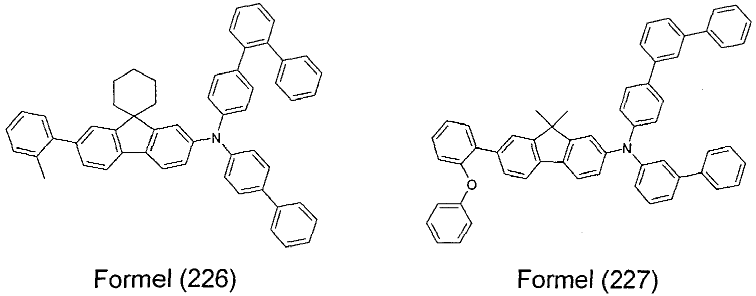

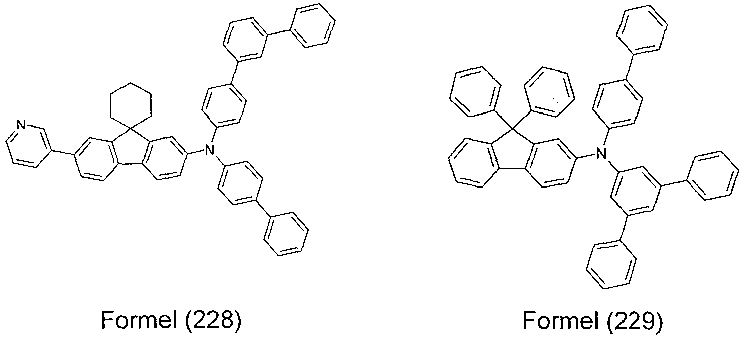

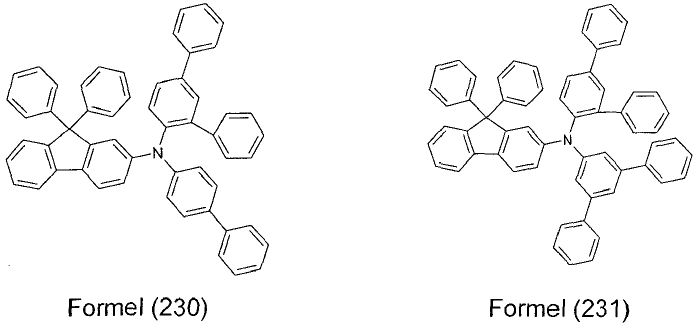

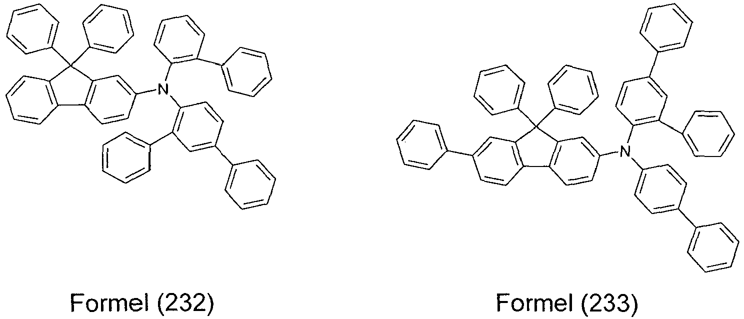

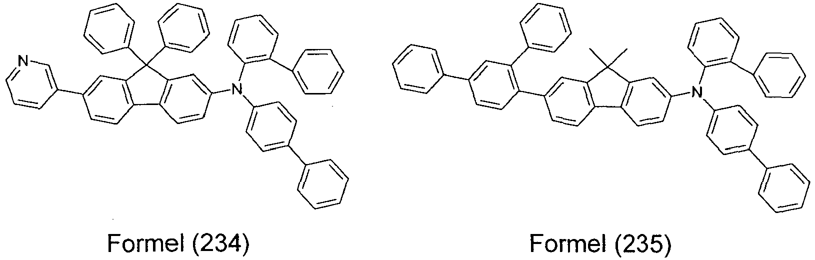

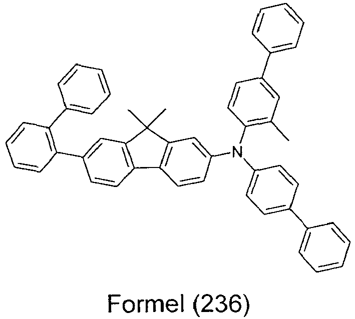

Die erfindungsgemäßen Verbindungen können mit anderen organisch funktionellen Materialien, die in elektronischen Vorrichtungen verwendet werden, als Zusammensetzungen eingesetzt werden. Dem Fachmann ist hierbei eine Vielzahl möglicher organisch funktioneller Materialien aus dem Stand der Technik bekannt. Die vorliegende Erfindung betrifft daher auch eine Zusammensetzung enthaltend eine oder meherer erfindungsgemäße Verbindung der Formel (167) und wenigstens ein weiteres organisch funktionelles Material ausgewählt aus der Gruppe bestehend aus fluoreszierenden Emitter, phosphoreszierenden Emitter, Host Materialien, Matrix Materialien, Elektronentransportmaterialien, Elektroneninjektionsmaterialien, Lochleitermaterialien, Lochinjektionsmaterialien, Elektronenblockiermaterialien und Lochblockiermaterialien.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Miniemulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Gegenstand der Erfindung ist daher weiterhin eine Formulierung, insbesondere eine Lösung, Dispersion oder Miniemulsion, enthaltend mindestens eine Verbindung gemäß Formel (167) oder mindestens ein Polymer, Oligomer oder Dendrimer enthaltend mindestens eine Einheit gemäß Formel (167) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (z.B. OLEDs oder OLECs). Abhängig von der Substitution werden die Verbindungen in unterschiedlichen Funktionen und Schichten eingesetzt.

Ein weiterer Gegenstand der vorliegenden Erfindung ist daher die Verwendung der Verbindungen gemäß Formel (167) in elektronischen Vorrichtungen sowie elektronische Vorrichtungen selbst, welche eine oder mehrere Verbindungen gemäß Formel (167) enthalten. Dabei sind die elektronischen Vorrichtungen bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs und OLECs).

Gegenstand der Erfindung sind, wie bereits oben ausgeführt, elektronische Vorrichtungen, enthaltend mindestens eine Verbindung gemäß Formel (167). Dabei sind die elektronischen Vorrichtungen bevorzugt ausgewählt aus den oben genannten Vorrichtungen. Besonders bevorzugt sind organische Elektrolumineszenzvorrichtungen (OLEDs), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (167) enthält.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cycfohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Die oben beschriebenen Verbindungen der Formel (1) können mit reaktiven Abgangsgruppen, wie Brom, Iod, Chlor, Boronsäure oder Boronsäureester substituiert werden. Diese können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (1), wobei die Bindung(en) zum Polymer, Oligomer oder Dendrimer an beliebigen in Formel (1) möglichen Positionen lokalisiert sein können. Je nach Verknüpfung der Verbindung gemäß Formel (1) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein. In den linear verknüpften Strukturen können die Einheiten gemäß Formel (1) ddirekt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formeln (1) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (1) in Oligomeren, Dendrimeren und Polymeren gelten dieselben Bevorzugungen wie oben für Verbindungen gemäß Formel (1) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (1) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL

Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Die erfindungsgemäßen Verbindungen können in solchen Vorrichtungen in einer Lochtransportschicht, einer emittierenden Schicht und/oder in einer anderen Schicht vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (1) in einer elektrolumineszierneden Vorrichtung enthaltend einen oder mehrere phosphoreszierende Dotanden eingesetzt wird. Dabei kann die Verbindung in unterschiedlichen Schichten, bevorzugt in einer Lochtransportschicht, einer Lochinjektionsschicht oder in einer emittierenden Schicht, verwendet werden. Die Verbindung gemäß Formel (1) kann aber auch erfindungsgemäß in einer elektronischen Vorrichtung enthaltend einen oder mehrere fluoreszierende Dotanden eingesetzt werden.

Vom Begriff phosphoreszierende Dotanden sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen Emitter können den Anmeldungen WO 00/70655, WO 01/41512, WO 02/02714, WO 02/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (1) in organischen Elektrolumineszenzvorrichtungen einsetzen.

Explizite Beispiele für geeignete phosphoreszierende Emitterverbindungen können weiterhin der folgenden Tabelle entnommen werden.

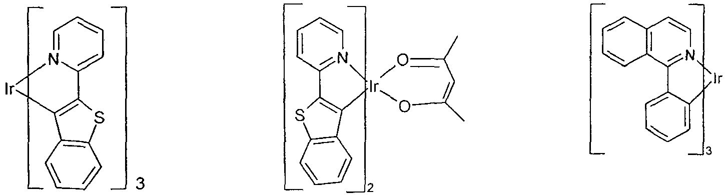

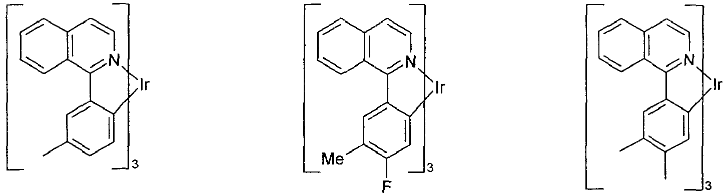

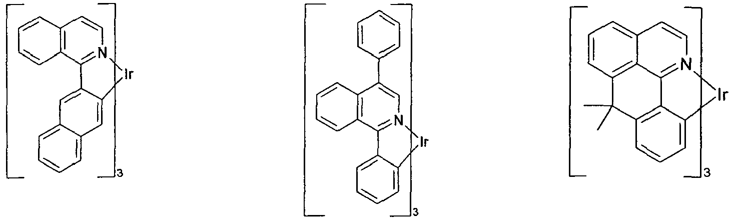

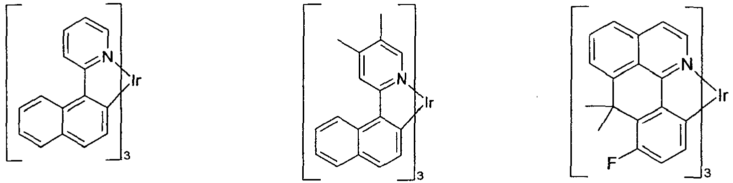

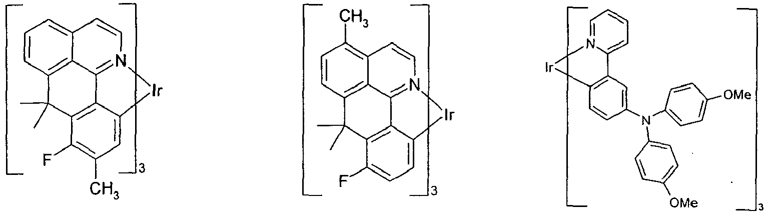

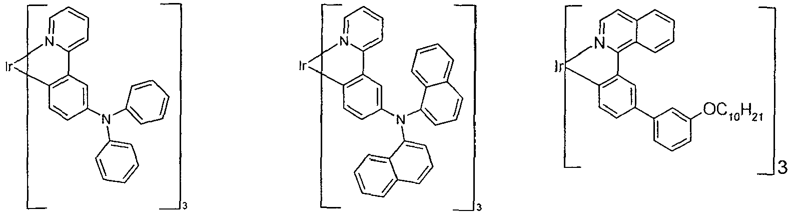

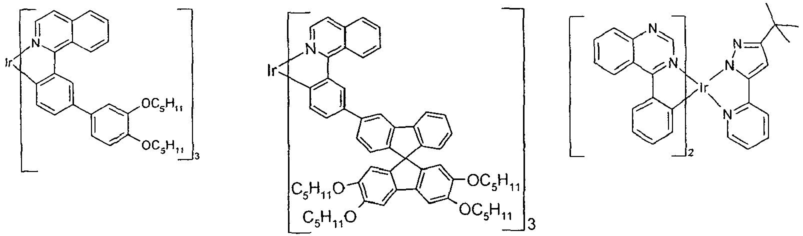

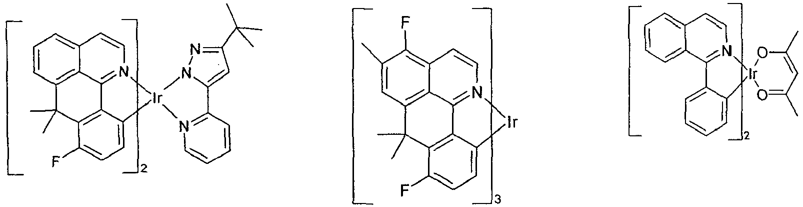

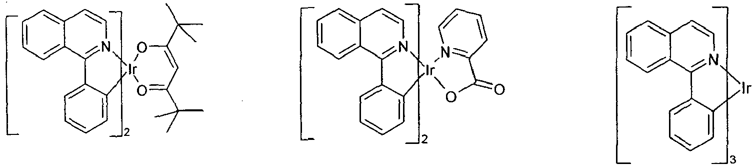

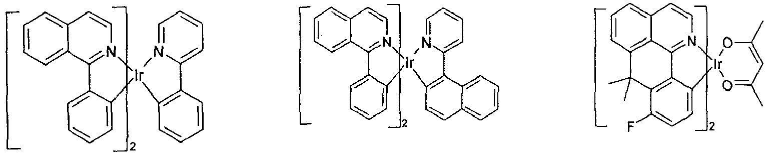

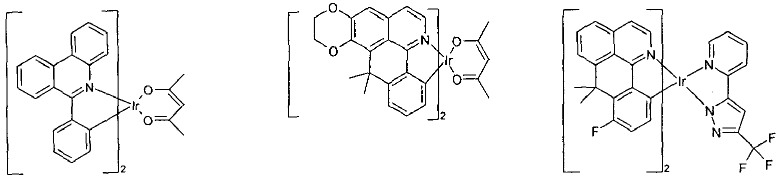

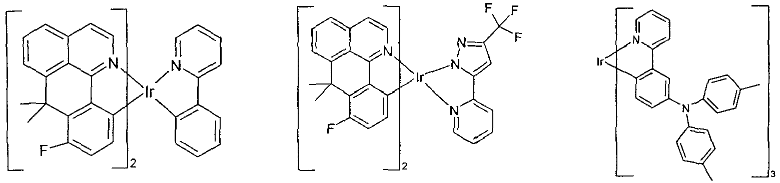

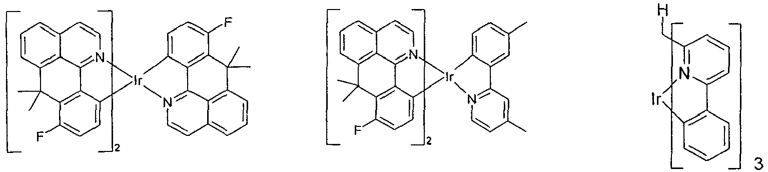

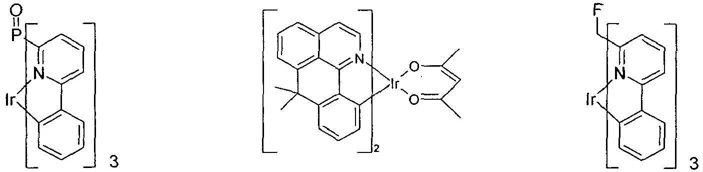

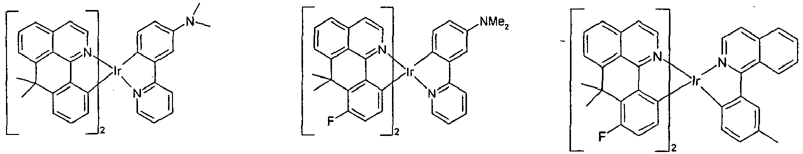

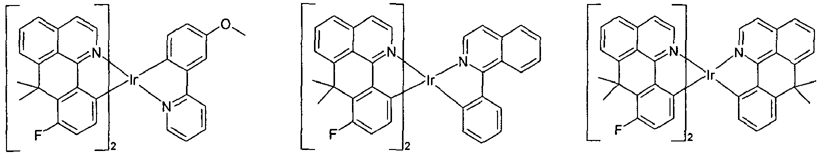

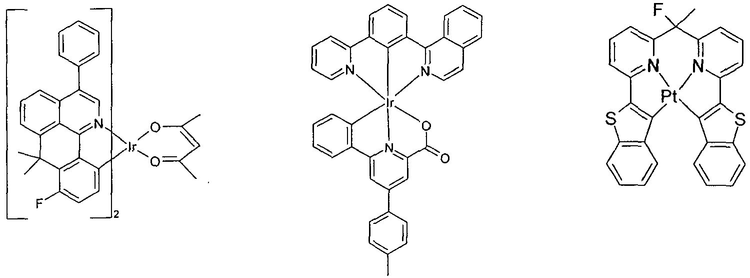

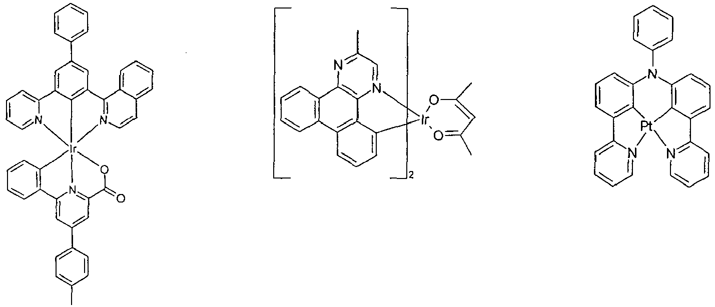

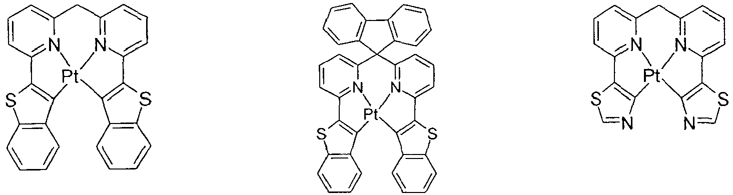

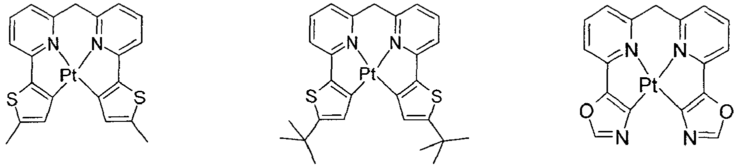

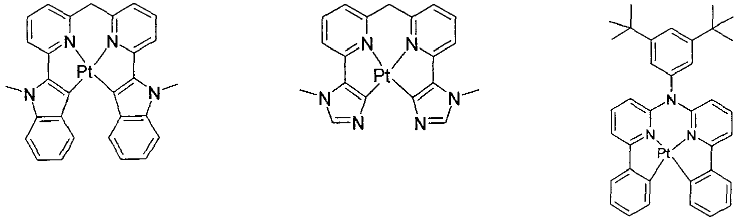

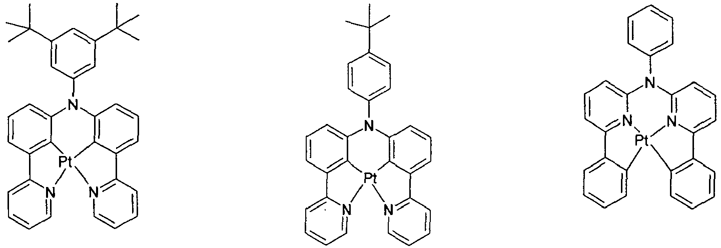

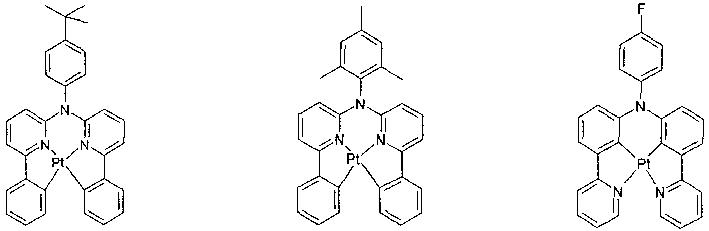

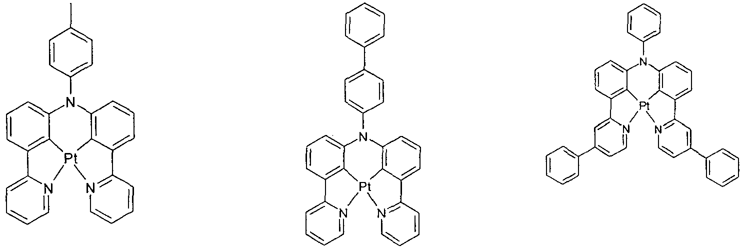

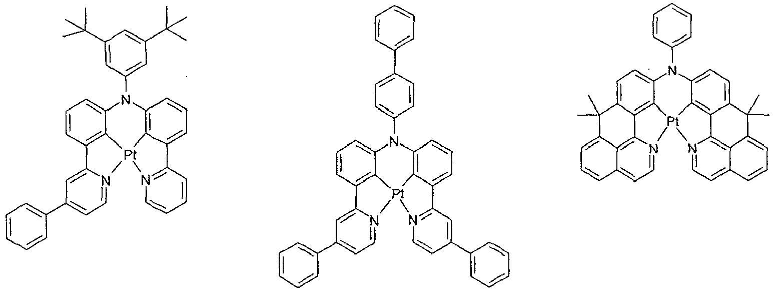

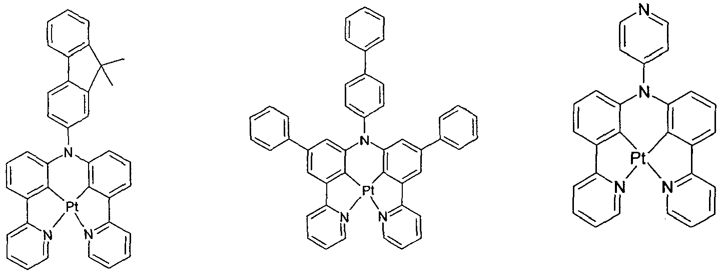

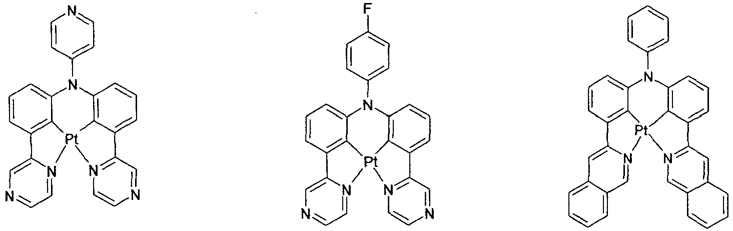

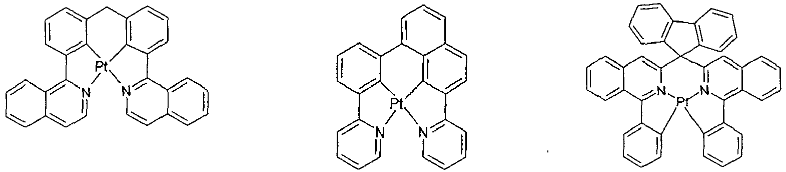

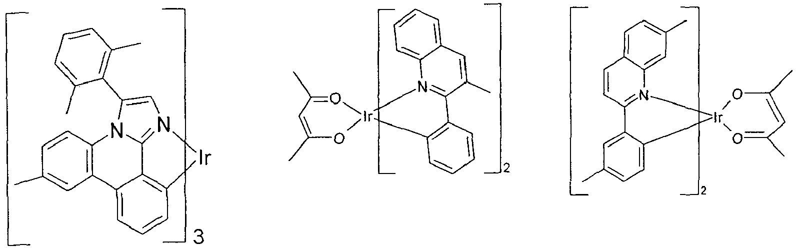

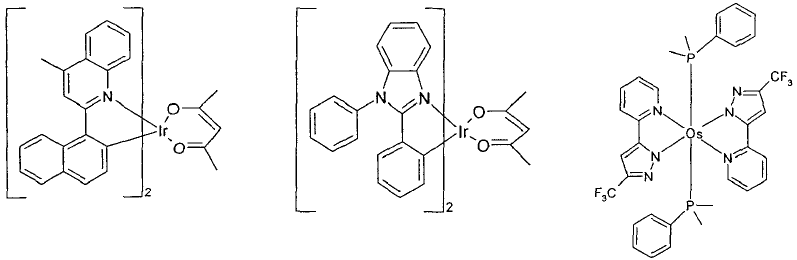

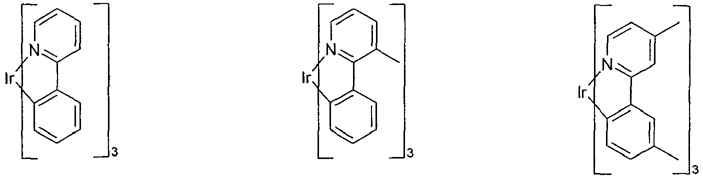

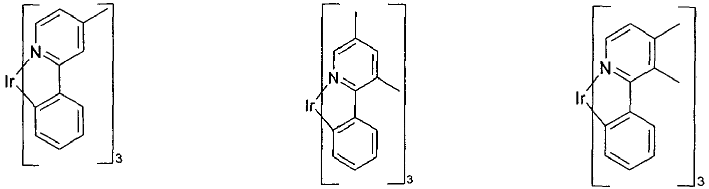

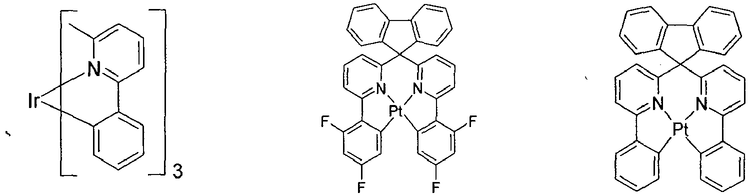

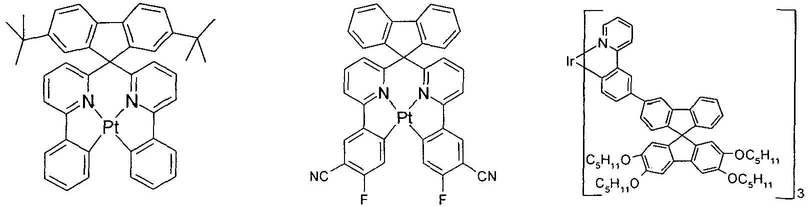

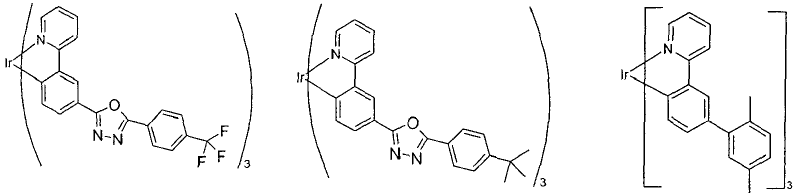

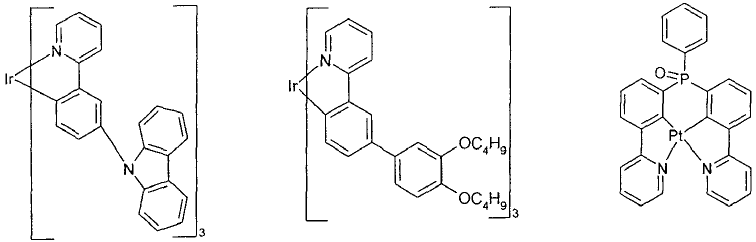

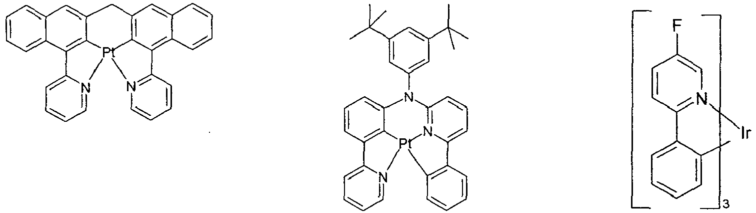

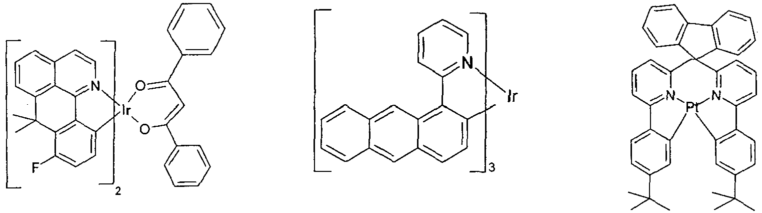

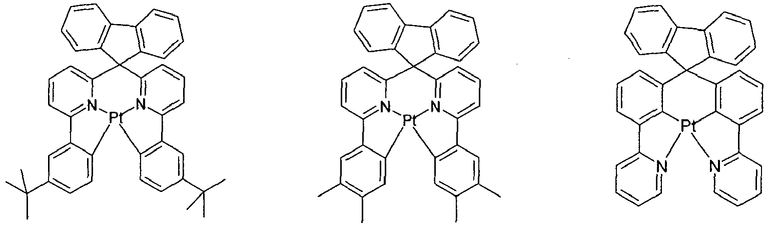

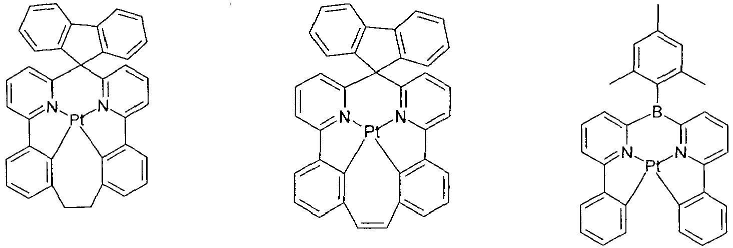

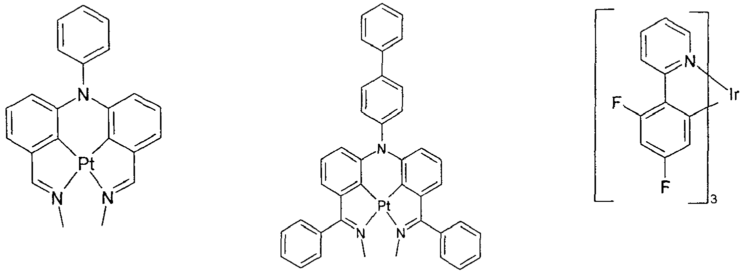

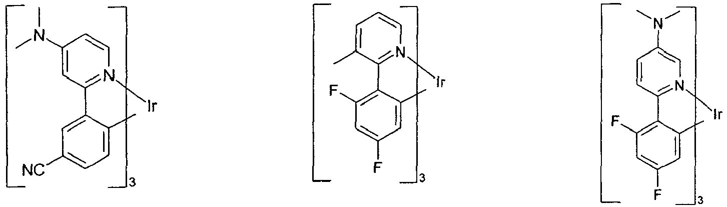

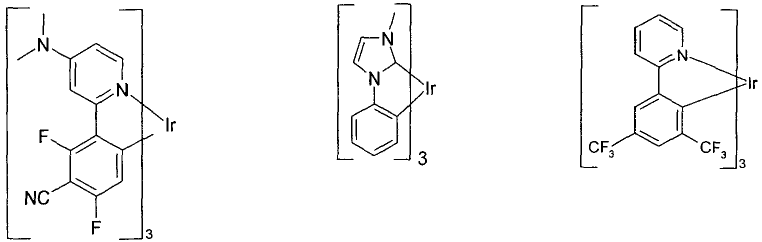

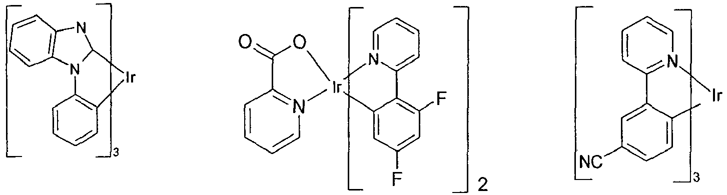

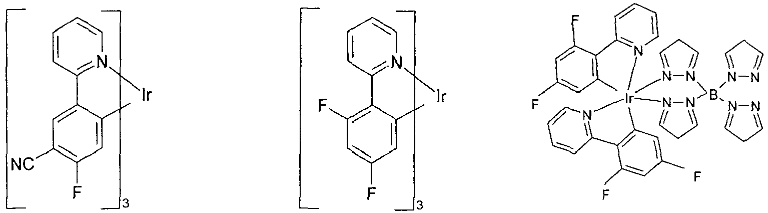

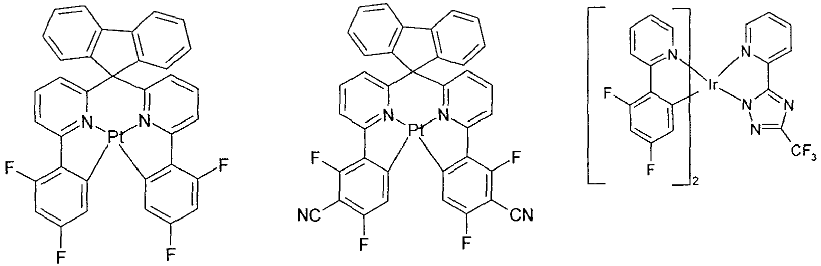

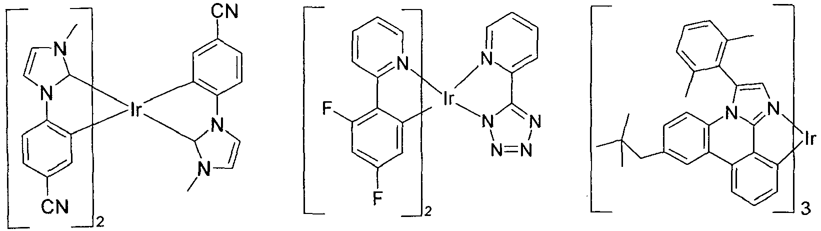

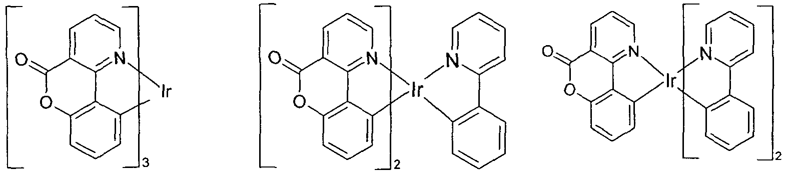

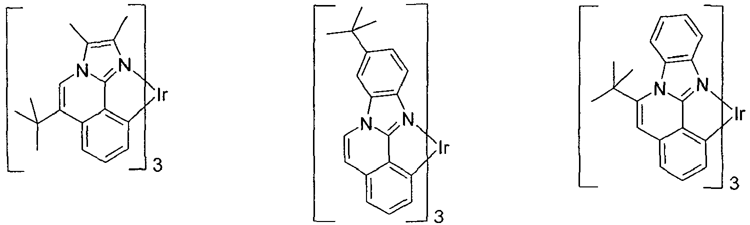

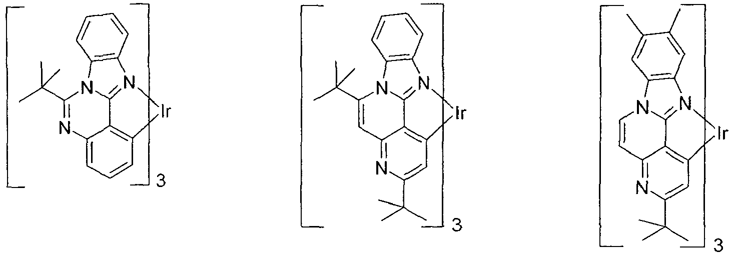

In einer bevorzugten Ausführungsform der Erfindung werden die Verbindungen gemäß den Formeln (1) oder (167) als Lochtransportmaterial eingesetzt. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Lochtransportschicht kann direkt an die Emissionschicht angrenzen. Wenn die Verbindungen gemäß Formel (1) als Lochtransportmaterial oder als Lochinjektionsmaterial verwendet werden, kann es bevorzugt sein, wenn sie mit Elektronenakzeptor-Verbindungen dotiert sind, beispielsweise mit F₄-TCNQ oder mit Verbindungen, wie sie in EP 1476881 oder EP 1596445 beschrieben werden. In einer weiteren bevorzugten Ausführungsform der Erfindung wird eine Verbindung gemäß Formel (1) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

Werden die Verbindungen gemäß den Formel (1) oder (167) als Lochtransportmaterial in einer Lochtransportschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen in der Lochtransportschicht eingesetzt werden.

In einer weiteren Ausführungsform der vorliegenden Erfindung werden die Verbindungen der Formeln (1) oder (167) als emittierende Materialien eingesetzt. Die Verbindungen werde hierzu bevorzugt in einer Emissionsschicht eingesetzt. Die Emissionsschicht enthält neben wenigstens einer der Verbindungen nach den Formeln (1) oder (167) weiterhin wenigstens ein Host Material. Dabei kann der Fachmann aus den bekannten Hostmaterialien ohne Schwierigkeiten und ohne erfinderisch zu sein auswählen.

In einer weiteren Ausführungsform der vorliegenden Erfindung werden die Verbindungen der Formeln (1) oder (167) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß den Formeln (1) oder (167) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Dotanden umfassen, bevorzugt einen oder mehrere phosphoreszierende Dotanden. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Bevorzugte phosphoreszierende Dotanden zur Verwendung in Mixed-Matrix-Systemen sind die in der obenstehenden Tabelle aufgeführten phosphoreszierenden Dotanden.

Im Folgenden werden die in den erfindungsgemäßen Vorrichtungen in den betreffenden Funktionen bevorzugt eingesetzten Materialien aufgeführt.

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Als Matrixmaterialien, bevorzugt für fluoreszierende Dotanden, kommen neben den erfindungsgemäßen Verbindungen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den erfindungsgemäßen Verbindungen aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAIQ.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß den Formeln (1) oder (167) in Displays, als Lichtquellen in Beleuchtungsanwendungen sowie als Lichtquellen in medizinischen und/oder kosmetischen Anwendungen (z.B. Lichttherapie) eingesetzt werden.

Vorrichtungen enthaltend die Verbindungen nach den Formeln (1) oder (167) können sehr vielseitig eingesetzt werden. So können, beispielsweise, elektrolumineszierende Vorrichtuungen enthaltend eine oder mehrere Verbindungen nach den Formeln (1) oder (167) in Displays für Fernseher, Mobiltelefone, Computer und Kameras eingesetzt werden. Die Vorrichtungen können aber auch in Beleuchtungsanwendungen verwendet werden. Weiterhin können elektrolumineszierende Vorrichtungen, z. B. in OLEDs oder OLECs, enthaltend wenigstens eine der Verbindung nach den Formeln (1) oder (167) in der Medizin oder Kosmetik zu Phototherapie genutzt werden. Somit kann eine Vielzahl von Erkrankungen (Psoriasis, atopische Dermatitis, Inflammation, Akne, Hautkrebs etc.) oder die Vermeidung sowie Reduktion von Hautfaltenbildung, Hautrötung und Hautalterung behandelt werden. Weiterhin können die lichtemittierenden Vorrichtungen dazu genutzt werden, um Getränke, Speisen oder Lebensmittel frisch zu halten oder um Geräte (bspw. medizinische Geräte) zu sterilisieren.

Die erfindungsgemäßen Verbindungen bzw. die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Lochtransportschicht oder einer Lochinjektionsschicht in elektronischen Vorrichtungen, wie beispielsweise in organischen Elektrolumineszenzvorrichtungen, insbesondere aufgrund ihrer hohen Lochbeweglichkeit.
2. Die erfindungsgemäßen Verbindungen weisen eine relativ niedrige Sublimationstemperatur, eine hohe Temperaturstabilität sowie eine hohe Oxidationsstabilität und eine hohe Glasübergangtemperatur auf, was sowohl für die Prozessierbarkeit, beispielweise aus Lösung oder aus der Gasphase, als auch für die Verbendung in elektronischen Vorrichtungen vorteilhaft ist.
3. Die Verwendung der erfindungsgemäßen Verbindungen in elektronischen Vorrichtungen, insbesondere eingesetzt als Lochtransport- oder Lochinjektionsmaterial führt zu hohen Effizienzen, geringen Betriebsspannungen sowie zu langen Lebensdauern.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

### Beispiele

### Materialien

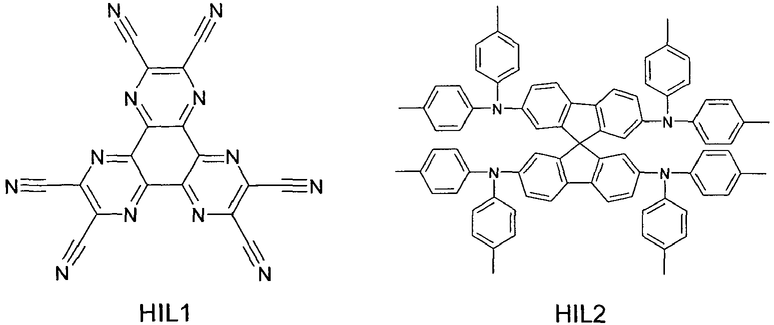

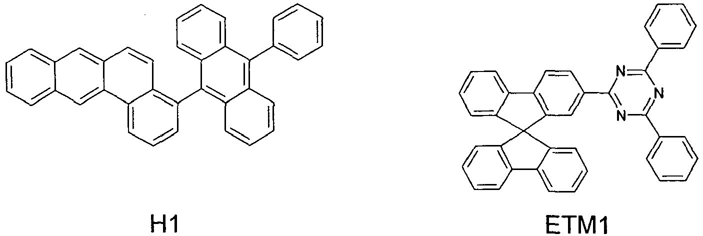

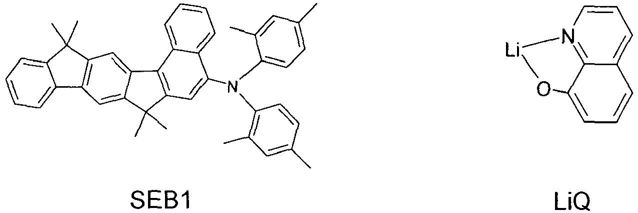

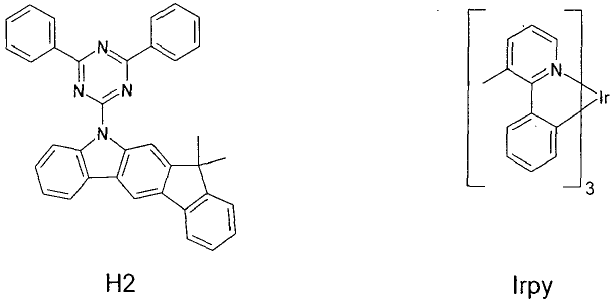

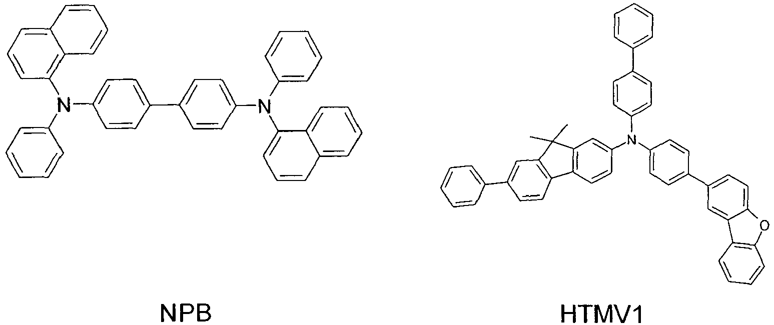

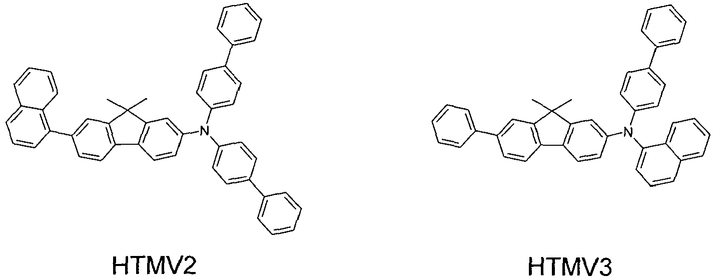

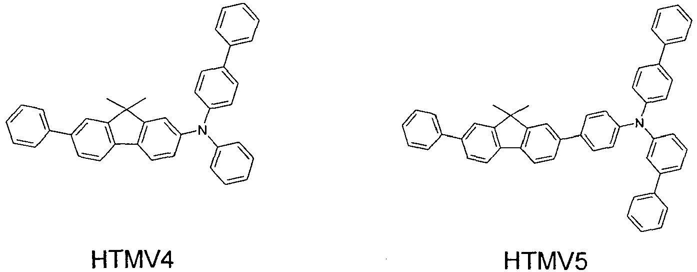

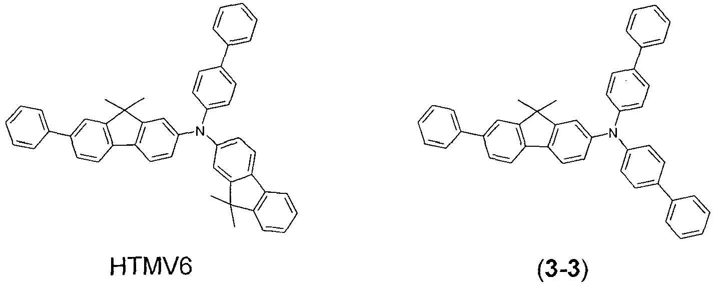

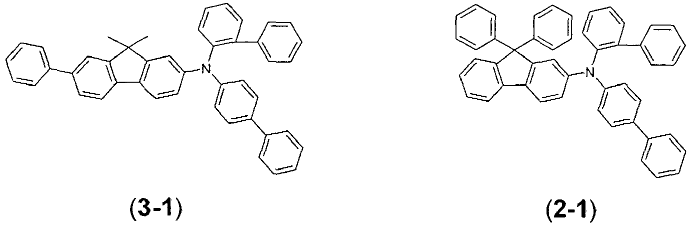

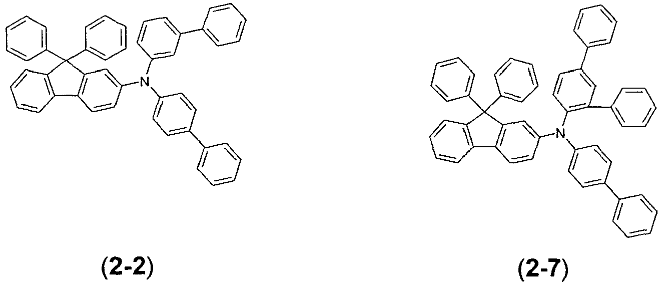

Die Materialien HIL1, HIL2 (EP 0676461), H1 (WO 2008/145239), ETM1 (WO 2005/053055), SEB1 (WO 2008/006449), LiQ und NPB sind dem Fachmann wohl bekannt. Deren Eigenschaften und Synthesen sind aus dem Stand der Technik bekannt. Die Verbindungen (**3-3**), (**3-1**), (**2-1**) und (**2-2**), und (**2-7**) sind erfindungsgemäß.

### Beispiel 1

### Synthese der Verbindung Biphenyl-2-yl-biphenyl-4-yl-(9,9-dimethyl-9H-fluoren-2-yl)-amin (1-1) sowie der Verbindungen (1-2) bis (1-5)

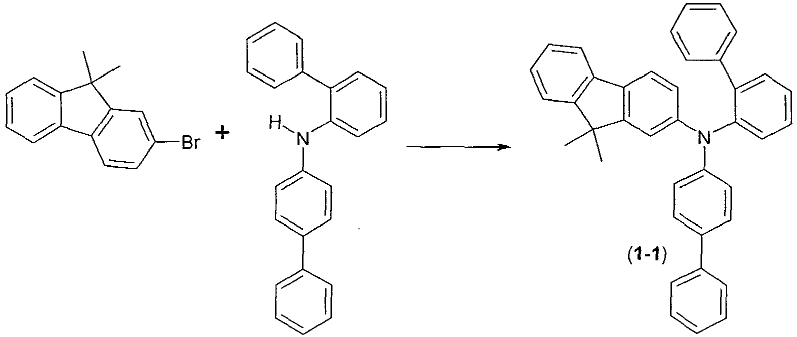

23,5 g Biphenyl-2-yl-biphenyl-4-yl-amin (73 mmol) und 20,0 g 2-Bromofluoren (73 mmol) werden in 500 mL Toluol gelöst: Die Lösung wird entgast und mit N₂ gesättigt. Danach wird sie mit 2,52 g (2,93 mmol) Tri-tert-Butylphosphin und 0,33 g (1,46 mmol) Palladium(II)acetat versetzt. Anschließend werden 10,8 g Natrium-tert-butylat (110 mmol) zugegeben. Die Reaktionsmischung wird 6 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 32,0 g (85% der Theorie).

Analog dazu werden die folgenden Verbindungen (1-2) bis (1-5) hergestellt:

| **Edukt 1** | **Edukt 2** | **Produkt** | **Ausbeute** |
|---|---|---|---|
| 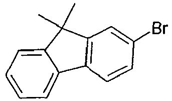 | 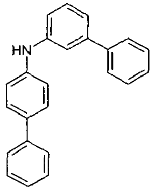 | 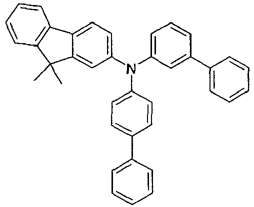 | 78% |
| | | **(1-2)** | |
| 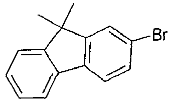 | 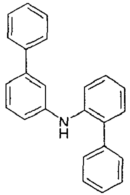 | 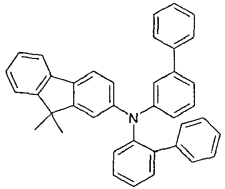 | 92% |
| | | **(1-3)** | |
| 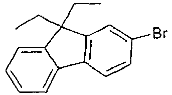 | 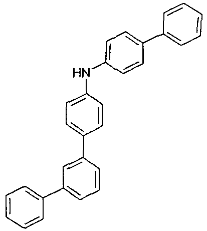 | 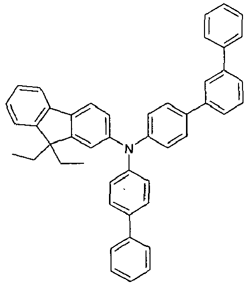 | 88% |
| | | **(1-4)** | |
| 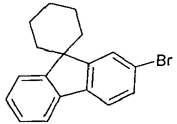 | 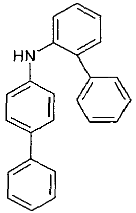 | 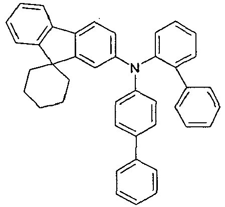 | 77% |
| | | **(1-5)** | |

### Beispiel 2

### Synthese der Verbindung Biphenyl-2-yl-biphenyl-4-yl-(9,9-diphenyl-9H-fluoren-3-yl)-amin (2-1) sowie der Verbindungen (2-2) bis (2-10)

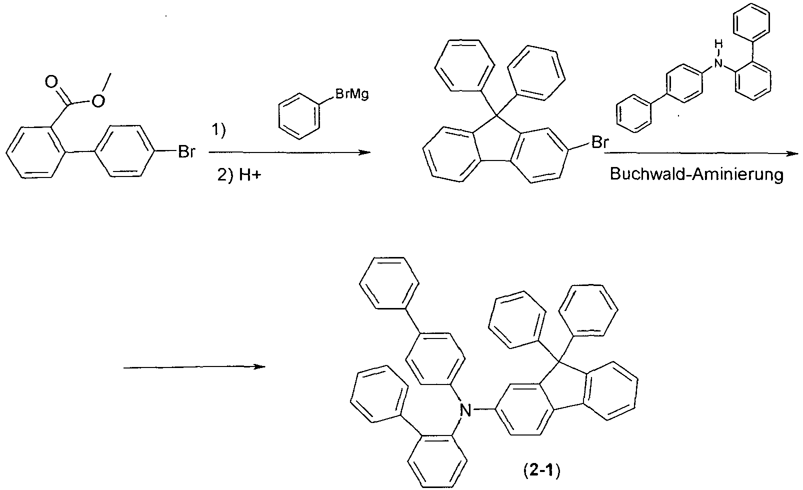

### 2-Bromo-9,9-di-phenyl-9H-fluoren (2-1)

30 g (103 mmol) 4'-Bromo-biphenyl-2-carbonsäuremethylester werden in einem ausgeheizten Kolben in 500 mL getrocknetem THF gelöst. Die klare Lösung wird auf -10°C abgekühlt und dann werden 102 mL (307 mmol) einer frisch hergestellten 3 M 2-Phenylmagnesiumbromid Lösung zugegeben. Die Reaktionsmischung wird langsam auf Raumtemperatur erwärmt und dann mit NH₄Cl gequencht (500 mL). Das Gemisch wird im Anschluss zwischen Essigester und Wasser verteilt, die organische Phase dreimal mit Wasser gewaschen und über Na₂SO₄ getrocknet und einrotiert. Der Rückstand wird vorsichtig mit 400 mL Essigsäure versetzt. Anschließend werden 80 mL rauchende HCl zugegeben. Der Ansatz wird auf 75°C erhitzt und 5 h auf dieser Temperatur gehalten. Dabei fällt ein weißer Feststoff aus. Der Ansatz wird nun auf Raumtemperatur abgekühlt und der ausgefallene Feststoff wird abgesaugt und mit Methanol nachgewaschen. Der Rückstand wird im Vakuum bei 40°C getrocknet. Die Ausbeute beträgt 29,4 g (74 mmol) (72% der Theorie).

Analog dazu werden folgenden bromierten Verbindungen hergestellt:

| **Edukt 1** | **Edukt 2** | **Produkt** | **Ausbeute** |
|---|---|---|---|
| 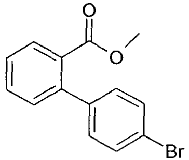 | 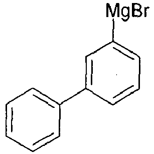 | 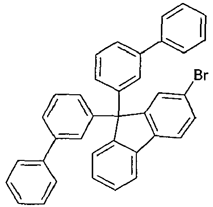 | 65% |
| 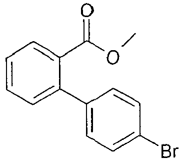 | 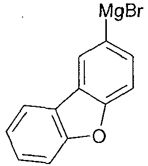 | 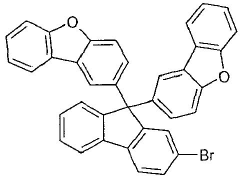 | 70% |
| 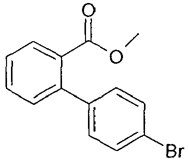 | 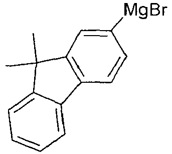 | 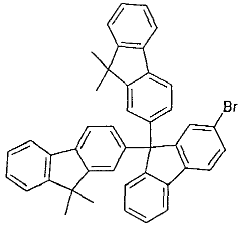 | 72% |
| 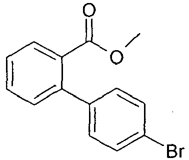 | 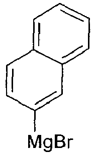 | 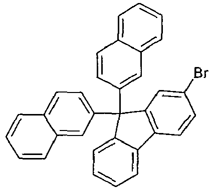 | 80% |

**Biphenyl-2-yl-biphenyl-4.-yl-(9,9-diphenyl-9H-fluoren-3-yl)-amin (2-1)** 17 g Biphenyl-2-yl-biphenyl-4-yl-amin (53 mmol) und 21 g 2-Bromo-9,9-diphenyl-9H-fluorene (53 mmol) werden in 350 mL Toluol gelöst: Die Lösung wird entgast und mit N₂ gesättigt. Danach wird sie mit 2,1 mL (2,1 mmol) einer 1 M Lösung Tri-tert-Butylphosphin und 0,24 g (1,06 mmol) Palladium(II)acetat versetzt und anschließend werden 12,7 g Natrium-tert-butylat (132 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 25 g (74% der Theorie).

Analog hierzu können die folgenden Verbindungen (**2-2**) bis (**2-10**) hergestellt werden.

| **Edukt 1** | **Edukt 2** | **Produkt** | **Ausbeute** |
|---|---|---|---|
| 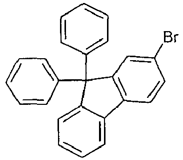 | 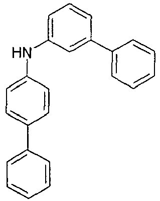 | 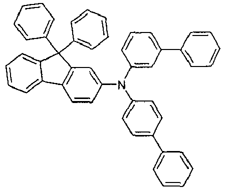 | 72% |
| | | **(2-2)** | |
| 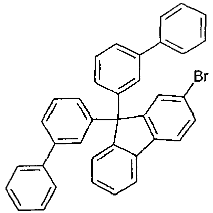 | 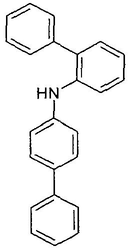 | 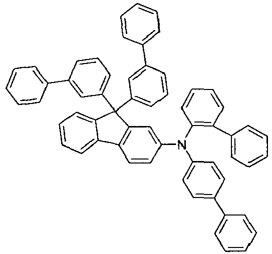 | 78% |
| | | **(2-3)** | |
| 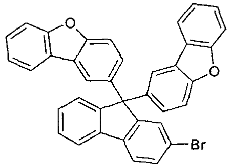 | 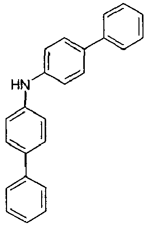 | 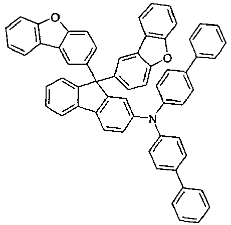 | 81% |
| | | **(2-4)** | |
| 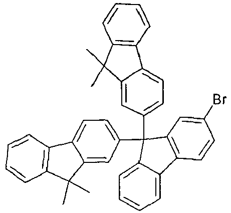 | 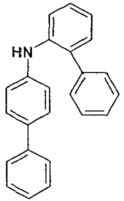 | 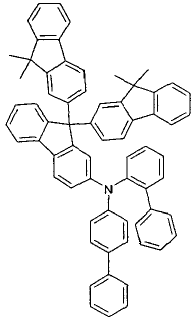 | 75% |
| | | **(2-5)** | |
| 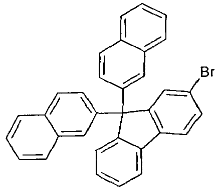 | 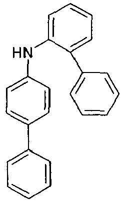 | 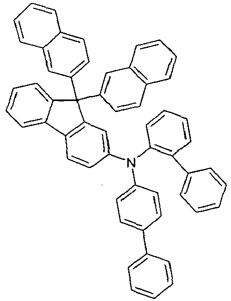 | 75% |
| | | **(2-6)** | |
| 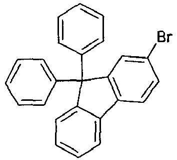 | 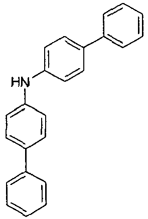 | 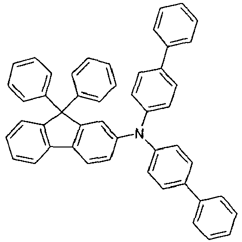 | 77% |
| | | **(2-7)** | |
| 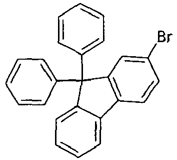 | 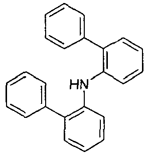 | 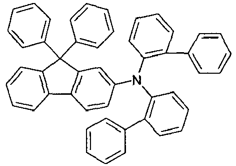 | 65% |
| | | **(2-8)** | |
| 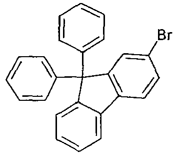 | 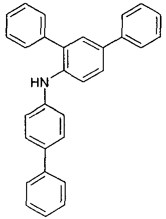 | 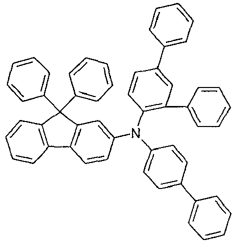 | 62% |
| | | **(2-9)** | |
| 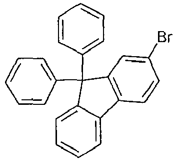 | 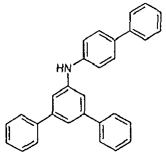 | 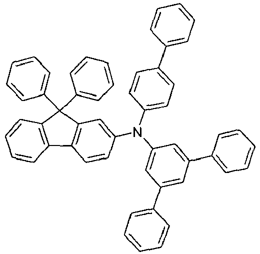 | 70% |
| | | **(2-10)** | |

### Beispiel 3

### Synthese der Verbindung Biphenyl-4-yl-biphenyl-2-yl-(9,9-dimethyl-7-phenyl-9H-fluoren-2-yl)-amin (3-1) sowie der Verbindungen (3-2) bis (3-8)

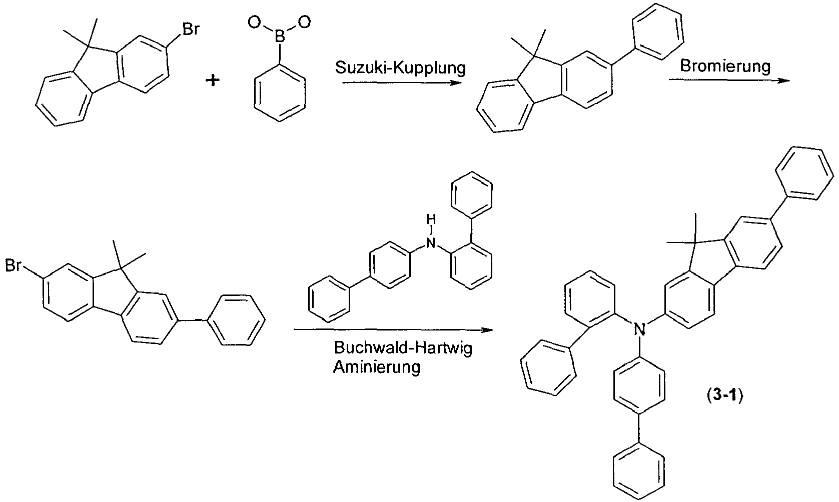

### 9,9-dimethyl-7-phenyl-9H-fluoren

8,9 g (73 mmol) Benzolboronsäure und 20 g (73 mmol) 2-Brom-9,9'-dimethyl-9H-fluoren werden in 330 mL Dimethoxyethan und 110 mL 2 M Na₂CO₃ Lösung suspendiert. Zu dieser Suspension werden 2,54 g (2,0 mmol) Palladium tetrakis(triphenylphosphin) gegeben. Die Reaktionsmischung wird 16 h unter Rückfluss erhitzt. Nach Erkalten wird die Reaktionsmischung mit Essigester verdünnt, die organische Phase abgetrennt, dreimal mit 100 mL Wasser gewaschen und anschließend zur Trockene eingeengt. Nach Filtration des Rohproduktes über Kieselgel mit Heptan/Essigsäureester (20:1) erhält man 18,8 g (95%) 9,9-dimethyl-7-phenyl-9H-fluoren.

Analog hierzu werden die folgenden Fluorene hergestellt.

| **Edukt 1** | **Edukt 2** | **Produkt** | **Ausbeute** |
|---|---|---|---|
| 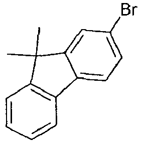 | 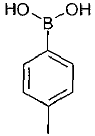 | 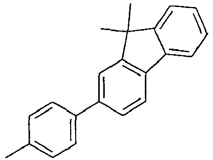 | 90% |
| 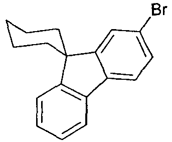 | 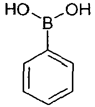 | 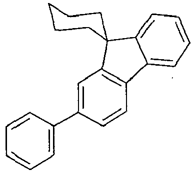 | 93% |
| 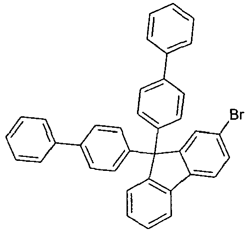 | 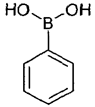 | 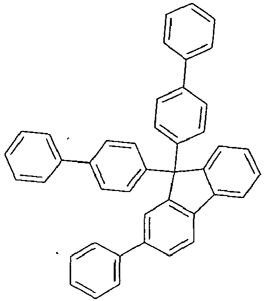 | 88% |
| 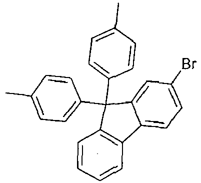 | 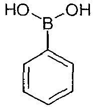 | 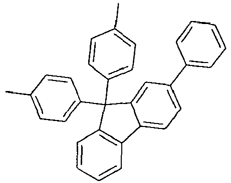 | 95% |
| 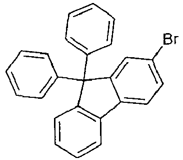 | 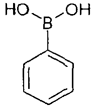 | 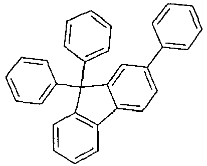 | 80% |

### 2-Bromo-9,9-dimethyl-7-phenyl-9H-fluoren

29,0 g (107 mmol) 9,9-Dimethyl-2-phenyl-9H-fluoren werden in 250 mL CHCl₃ gelöst und bei -10°C langsam mit 17,2 g (107 mmol) Brom, gelöst in 50 mL CHCl₃, versetzt. Nach vollständiger Umsetzung wird Wasser dazu gegeben, die organische Phase abgetrennt, getrocknet und eingeengt. Das Rohprodukt wird anschließend mit MeOH/Heptan (1:1) mehrfach heiß ausgerührt. Die Ausbeute beträgt 33,3 g g (89% der Theorie) des Produktes als weißen Feststoff.

Analog hierzu werden die folgenden bromierten Verbindungen hergestellt.

| **Edukt 1** | **Produkt** | **Ausbeute** |
|---|---|---|
| 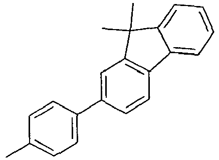 | 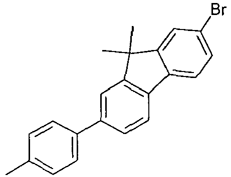 | 80% |
| 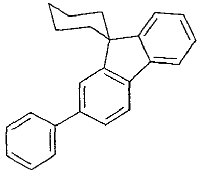 | 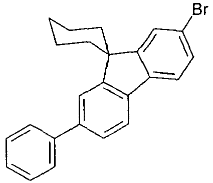 | 75% |
| 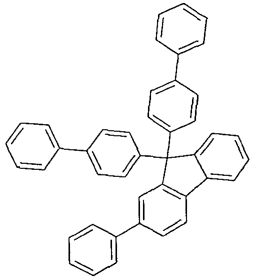 | 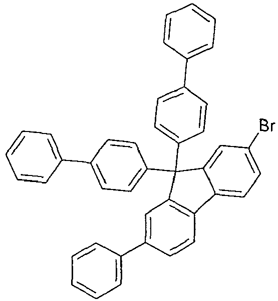 | 72% |
| 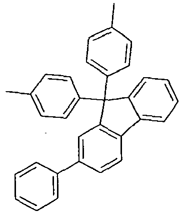 | 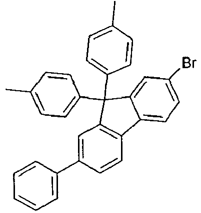 | 65% |
| 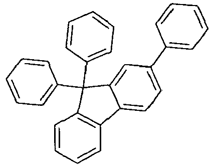 | 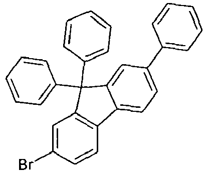 | 80% |

### Biphenyl-4-yl-biphenyl-2-yl-(9,9-dimethyl-7-phenyl-9H-fluoren-2-yl)-amin (3-1)

19,9 g Biphenyl-2-yl-biphenyl-4-yl-amine (62 mmol) und 21,6 g 2-Bromo-9,9-dimethyl-7-phenyl-9H-fluoren (62 mmol) werden in 400 mL Toluol gelöst: Die Lösung wird entgast und mit N₂ gesättigt. Danach wird sie mit 3 mL (3 mmol) einer 1 M Tri-tert-Butylphosphin Lösung und 0,57 g (2 mmol) Palladium(II)acetat versetzt. Anschließend werden 14,9 g Natrium-tert-butylat (155 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 29,7 g (82% der Theorie).

Analog hierzu werden die Verbindungen (**3-2**) bis (**3-8**) hergestellt.

| **Edukt 1** | **Edukt 2** | **Produkt** | **Ausbeute** |
|---|---|---|---|
| 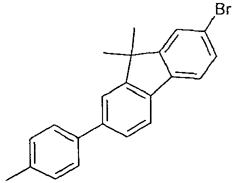 | 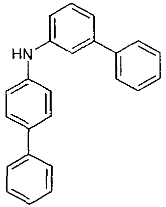 | 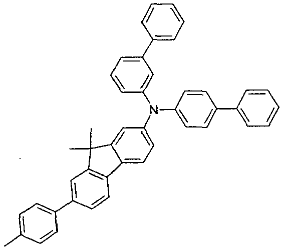 | 77% |
| | | **(3-2)** | |
| 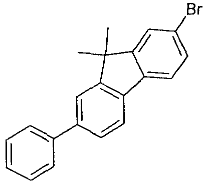 | 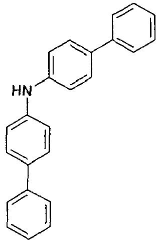 | 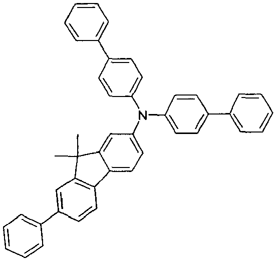 | 87% |
| | | **(3-3)** | |
| 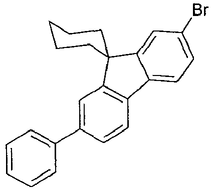 | 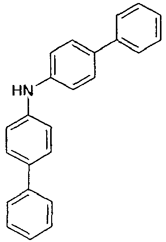 | 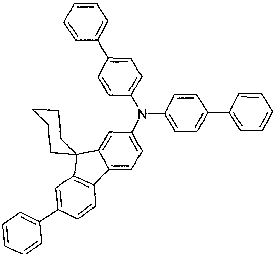 | 84% |
| | | **(3-4)** | |
| 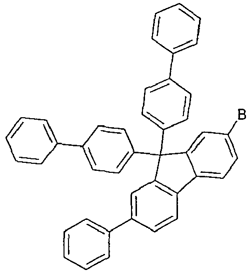 | 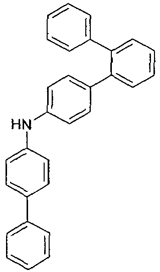 | 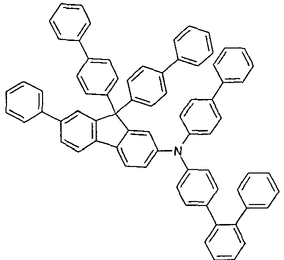 | 75% |
| | | **(3-5)** | |
| 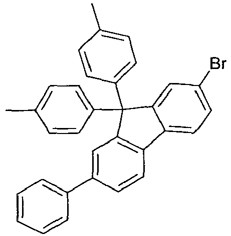 | 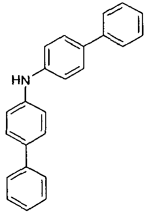 | 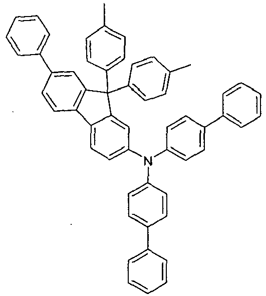 | 82% |
| | | **(3-6)** | |
| 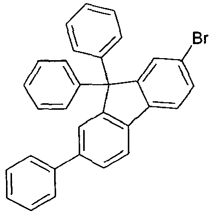 | 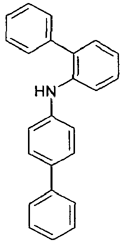 | 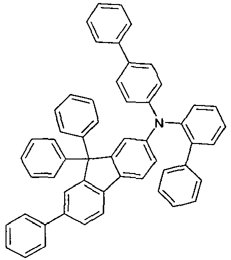 | 85% |
| | | **(3-7)** | |
| 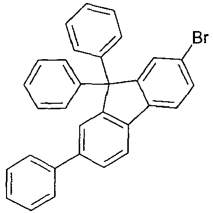 | 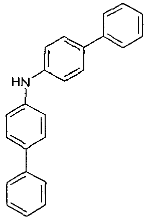 | 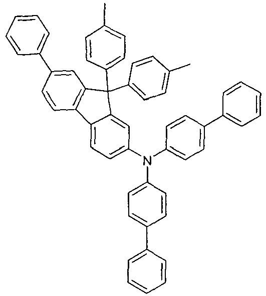 | 76% |
| | | **(3-8)** | |

### Beispiel 4

### Synthese der Vergleichsverbindungen HTMV1 bis HTMV6

Analog zu der in Beispiel 3 beschriebenen Synthese von Verbindung (**3-1**) werden auch die folgenden Vergleichsverbindungen (**HTMV1**) bis (**HTMV6**) hergestellt.

| **Edukt 1** | **Edukt 2** | **Produkt** |
|---|---|---|
| 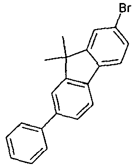 | 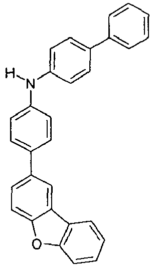 | 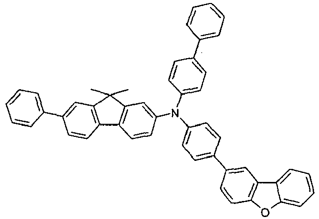 |
| | | **HTMV1** |
| 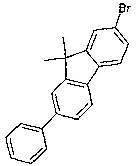 | 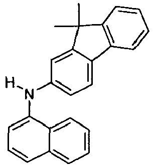 | 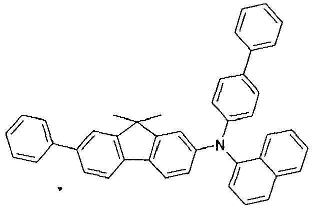 |
| | | **HTMV3** |
| 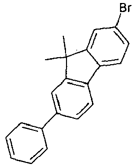 | 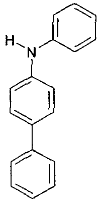 | 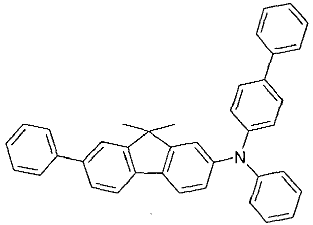 |
| | | **HTMV4** |
| 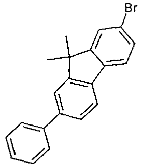 | 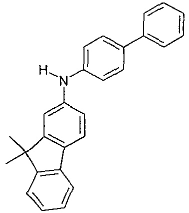 | 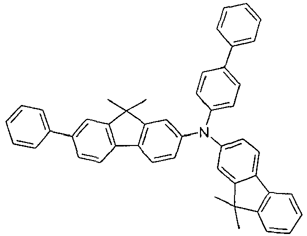 |
| | | **HTMV5** |
| 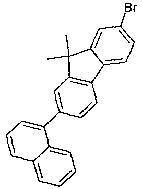 | 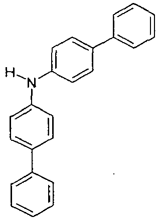 | 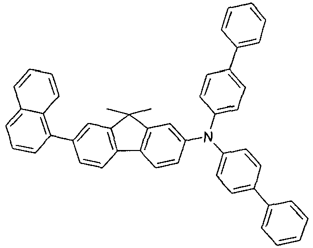 |
| | | **HTMV2** |
| 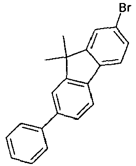 | 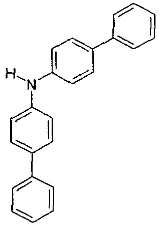 | 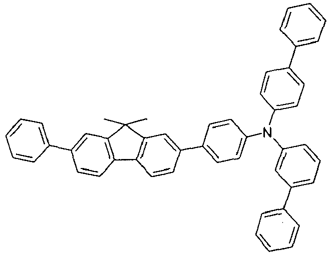 |
| | | **HTMV6** |

### Beispiel 5

### Charakterisierung der Verbindungen

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen (siehe Tabellen 1, 3 und 2, 4) werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / Optionale Lochinjektionsschicht (HIL1) / Lochtransportschicht (HTL) / Lochinjektionsschicht (HIL2) / Elektronenblockerschicht (EBL) / Emissionsschicht (EML) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 und 3 zu entnehmen. Die zur Herstellung der OLEDs benötigten Materialien sind oben angegeben.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB1 (95%:5%) bedeutet hierbei, dass das Material H1 in einem Volumenanteil von 95% und SEB1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 1000 cd/m² bezeichnet die externe Quanteneffizienz bei einer Betriebsleuchtdichte von 1000 cd/m². LD80 @ 6000 cd/m² ist die Lebensdauer bis das OLED bei einer Helligkeit von 6000 cd/m² auf 80% der Anfangsintensität, also auf 4800 cd/m² abgefallen ist. Die Daten der verschiedenen OLEDs sind in den Tabellen 2 und 4 zusammengefasst.

### Verwendung von erfindungsgemäßen Verbindungen als Lochtransportmaterialien in fluorszierenden und phosphoreszierenden OLEDs

Insbesondere eignen sich die erfindungsgemäßen Verbindungen als HIL, HTL oder EBL in OLEDs. Sie eignen sich als Einzelschicht, aber auch als Mixed Komponente als HIL, HTL, EBL oder innerhalb der EML. Verglichen mit NPB Referenz Bauteilen (V1, V8) zeigt die Proben mit den erfindungsgemäßen Verbindungen neben höheren Effizienzen, auch deutlich verbesserte Lebensdauern sowohl für Singulett blau als auch für Triplett grün.
Im Vergleich zu den Referenzmaterialien HTMV1 - HTMV6 (V2-V10) haben die erfindungsgemäßen Verbindungen gleiche oder bessere Effizienzen und verbesserte Lebensdauern.

| **Tabelle 1: Aufbau der OLEDs** | | | | | | |
|---|---|---|---|---|---|---|
| Schichtaufbau: Substrat/HlL1/HTL/HIL2/EBL/EML/ETL/EIL 1 nm LiQ/Kathode | | | | | | |
| ***Bsp.*** | ***HIL1*** | ***HTL*** | ***HIL2*** | ***EBL*** | ***EML*** | ***ETL*** |
| | *Dicke* / *nm* | *Dicke* / *nm* | *Dicke* / *um* | *Dicke l nm* | *Dicke* / *um* | *Dicke* / *nm* |
| *V1* | *HIL1* | *HIL2* | *HIL1* | *NPB* | *H1(95%):SEB1(5%)* | *ETMI(50%): LiQ(50%)* |
| | *5 nm* | *140 nm* | *5 nm* | *20 nm* | *20 nm* | *30 nm* |
| *V2* | *HIL1* | *HIL2* | *HIL1* | *HTMV1* | *H1(95%):SEB1(5%)* | *ETM1 (50%): LiQ(50%)* |
| | *5 nm* | *140 nm* | *5nm* | *20 nm* | *20 nm* | *30 nm* |
| *V3* | *HIL1* | *HIL2* | *HIL1* | *HTMV2* | *H1(95%):SE81(5%)* | *ETM1(50%):LiQ(50%)* |
| | *5nm* | *140 nm* | *5 nm* | *20 nm* | *20 nm* | *30 nm* |
| *V4* | *HIL1* | *HIL2* | *HIL1* | *HTMV3* | *H1(95%):SEB1(5%)* | *ETM1(50%):LiQ(50%)* |
| | *5nm* | *140 nm* | *5 nm* | *20 nm* | *20 nm* | *30 nm* |
| *V5* | *HIL1* | *HIL2* | *HIL1* | *HTMV4* | *H1(9591):SEBI(5%)* | *ETM1(50%):LiQ(50%)* |
| | *5 nm* | *140 nm* | *5 nm* | *20 nm* | *20 nm* | *30 nm* |
| *V6* | *HIL1* | *HIL2* | *HIL1* | *HTMV5* | *H1(95%):SEB1(5%)* | *ETM1 (50%): LiQ(50%)* |
| | *5 nm* | *140 nm* | *5 nm* | *20 nm* | *20 nm* | *30 nm* |
| *V7* | *HIL1* | *HIL2* | *HIL1* | *HTMV6* | *H1(95%):SEB1(5%)* | *ETMI(50%): LiQ(50%)* |
| | *5 nm* | *140 nm* | *5 nm* | *20 nm* | *20 nm* | *30 nm* |
| *E1* | *HIL1* | *HIL2* | *HIL1* | (3-3) | *H1(95%):SEB1(5%)* | *ETM1 (50%): LiQ(50%)* |
| | *5 nm* | *140 nm* | *5 nm* | *20 nm* | *20 nm* | *30 nm* |
| E2 | *HIL1* | *HIL2* | *HIL1* | *(3-1)* | *H1(95%):SEB1(5%)* | *ETM1(50%):LiQ(50%)* |
| | *5 nm* | *140 nm* | *5 nm* | *20 nm* | *20 nm* | *30 nm* |
| *E3* | *HIL1* | *HIL2* | *HIL1* | (2-1) | *H1(95%):SEB1(5%)* | *ETMI(50%): LiQ(50%)* |
| | *5 nm* | *140 nm* | *5 nm* | *20 nm* | *20 nm* | *30 nm* |
| *E4* | *HIL1* | *HIL2* | *HIL1* | (2-2) | *H1(95%):SEB1(5%)* | *ETM1 (50%): LiQ(50%)* |
| | *5 nm* | *140 nm* | *5nm* | *20 nm* | *20 nm* | *30 nm* |
| *E5* | *HIL1* | *HIL2* | *HIL1* | *(2-7)* | *H1(95%):SEB1(5%)* | *ETM1(50%):LiQ(50%)* |
| | *5 nm* | *140 nm* | *5 nm* | *20 nm* | *20 nm* | *30 nm* |

| **Tabelle 2: Daten der OLEDs** | | | | |
|---|---|---|---|---|
| ***Bsp.*** | ***EQE* @ *1000 cd*/*m2*** | ***LD80* @ *6000 cd*/*m²*** | ***CIE*** | |
| | *%* | *[h]* | *x* | *y* |
| *V1* | *4.8* | *70* | 0.14 | 0.17 |
| *V2* | *6.9* | *120* | 0.13 | 0.14 |
| *V3* | *7.0* | *115* | 0.13 | 0.15 |
| *V4* | *6.8* | *105* | 0.13 | 0.15 |
| *V5* | *6.6* | *105* | 0.13 | 0.15 |
| *V6* | *6.6* | *120* | 0.13 | 0.14 |
| *V7* | *7.3* | *15* | 0.14 | 0.15 |
| *E1* | *7.2* | *140* | 0.13 | 0.15 |
| *E2* | *7.0* | *135* | 0.13 | 0.14 |
| *E3* | *6.6* | *150* | 0.13 | 0.15 |
| *E4* | *6.4* | *145* | 0.13 | 0.15 |
| *E5* | *6.6* | *155* | 0.13 | 0.15 |

| **Tabelle 3: Aufbau der OLEDs** | | | | | |
|---|---|---|---|---|---|
| Schichtaufbau: Substrat/HTL/HIL2/EBL/EML/ETL/Kathode | | | | | |
| ***Bsp.*** | ***HTL*** | ***HIL2*** | ***EBL*** | ***EML*** | ***ETL*** |
| | *Dicke* / *nm* | *Dicke* / *nm* | *Dicke* / *nm* | *Dicke* / *nm* | *Dicke* / *nm* |
| *V8* | *HIL2* | *HIL1* | *NPB* | *H2(68%):lrpy(12%)* | *ETM1(50%):LiQ(50%)* |
| | *70 nm* | *5 nm* | *20 nm* | *30 nm* | *40 nm* |
| *V9* | *HIL2* | *HIL1* | *HTMV5* | *H2(88%):lrpy(12%)* | *ETM1(50%):LiQ(50%)* |
| | *70 nm* | *5 nm* | *20 nm* | *30 nm* | *40 nm* |
| *V10* | *HIL2* | *HIL1* | *HTMV6* | *H2(88%):lrpy(12%)* | *ETM1(50%):LiQ(50%)* |
| | *70 nm* | *5 nm* | *20 nm* | *30 nm* | *40 nm* |
| *E6* | *HIL2* | *HIL1* | *(2-1)* | *H2*(*88*%)*:lrpy(12%)* | *ETM1 (50%): LiQ(50%)* |
| | *70 nm* | *5 nm* | *20 nm* | *30 nm* | *40 nm* |
| *E7* | *HIL2* | *HIL1* | *(2-2)* | *H2(88%):lrpy(12%)* | *ETM1(50%):LiQ(50%)* |
| | *70 nm* | *5 nm* | *20 nm* | *30 nm* | *40 nm* |
| *E8* | *HIL2* | *HIL1* | *(2-7)* | *H2(88%):lrpy(12%)* | *ETM1(50%):LiQ(50%)* |
| | *70 nm* | *5 nm* | *20 nm* | *30 nm* | *40 nm* |

| **Tabelle 4: Daten der OLEDs** | | | | |
|---|---|---|---|---|
| ***Bsp.*** | ***Effizienz* @ *1000 cd*/*m2*** | ***LD80* @ *8000 cd*/*m²*** | ***CIE*** | |
| | *%* | *[h]* | *x* | *y* |
| *V8* | *13.4* | *85* | 0.32 | 0.63 |
| *V9* | *17.0* | *155* | 0.37 | 0.61 |
| *V10* | *18.1* | *65* | 0.37 | 0.61 |
| *E6* | *17.6* | *195* | 0.37 | 0.61 |
| *E7* | *17.1* | *185* | 0.37 | 0.61 |
| *E8* | *17.5* | *200* | 0.37 | 0.61 |

## Patentansprüche

1. Elektrolumineszierende Vorrichtung enthaltend wenigstens eine Verbindung der allgemeinen Formel (1) 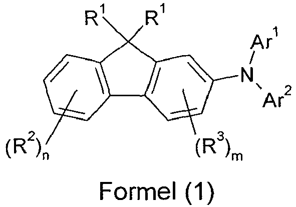 wobei für die auftretenden Symbole und Indices gilt:
Ar¹, Ar² sind bei jedem Auftreten gleich oder verschieden ein Aromat oder Heteroaromat mit 10 bis 60 aromatischen Ringatomen, welche mit einem oder mehreren Resten R⁴, die gleich oder verschieden voneinander sind, substituiert sein können, wobei beide Gruppen Ar¹ oder Ar² jeweils mindestens zwei oder mehr aromatische oder heteroaromatische Ringe enthalten;
R¹ ist bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵-, -C≡C-, Si(R⁵)₂, C=O, C=S, C=NR⁵, -C(=O)O-, -C(=O)NR⁵-, P(=O)(R⁵), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 Ringatomen, das jeweils durch einen oder mehrere Reste R⁵ substituiert sein kann, oder ein kondensiertes Ringsystem mit 9 bis 30 Ringatomen, das jeweils durch einen oder mehrere Reste R⁵ substituiert sein kann, wobei im Fall aromatischer oder heteroaromatischer kondensierter Ringe nicht mehr als 10 Ringatome enthalten sein dürfen; beide Reste R¹ können auch miteinander einen Ringschluss bilden können, so dass eine Spiro Verbindung entsteht, wobei an den durch die beiden Reste R¹ gebildeten Ring keine aromatischen oder heteroaromatischen Ringe kondensiert sind;
R², R³ und R⁴ sind bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵-, -C≡C-, Si(R⁵)₂, C=O, C=S, C=NR⁵, -C(=O)O-, -C(=O)NR⁵- P(=O)(R⁵) -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 Ringatomen, das jeweils durch einen oder mehrere Reste R⁵ substituiert sein kann;
R⁵ ist bei jedem Auftreten gleich oder verschieden H, D, F, Cl, Br, I, C(=O)R⁶, CN, Si(R⁶)₃, NO₂, P(=O)(R⁶)₂, S(=O)R⁶, S(=O)₂R⁶, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁶ substituiert sein können und wobei eine oder mehrere CH₂-Gruppen in den oben genannten Gruppen durch -R⁶C=CR⁶-, -C≡C-, Si(R⁶)₂, C=O, C=S, C=NR⁶, -C(=O)O-, -C(=O)NR⁶-, P(=O)(R⁶), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R⁶ substituiert sein kann, oder eine Aryloxy- oder Heteroaryloxygruppe mit 5 bis 30 aromatischen Ringatomen, die durch einen oder mehrere Reste R⁶ substituiert sein kann;
R⁶ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können;
n ist 0, 1, 2, 3 oder 4;
m ist 0, 1, 2 oder 3;
mit der Maßgabe, dass die Verbindung nach Formel (1) neben der einen Fluoren Gruppe und neben den möglichen kondensierten oder polycyclischen Gruppen in Position 9 des Fluorens keine weiteren polycyclischen oder kondensierten Gruppen enthält.

2. Vorrichtung gemäß Anspruch 1, **dadurch gekennzeichnet, dass** beide Reste R¹ in der Verbindung nach Formel (1) identischen sind.

3. Vorrichtung gemäß Anspruch 1 oder 2, **dadurch gekennzeichnet, dass** m gleich 1 und n gleich 0, 1 oder 2 ist.

4. Vorrichtung gemäß einem oder mehreren der Ansprüche 1 bis 3, **dadurch gekennzeichnet, dass** sie wenigstens eine Verbindung der allgemeinen Formel (2) enthält 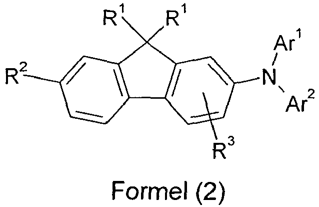 wobei für die Symbole die Definitionen aus Anspruch 1 gelten.

5. Vorrichtung gemäß einem oder mehreren der Ansprüche 1 bis 4, **dadurch gekennzeichnet, dass** R³ gleich H ist.

6. Vorrichtung gemäß einem oder mehreren der Ansprüche 1 bis 5, **dadurch gekennzeichnet, dass** Ar¹ und Ar², gleich oder verschieden sind und ausgewählt werden aus einer Biphenyl-, Terphenyl- oder Quarterphenylgruppe, die mit einem oder mehreren Resten R⁴ substituiert sein können, wobei bevorzugt ist, wenn die Gruppen nicht substituiert sind.

7. Vorrichtung gemäß einem oder mehreren der Ansprüche 1 bis 6, **dadurch gekennzeichnet, dass** beide Reste R¹ identisch sind und ausgewählt werden aus einem aromatischen oder heteroaromatischen Ringsystem mit 6 bis 30 Ringatomen, das jeweils durch einen oder mehrere Reste R⁵ substituiert sein kann, oder ein kondensiertes Ringsystem mit 9 bis 30 Ringatomen, das jeweils durch einen oder mehrere Reste R⁵ substituiert sein kann, wobei im Fall aromatischer oder heteroaromatischer kondensierter Ringe nicht mehr als 10 Ringatome enthalten sein dürfen und wobei R² gleich H ist.

8. Vorrichtung gemäß einem oder mehreren der Ansprüche 1 bis 6, **dadurch gekennzeichnet, dass** beide Reste R¹ identisch sind und ausgewählt werden aus einer geradkettigen Alkylgruppe mit 1 bis 20 C-Atomen oder einer verzweigten oder cyclische Alkylgruppe mit 3 bis 20 C-Atomen, wobei die Gruppen jeweils mit einem oder mehreren Resten R⁵ substituiert sein können und wobei ein oder mehrere H-Atome in den oben genannten Gruppen durch D, F, Cl, Br, I, CN oder NO₂ ersetzt sein können und wobei R² ein aromatisches oder heteroaromatisches Ringsystem mit 6 bis 30 aromatischen Ringatomen, das jeweils durch einen oder mehrere Reste R⁵ substituiert sein kann.

9. Vorrichtung gemäß einem oder mehreren der Ansprüche 1 bis 8, **dadurch gekennzeichnet, dass** es sich um einen organischen lichtemittierenden Transistor (OLETs), eine organische Feld Quench-Vorrichtung (OFQDs), eine organische lichtemittierende elektrochemische Zellen (OLECs, LECs oder LEECs), eine organische Laserdiode (O-Laser) und organische lichtemittierende Diode (OLEDs) handelt.

10. Vorrichtung gemäß einem oder mehreren der Ansprüche 1 bis 10, insbesondere eine organische lichtemittierende Diode (OLED), **dadurch gekennzeichnet, dass** die Verbindung in einer der folgenden Funktionen eingesetzt wird: als Lochtransportmaterial in einer Lochtransport- oder Lochinjektionsschicht, als Matrixmaterial in einer emittierenden Schicht, als Elektronenblockiermaterial, als Excitonenblockiermaterial.

11. Verbindung der allgemeinen Formel (167) 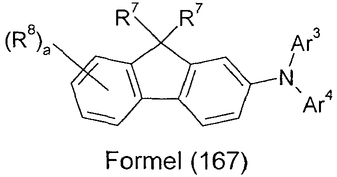 wobei für die verwendeten Symbole in Formel (167) gilt:
Ar³, Ar⁴ sind bei jedem Auftreten gleich oder verschieden ein aromatisches oder heteroaromatisches Ringsystem mit 10 bis 60 Ringatomen, welche mit einem oder mehreren Resten R⁵, die gleich oder verschieden voneinander sind, substituiert sein können, wobei beide Gruppen Ar³ und Ar⁴ jeweils mindestens zwei oder mehr aromatische oder heteroaromatische, bevorzugt aromatische, Ringe enthalten;
R⁷ ist bei jedem Auftreten gleich und ausgewählt aus der Gruppe bestehend aus einer geradkettigen Alkyl-, Alkoxy- oder Thioalkylgruppe mit 1 bis 20 C-Atomen oder einer verzweigte oder cyclische Alkyl-, Alkoxy- oder Thioalkylgruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R⁵ substituiert sein können und wobei ein oder mehrere H-Atome in den oben genannten Gruppen durch D, CN oder NO₂ ersetzt sein können, oder ein aromatisches oder heteroaromatisches Ringsystem mit 6 bis 30 Ringatomen, das jeweils durch einen oder mehrere Reste R⁵ substituiert sein kann, wobei R⁵ wie oben angegeben definiert ist oder ein kondensiertes Ringsystem mit 9 bis 30 Ringatomen, das jeweils durch einen oder mehrere Reste R⁵ substituiert sein kann, wobei im Fall aromatischer oder heteroaromatischer kondensierter Ringe nicht mehr als 10 Ringatome indem kondensierten Ringsystem enthalten sein dürfen; beide Reste R⁷ können auch miteinander einen Ringschluss bilden können, so dass eine Spiro Verbindung entsteht, wobei an den durch die beiden Reste R⁷ gebildeten Ring keine aromatischen oder heteroaromatischen Ringe kondensiert sind, und wobei, wenn R⁷ eine geradkettige oder verzweigte Alkylgruppe ist, R⁸ ein aromatisches oder heteroaromatisches Ringsystem mit 6 bis 30 Ringatomen ist, das jeweils durch einen oder mehrere Reste R⁵ substituiert sein kann und wobei R⁵ wie oben angegeben definiert;
R⁸ ist H, D oder ein aromatisches oder heteroaromatisches Ringsystem mit 6 bis 30 Ringatomen, das jeweils durch einen oder mehrere Reste R⁵ substituiert sein kann, wobei R⁵ wie oben angegeben definiert ist und wobei wenn R⁸ gleich H ist, R⁷ ein aromatisches oder heteroaromatisches Ringsystem mit 6 bis 30 aromatischen Ringatomen ist, das jeweils durch einen oder mehrere Reste R⁵ substituiert sein kann, wobei R⁵ wie oben angegeben definiert ist;
a ist entweder 1, 2, 3 oder 4, bevorzugt 1 oder 2, ganz bevorzugt 1;
mit der Maßgabe, dass die Verbindung nach Formel (167) neben der einen Fluoren Gruppe und neben den möglichen kondensierten oder polycyclischen Gruppen in Position 9 des Fluorens keine weiteren polycyclischen oder kondensierten Gruppen enthält
und mit der Maßgabe, dass die Verbindung keine Halogene enthält.

12. Verbindung nach Anspruch 11, **dadurch gekennzeichnet, dass** sie die allgemeine Formel (168) hat 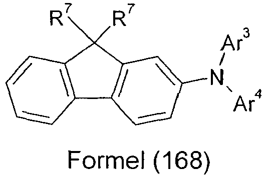 wobei für die verwendeten Symbole gilt, dass
Ar³, Ar⁴ bei jedem Auftreten gleich oder verschieden sind und ausgewählt werden aus einer Biphenyl-, Terphenyl- und Quarterphenylgruppe, die mit einem oder mehreren Resten R⁵ substituiert sein können, wobei bevorzugt ist, wenn diese Gruppen nicht substituiert sind;
R⁷ gleich bei jedem Auftreten ist und ausgewählt aus einem aromatischen oder heteroaromatischen Ringsystem mit 6 bis 30 Ringatomen, das jeweils durch einen oder mehrere Reste R⁵ substituiert sein kann, wobei R⁵ wie oben angegeben definiert ist oder ein kondensiertes Ringsystem mit 9 bis 30 Ringatomen, das jeweils durch einen oder mehrere Reste R⁵ substituiert sein kann, wobei im Fall aromatischer oder heteroaromatischer kondensierter Ringe nicht mehr als 10 Ringatome indem kondensierten Ringsystem enthalten sein dürfen.

13. Verbindung nach Anspruch 11, **dadurch gekennzeichnet, dass** sie die allgemeine Formel (169) hat 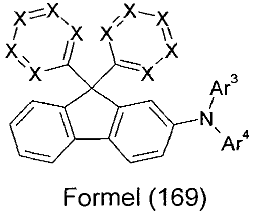 wobei für die verwendeten Symbole gilt, dass
X gleich oder verschieden bei jedem Auftreten N oder CR⁵ ist, bevorzugt ist X gleich CR⁵;
Ar³ und Ar⁴ bei jedem Auftreten gleich oder verschieden sind und ausgewählt werden aus einer Biphenyl-, Terphenyl- und Quarterphenylgruppe, die jeweils mit einem oder mehreren Resten R⁵ substituiert sein kann, wobei bevorzugt ist, wenn diese Gruppen nicht substituiert sind;
und wobei R⁵ wie in Anspruch 1 angegeben definiert ist.

14. Verfahren zur Herstellung einer Verbindung gemäß einem oder mehreren der Ansprüche 11 bis 13 mittels einstufiger Buchwald Kupplung durch Umsetzung eines Fluorenderivats, das eine Abgangsgruppe enthält, mit Ar³-NH-Ar⁴.

15. Verfahren zur Herstellung einer Verbindung gemäß einem oder mehreren der Ansprüche 11 bis 13 mittels zweistufiger Buchwald Kupplung durch stufenweise Umsetzung eines Phenanthrenderivats, das eine Abgangsgruppe enthält, mit (1) Ar³-NH₂ und (2) NH₂-Ar⁴.

16. Oligomer, Polymer oder Dendrimer enthaltend eine oder mehrere Verbindungen gemäß einem oder mehreren der Ansprüche 11 bis 13, wobei die Bindung(en) zum Polymer, Oligomer oder Dendrimer an beliebigen Positionen lokalisiert sein können.

17. Zusammensetzung enthaltend eine oder mehrere Verbindung gemäß einem oder mehreren der Ansprüche 11 bis 13 und wenigstens ein weiteres organisch funktionelles Material ausgewählt aus der Gruppe bestehend aus fluoreszierenden Emitter, phosphoreszierenden Emitter, Host Materialien, Matrix Materialien, Elektronentransportmaterialien, Elektroneninjektionsmaterialien, Lochleitermaterialien, Lochinjektionsmaterialien, Elektronenblockiermaterialien und Lochblockiermaterialien.

18. Formulierung enthaltend mindestens eine Verbindung gemäß einem oder mehreren der Ansprüche 11 bis 13 oder mindestens ein Polymer, Oligomer oder Dendrimer gemäß Anspruch 16 oder mindestens eine Zusammensetzung gemäß Anspruch 17 und mindestens ein Lösungsmittel.

19. Elektronische Vorrichtung, enthaltend mindestens eine Verbindung gemäß einem oder mehreren der Ansprüche 11 bis 13 oder mindestens ein Polymer, Oligomer oder Dendrimer gemäß Anspruch 16 oder mindestens eine Zusammensetzung nach Anspruch 17.

20. Elektronische Vorrichtung gemäß Anspruch 19, **dadurch gekennzeichnet, dass** sie ausgewählt ist aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs).

21. Elektronische Vorrichtung gemäß Anspruch 19 oder 20, welche ausgewählt ist aus der Gruppe der organischen Elektrolumineszenzvorrichtungen, insbesondere eine organische lichtemittierende Diode (OLED), **dadurch gekennzeichnet, dass** die Verbindung gemäß einem oder mehreren der Ansprüche 11 bis 13 oder das Polymer, Oligomer oder Dendrimer gemäß Anspruch 16 oder eine Zusammensetzung nach Anspruch 17 in einer oder mehreren der folgenden Funktionen eingesetzt wird: als Lochtransportmaterial in einer Lochtransport- oder Lochinjektionsschicht, als Matrixmaterial in einer emittierenden Schicht, als Elektronenblockiermaterial, als Excitonenblockiermaterial.

## Claims

1. Electroluminescent device comprising at least one compound of the general formula (1) 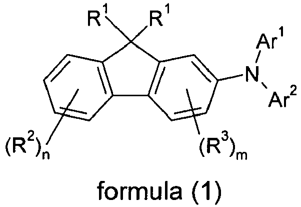 where the following applies to the symbols and indices occurring:
Ar¹, Ar² are on each occurrence, identically or differently, an aromatic or heteroaromatic group having 10 to 60 aromatic ring atoms, which may be substituted by one or more radicals R⁴, which are identical to or different from one another, where the two groups Ar¹ and Ar² each contain at least two or more aromatic or heteroaromatic rings;
R¹ is on each occurrence, identically or differently, H, D, F, Cl, Br, I, C(=O)R⁵, CN, Si(R⁵)₃, NO₂, P(=O)(R⁵)₂, S(=O)R⁵, S(=O)₂R⁵, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁵ and where one or more CH₂ groups in the above-mentioned groups may be replaced by -R⁵C=CR⁵-, -C=C-, Si(R⁵)₂, C=O, C=S, C=NR⁵, -C(=O)O-, -C(=O)NR⁵-, P(=O)(R⁵), -O-, -S-, SO or SO₂ and where one or more H atoms in the above-mentioned groups may be replaced by D, F, Cl, Br, I, CN or NO₂, or an aromatic or heteroaromatic ring system having 6 to 30 ring atoms, which may in each case be substituted by one or more radicals R⁵, or a condensed ring system having 9 to 30 ring atoms, which may in each case be substituted by one or more radicals R⁵, where, in the case of aromatic or heteroaromatic condensed rings, not more than 10 ring atoms may be present; the two radicals R¹ may also form a ring closure with one another, so that a spiro compound forms, where no aromatic or heteroaromatic rings are condensed onto the ring formed by the two radicals R¹;
R², R³ and R⁴ are on each occurrence, identically or differently, H, D, F, Cl, Br, I, C(=O)R⁵, CN, Si(R⁵)₃, NO₂, P(=O)(R⁵)2, S(=O)R⁵, S(=O)₂R⁵, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁵ and where one or more CH₂ groups in the above-mentioned groups may be replaced by -R⁵C=CR⁵-, -C≡C-, Si(R⁵)₂, C=O, C=S, C=NR⁵, -C(=O)O-, -C(=O)NR⁵-, P(=_{O})(R⁵), -O-, -S-, SO or SO₂ and where one or more H atoms in the above-mentioned groups may be replaced by D, F, Cl, Br, I, CN or NO₂, or an aromatic or heteroaromatic ring system having 6 to 30 ring atoms, which may in each case be substituted by one or more radicals R⁵;
R⁵ is on each occurrence, identically or differently, H, D, F, Cl, Br, I, C(=O)R⁶, CN, Si(R⁶)₃, NO₂, P(=O)(R⁶)₂, S(=O)R⁶, S(=O)₂R⁶, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁶ and where one or more CH₂ groups in the above-mentioned groups may be replaced by -R⁶C=CR⁶-, -C≡C-, Si(R⁶)₂, C=O, C=S, C=NR⁶, -C(=O)O-, -C(=O)NR⁶-, P(=O)(R⁶), -O-, -S-, SO or SO₂ and where one or more H atoms in the above-mentioned groups may be replaced by D, F, Cl, Br, I, CN or NO₂, or an aromatic or heteroaromatic ring system having 5 to 30 aromatic ring atoms, which may in each case be substituted by one or more radicals R⁶, or an aryloxy or heteroaryloxy group having 5 to 30 aromatic ring atoms, which may be substituted by one or more radicals R⁶;
R⁶ is on each occurrence, identically or differently, H, D, F or an aliphatic, aromatic or heteroaromatic organic radical having 1 to 20 C atoms, in which, in addition, one or more H atoms may be replaced by D or F;
n is 0, 1, 2, 3 or 4;
m is 0, 1, 2 or 3;
with the proviso that the compound of the formula (1), besides the one fluorene group and besides the possible condensed or polycyclic groups in position 9 of the fluorene, contains no further polycyclic or condensed groups.

2. Device according to Claim 1, **characterised in that** the two radicals R¹ in the compound of the formula (1) are identical.

3. Device according to Claim 1 or 2, **characterised in that** m is equal to 1 and n is equal to 0, 1 or 2.

4. Device according to one or more of Claims 1 to 3, **characterised in that** it comprises at least one compound of the general formula (2) 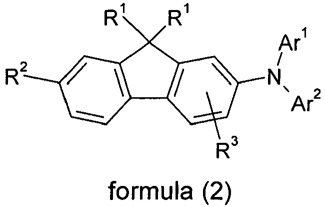 where the definitions from Claim 1 apply to the symbols.

5. Device according to one or more of Claims 1 to 4, **characterised in that** R³ is equal to H.

6. Device according to one or more of Claims 1 to 5, **characterised in that** Ar¹ and Ar² are identical or different and are selected from a biphenyl, terphenyl or quaterphenyl group, each of which may be substituted by one or more radicals R⁴, where it is preferred for the groups to be unsubstituted.

7. Device according to one or more of Claims 1 to 6, **characterised in that** the two radicals R¹ are identical and are selected from an aromatic or heteroaromatic ring system having 6 to 30 ring atoms, which may in each case be substituted by one or more radicals R⁵, or a condensed ring system having 9 to 30 ring atoms, which may in each case be substituted by one or more radicals R⁵, where, in the case of aromatic or heteroaromatic condensed rings, not more than 10 ring atoms may be present and where R² is equal to H.

8. Device according to one or more of Claims 1 to 6, **characterised in that** the two radicals R¹ are identical and are selected from a straight-chain alkyl group having 1 to 20 C atoms or a branched or cyclic alkyl group having 3 to 20 C atoms, where the groups may each be substituted by one or more radicals R⁵ and where one or more H atoms in the above-mentioned groups may be replaced by D, F, Cl, Br, I, CN or NO₂ and where R² is an aromatic or heteroaromatic ring system having 6 to 30 aromatic ring atoms, which may in each case be substituted by one or more radicals R⁵.

9. Device according to one or more of Claims 1 to 8, **characterised in that** it is an organic light-emitting transistor (OLET), an organic field-quench device (OFQD), an organic light-emitting electrochemical cell (OLEC, LEC or LEEC), an organic laser diode (O-laser) or an organic light-emitting diode (OLED).

10. Device according to one or more of Claims 1 to 9, in particular an organic light-emitting diode (OLED), **characterised in that** the compound is employed in one of the following functions: as hole-transport material in a hole-transport or hole-injection layer, as matrix material in an emitting layer, as electron-blocking material or as exciton-blocking material.

11. Compound of the general formula (167) 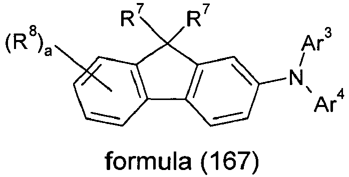 where the following applies to the symbols used in formula (167):
Ar³, Ar⁴ are on each occurrence, identically or differently, an aromatic or heteroaromatic ring system having 10 to 60 ring atoms, which may be substituted by one or more radicals R⁵, which are identical to or different from one another, where the two groups Ar³ and Ar⁴ each contain at least two or more aromatic or heteroaromatic, preferably aromatic, rings;
R⁷ is identical on each occurrence and is selected from the group consisting of a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may each be substituted by one or more radicals R⁵ and where one or more H atoms in the above-mentioned groups may be replaced by D, CN or NO₂, or an aromatic or heteroaromatic ring system having 6 to 30 ring atoms, which may in each case be substituted by one or more radicals R⁵, where R⁵ is defined as indicated above, or a condensed ring system having 9 to 30 ring atoms, which may in each case be substituted by one or more radicals R⁵, where, in the case of aromatic or heteroaromatic condensed rings, not more than 10 ring atoms may be present in the condensed ring system; the two radicals R⁷ may also form a ring closure with one another, so that a spiro compound forms, where no aromatic or heteroaromatic rings are condensed onto the ring formed by the two radicals R⁷, and where, if R⁷ is a straight-chain or branched alkyl group, R⁸ is an aromatic or heteroaromatic ring system having 6 to 30 ring atoms, which may in each case be substituted by one or more radicals R⁵, and where R⁵ is defined as indicated above;
R⁸ is H, D or an aromatic or heteroaromatic ring system having 6 to 30 ring atoms, which may in each case be substituted by one or more radicals R⁵, where R⁵ is defined as indicated above and where, if R⁸ is equal to H, R⁷ is an aromatic or heteroaromatic ring system having 6 to 30 aromatic ring atoms, which may in each case be substituted by one or more radicals R⁵, where R⁵ is defined as indicated above;
a is either 1, 2, 3 or 4, preferably 1 or 2, very preferably 1;
with the proviso that the compound of the formula (167), besides the one fluorene group and besides the possible condensed or polycyclic groups in position 9 of the fluorene, contains no further polycyclic or condensed groups
and with the proviso that the compound contains no halogens.

12. Compound according to Claim 11, **characterised in that** it has the general formula (168) 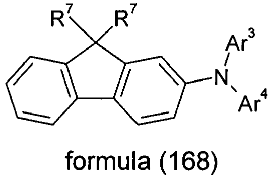 where, for the symbols used,
Ar³, Ar⁴ are identical or different on each occurrence and are selected from a biphenyl, terphenyl or quaterphenyl group, which may be substituted by one or more radicals R⁵, where it is preferred for these groups to be unsubstituted;
R⁷ is identical on each occurrence and is selected from an aromatic or heteroaromatic ring system having 6 to 30 ring atoms, which may in each case be substituted by one or more radicals R⁵, where R⁵ is defined as indicated above, or a condensed ring system having 9 to 30 ring atoms, which may in each case be substituted by one or more radicals R⁵, where, in the case of aromatic or heteroaromatic condensed rings, not more than 10 ring atoms may be present in the condensed ring system.

13. Compound according to Claim 11, **characterised in that** it has the general formula (169) 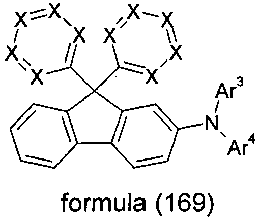 where, for the symbols used,
X is, identically or differently on each occurrence, N or CR⁵, preferably X is equal to CR⁵;
Ar³ and Ar⁴ are identical or different on each occurrence and are selected from a biphenyl, terphenyl and quaterphenyl group, each of which may be substituted by one or more radicals R⁵, where it is preferred for these groups to be unsubstituted; and where R⁵ is defined as indicated in Claim 1.

14. Process for the preparation of a compound according to one or more of Claims 11 to 13 by means of one-step Buchwald coupling by reaction of a fluorene derivative containing a leaving group with Ar³-NH-Ar⁴.

15. Process for the preparation of a compound according to one or more of Claims 11 to 13 by means of two-step Buchwald coupling by stepwise reaction of a phenanthrene derivative containing a leaving group with (1) Ar³-NH₂ and (2) NH₂-Ar⁴.

16. Oligomer, polymer or dendrimer containing one or more compounds according to one or more of Claims 11 to 13, where the bond(s) to the polymer, oligomer or dendrimer may be localised at any desired positions.

17. Composition comprising one or more compounds according to one or more of Claims 11 to 13 and at least one further organically functional material selected from the group consisting of fluorescent emitters, phosphorescent emitters, host materials, matrix materials, electron-transport materials, electron-injection materials, hole-conductor materials, hole-injection materials, electron-blocking materials and hole-blocking materials.

18. Formulation comprising at least one compound according to one or more of Claims 11 to 13 or at least one polymer, oligomer or dendrimer according to Claim 16 or at least one composition according to Claim 17 and at least one solvent.

19. Electronic device comprising at least one compound according to one or more of Claims 11 to 13 or at least one polymer, oligomer or dendrimer according to Claim 16 or at least one composition according to Claim 17.

20. Electronic device according to Claim 19, **characterised in that** it is selected from organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs).

21. Electronic device according to Claim 19 or 20 which is selected from the group of organic electroluminescent devices, in particular an organic light-emitting diode (OLED), **characterised in that** the compound according to one or more of Claims 11 to 13 or the polymer, oligomer or dendrimer according to Claim 16 or a composition according to Claim 17 is employed in one or more of the following functions: as hole-transport material in a hole-transport or hole-injection layer, as matrix material in an emitting layer, as electron-blocking material or as exciton-blocking material.

## Revendications

1. Dispositif électroluminescent comprenant au moins un composé de la formule générale (1) : 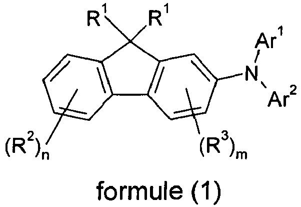 dans laquelle ce qui suit s'applique aux symboles et indices présents :
Ar¹, Ar² sont, pour chaque occurrence, de manière identique ou différente, un groupe aromatique ou hétéroaromatique comportant 10 à 60 atomes de cycle aromatique, lesquels peuvent être substitués par un radical ou plusieurs radicaux R⁴, lesquels sont identiques les uns aux autres ou différents les uns des autres, où les deux groupes Ar¹ et Ar² contiennent chacun au moins deux cycles aromatiques ou hétéroaromatiques ou plus ;
R¹ est, pour chaque occurrence, de manière identique ou différente, H, D, F, Cl, Br, I, C(=O)R⁵, CN, Si(R⁵)₃, NO₂, P(=O)(R⁵)₂, S(=O)R⁵, S(=O)₂R⁵, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁵ et où un ou plusieurs groupes CH₂ dans les groupes mentionnés ci avant peut/ peuvent être remplacé(s) par -R⁵C=CR⁵-, -C≡C-, Si(R⁵)₂, C=O, C=S, C=NR⁵, -C(=O)O-, -C(=O)NR⁵-, P(=O)(R⁵), -O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant 6 à 30 atomes de cycle, lequel peut, dans chaque cas, être substitué par un radical ou plusieurs radicaux R⁵, ou un système de cycle condensé comportant 9 à 30 atomes de cycle, lequel peut, dans chaque cas, être substitué par un radical ou plusieurs radicaux R⁵, où, dans le cas des cycles condensés aromatiques ou hétéroaromatiques, pas plus de 10 atomes de cycle ne peuvent être présents ; les deux radicaux R¹ peuvent également former une fermeture de cycle l'un avec l'autre, de telle sorte qu'un composé spiro se forme, où aucun cycle aromatique ou hétéroaromatique n'est condensé sur le cycle formé par les deux radicaux R¹ ;
R², R³ et R⁴ sont, pour chaque occurrence, de manière identique ou différente, H, D, F, CI, Br, I, C(=O)R⁵, CN, Si(R⁵)₃, NO₂, P(=O)(R⁵)₂, S(=O)R⁵, S(=O)₂R⁵, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitué par un radical ou plusieurs radicaux R⁵ et où un ou plusieurs groupes CH₂ dans les groupes mentionnés ci avant peut/ peuvent être remplacé(s) par -R⁵C=CR⁵-, -C≡C-, Si(R⁵)₂, C=O, C=S, C=NR⁵, -C(=O)O-, -C(=O)NR⁵-, P(=O)(R⁵), -O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant 6 à 30 atomes de cycle, lequel peut, dans chaque cas, être substitué par un radical ou plusieurs radicaux R⁵ ;
R⁵ est, pour chaque occurrence, de manière identique ou différente, H, D, F, CI, Br, I, C(=O)R⁶, CN, Si(R⁶)₃, NO₂, P(=O)(R⁶)₂, S(=O)R⁶ S(=O)₂R⁶, un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁶ et où un ou plusieurs groupe(s) CH₂ dans les groupes mentionnés ci avant peut/ peuvent être remplacé(s) par -R⁶C=CR⁶-, -C≡C-, Si(R⁶)₂, C=O, C=S, C=NR⁶, -C(=O)O-, -C(=O)NR⁶-, P(=O)(R⁶), -O-, -S-, SO ou SO₂ et où un ou plusieurs atome(s) de H dans les groupes mentionnés ci avant peut/peuvent être remplacé(s) par D, F, Cl, Br, I, CN ou NO₂, ou un système de cycle aromatique ou hétéroaromatique comportant 5 à 30 atomes de cycle aromatique, lequel peut, dans chaque cas, être substitué par un radical ou plusieurs radicaux R⁶, ou un groupe aryloxy ou hétéroaryloxy comportant 5 à 30 atomes de cycle aromatique, lequel peut être substitué par un radical ou plusieurs radicaux R⁶ ;
R⁶ est, pour chaque occurrence, de manière identique ou différente, H, D, F ou un radical organique aromatique ou hétéroaromatique aliphatique comportant 1 à 20 atome(s) de C, où, en outre, un ou plusieurs atome(s) de H peut/peuvent être remplacé(s) par D ou F ;
n est 0, 1, 2, 3 ou 4 ;
m est 0, 1, 2 ou 3 ;
étant entendu que le composé de la formule (1), en plus du groupe fluorène et en plus des groupes condensés ou polycycliques éventuels à la position 9 du fluorène, ne contient pas d'autres groupes polycycliques ou condensés.

2. Dispositif selon la revendication 1, **caractérisé en ce que** les deux radicaux R¹ dans le composé de la formule (1) sont identiques.

3. Dispositif selon la revendication 1 ou 2, **caractérisé en ce que** m est égal à 1 et n est égal à 0, 1 ou 2.

4. Dispositif selon une ou plusieurs des revendications 1 à 3, **caractérisé en ce qu'**il comprend au moins un composé de la formule générale (2) : 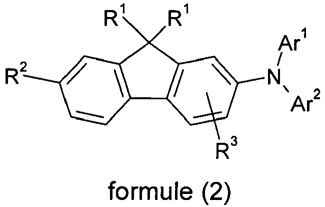 dans laquelle les définitions issues de la revendication 1 s'appliquent aux symboles.

5. Dispositif selon une ou plusieurs des revendications 1 à 4, **caractérisé en ce que** R³ est égal à H.

6. Dispositif selon une ou plusieurs des revendications 1 à 5, **caractérisé en ce que** Ar¹ et Ar² sont identiques ou différents et sont choisis parmi un groupe biphényle, terphényle ou quaterphényle dont chacun peut être substitué par un radical ou plusieurs radicaux R⁴, où le fait que les groupes soient non substitués a la préférence.

7. Dispositif selon une ou plusieurs des revendications 1 à 6, **caractérisé en ce que** les deux radicaux R¹ sont identiques et sont sélectionnés parmi un système de cycle aromatique ou hétéroaromatique comportant 6 à 30 atomes de cycle, lequel peut, dans chaque cas, être substitué par un radical ou plusieurs radicaux R⁵, ou un système de cycle condensé comportant 9 à 30 atomes de cycle, lequel peut, dans chaque cas, être substitué par un radical ou plusieurs radicaux R⁵, où, dans le cas de cycles condensés aromatiques ou hétéroaromatiques, pas plus de 10 atomes de cycle ne peuvent être présents et où R² est égal à H.

8. Dispositif selon une ou plusieurs des revendications 1 à 6, **caractérisé en ce que** les deux radicaux R¹ sont identiques et sont choisis parmi un groupe alkyle en chaîne droite comportant 1 à 20 atome(s) de C ou un groupe alkyle ramifié ou cyclique comportant 3 à 20 atomes de C, où les groupes peuvent chacun être substitués par un radical ou plusieurs radicaux R⁵ et où un ou plusieurs atome(s) de H dans les groupes mentionnés ci avant peut/peuvent être remplacé(s) par D, F, Cl, Br, I, CN ou NO₂ et où R² est un système de cycle aromatique ou hétéroaromatique comportant 6 à 30 atomes de cycle aromatique, lequel peut, dans chaque cas, être substitué par un radical ou plusieurs radicaux R⁵.

9. Dispositif selon une ou plusieurs des revendications 1 à 8, **caractérisé en ce qu'**il s'agit d'un transistor à émission de lumière organique (OLET), d'un dispositif à extinction de champ organique (OFQD), d'une cellule électrochimique à émission de lumière organique (OLEC, LEC ou LEEC), dune diode laser organique (O-laser) ou d'une diode à émission de lumière organique (OLED).

10. Dispositif selon une ou plusieurs des revendications 1 à 9, en particulier une diode à émission de lumière organique (OLED), **caractérisé en ce que** le composé est utilisé pour l'une des fonctions qui suivent : en tant que matériau de transport de trous dans une couche de transport de trous ou d'injection de trous, en tant que matériau de matrice dans une couche d'émission, en tant que matériau de blocage d'électrons ou en tant que matériau de blocage d'excitons.

11. Composé de la formule générale (167) : 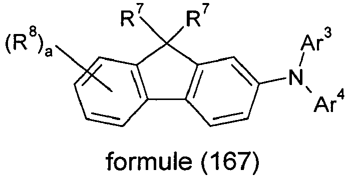 dans laquelle ce qui suit s'applique aux symboles utilisés dans la formule (167) :
Ar³, Ar⁴ sont, pour chaque occurrence, de manière identique ou différente, un système de cycle aromatique ou hétéroaromatique comportant 10 à 60 atomes de cycle, lequel peut être substitué par un radical ou plusieurs radicaux R⁵, lesquels sont identiques les uns aux autres ou différents les uns des autres, où les deux groupes Ar³ et Ar⁴ contiennent chacun au moins deux cycles aromatiques ou hétéroaromatiques, de façon préférable, aromatiques ;
R⁷ est identique pour chaque occurrence et est choisi parmi le groupe constitué par un groupe alkyle, alcoxy ou thioalkyle en chaîne droite comportant 1 à 20 atome(s) de C ou un groupe alkyle, alcoxy ou thioalkyle ramifié ou cyclique comportant 3 à 20 atomes de C ou un groupe alkényle ou alkynyle comportant 2 à 20 atomes de C, où les groupes mentionnés ci avant peuvent chacun être substitués par un radical ou plusieurs radicaux R⁵ et où un ou plusieurs atome(s) de H dans les groupes mentionnés ci avant peut/peuvent être remplacé(s) par D, CN ou NO₂, ou un système de cycle aromatique ou hétéroaromatique comportant 6 à 30 atomes de cycle, lequel peut, dans chaque cas, être substitué par un radical ou plusieurs radicaux R⁵, où R⁵ est défini comme indiqué ci avant, ou un système de cycle condensé comportant 9 à 30 atomes de cycle, lequel peut, dans chaque cas, être substitué par un radical ou plusieurs radicaux R⁵, où, dans le cas de cycles condensés aromatiques ou hétéroaromatiques, pas plus de 10 atomes de cycle ne peuvent être présents dans le système de cycle condensé ; les deux radicaux R⁷ peuvent également former une fermeture de cycle l'un avec l'autre, de telle sorte qu'un composé spiro se forme, où aucun cycle aromatique ou hétéroaromatique n'est condensé sur le cycle formé par les deux radicaux R⁷,
et où, si R⁷ est un groupe alkyle en chaîne droite ou ramifié, R⁸ est un système de cycle aromatique ou hétéroaromatique comportant 6 à 30 atomes de cycle, lequel peut, dans chaque cas, être substitué par un radical ou plusieurs radicaux R⁵, et où R⁵ est défini comme indiqué ci avant ;
R⁸ est H, D ou un système de cycle aromatique ou hétéroaromatique comportant 6 à 30 atomes de cycle, lequel peut, dans chaque cas, être substitué par un radical ou plusieurs radicaux R⁵, où R⁵ est défini comme indiqué ci avant et où, si R⁸ est égal à H, R⁷ est un système de cycle aromatique ou hétéroaromatique comportant 6 à 30 atomes de cycle aromatique, lequel peut, dans chaque cas, être substitué par un radical ou plusieurs radicaux R⁵, où R⁵ est défini comme indiqué ci avant ;
a est 1, 2, 3 ou 4, de façon préférable 1 ou 2, de façon très préférable 1 ;
étant entendu que le composé de la formule (167), en plus du groupe fluorène et en plus des éventuels groupes condensés ou polycycliques à la position 9 du fluorène, ne contient aucun autre groupe polycyclique ou condensé,
et étant entendu que le composé ne contient pas d'halogènes.

12. Composé selon la revendication 11, **caractérisé en ce qu'**il présente la formule générale (168) : 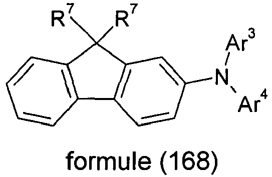 dans laquelle, en ce qui concerne les symboles utilisés,
Ar³, Ar⁴ sont identiques ou différents pour chaque occurrence et sont choisis parmi un groupe biphényle, terphényle ou quaterphényle, lequel peut être substitué par un radical ou plusieurs radicaux R⁵, où il est préférable que ces groupes soient non substitués ;
R⁷ est identique pour chaque occurrence et est choisi parmi un système de cycle aromatique ou hétéroaromatique comportant 6 à 30 atomes de cycle, lequel peut, dans chaque cas, être substitué par un radical ou plusieurs radicaux R⁵, où R⁵ est défini comme indiqué ci avant, ou un système de cycle condensé comportant 9 à 30 atomes de cycle, lequel peut, dans chaque cas, être substitué par un radical ou plusieurs radicaux R⁵, où, dans le cas de cycles condensés aromatiques ou hétéroaromatiques, pas plus de 10 atomes de cycle ne peuvent être présents dans le système de cycle condensé.

13. Composé selon la revendication 11, **caractérisé en ce qu'**il présente la formule générale (169) : 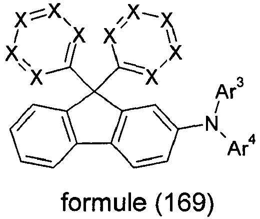 dans laquelle, en ce qui concerne les symboles utilisés,
X est, de manière identique ou différente pour chaque occurrence, N ou CR⁵, de façon préférable, X est égal à CR⁵ ;
Ar³ et Ar⁴ sont identiques ou différents pour chaque occurrence et sont choisis parmi un groupe biphényle, terphényle et quaterphényle dont chacun peut être substitué par un radical ou plusieurs radicaux R⁵, où il est préférable que ces groupes soient non substitués ;
et où R⁵ est défini comme indiqué selon la revendication 1.

14. Procédé pour la préparation d'un composé selon une ou plusieurs des revendications 11 à 13 au moyen d'un couplage de Buchwald à une seule étape par réaction d'un dérivé de fluorène contenant un groupe partant avec Ar³-NH-Ar⁴.

15. Procédé pour la préparation d'un composé selon une ou plusieurs des revendications 11 à 13 au moyen d'un couplage de Buchwald à deux étapes par réaction étape par étape ou progressive d'un dérivé de phénanthrène contenant un groupe partant avec (1) Ar³-NH₂ et (2) NH₂-Ar⁴.

16. Oligomère, polymère ou dendrimère contenant un ou plusieurs composé(s) selon une ou plusieurs des revendications 11 à 13, où la/les liaison(s) sur le polymère, l'oligomère ou le dendrimère peut/peuvent être localisée(s) au niveau de n'importe quelles positions souhaitées.

17. Composition comprenant un ou plusieurs composé(s) selon une ou plusieurs des revendications 11 à 13 et au moins un autre matériau organiquement fonctionnel choisi parmi le groupe constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous.

18. Formulation comprenant au moins un composé selon une ou plusieurs des revendications 11 à 13 ou au moins un polymère, un oligomère ou un dendrimère selon la revendication 16 ou au moins une composition selon la revendication 17 et au moins un solvant.

19. Dispositif électronique comprenant au moins un composé selon une ou plusieurs des revendications 11 à 13 ou au moins un polymère, un oligomère ou un dendrimère selon la revendication 16 ou au moins une composition selon la revendication 17.

20. Dispositif électronique selon la revendication 19, **caractérisé en ce qu'**il est choisi parmi les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC), les diodes laser organiques (O-laser) et les dispositifs électroluminescents organiques (OLED).

21. Dispositif électronique selon la revendication 19 ou 20, lequel est choisi parmi le groupe des dispositifs électroluminescents organiques, en particulier une diode émettrice de lumière organique (OLED), **caractérisé en ce que** le composé selon une ou plusieurs des revendications 11 à 13 ou le polymère, l'oligomère ou le dendrimère selon la revendication 16 ou une composition selon la revendication 17 est utilisé(e) au niveau d'une ou de plusieurs des fonctions qui suivent : en tant que matériau de transport de trous dans une couche de transport de trous ou d'injection de trous, en tant que matériau de matrice dans une couche d'émission, en tant que matériau de blocage d'électrons ou en tant que matériau de blocage d'excitons.
